(19) Europäisches Patentamt
European Patent Office
Office européen des brevets

(11) Publication number: **0 206 741 B1**

(12) **EUROPEAN PATENT SPECIFICATION**

(45) Date of publication of patent specification: **10.04.91**

(51) Int. Cl.[5]: **C07D 311/24**, C07C 323/56, C07K 5/06, C07D 257/04, A61K 31/35, A61K 31/19, A61K 31/41

(21) Application number: **86304665.2**

(22) Date of filing: **17.06.86**

# (54) Leukotriene antagonists.

(30) Priority: **18.06.85 US 746203**

(43) Date of publication of application:
**30.12.86 Bulletin 86/52**

(45) Publication of the grant of the patent:
**10.04.91 Bulletin 91/15**

(84) Designated Contracting States:
**AT BE CH DE FR GB IT LI LU NL SE**

(56) References cited:
**EP-A- 0 104 885**
**EP-A- 0 106 565**
**EP-A- 0 150 118**
**GB-A- 2 128 999**

(73) Proprietor: **MERCK FROSST CANADA INC.**
**16711 Trans-Canada Highway**
**Kirkland Quebec(CA)**

(72) Inventor: **Young, Robert N.**
**216 Senneville Road Senneville**
**Quebec Canada H9X 3L2(CA)**
Inventor: **Frenette, Richard**
**1915 de Limbourg Vimont Laval**
**Quebec Canada H7M 457(CA)**
Inventor: **Gauthier, Jacques-Yves**
**412 des Prairies Laval**
**Quebec Canada H7N 2W5(CA)**

(74) Representative: **Crampton, Keith John Allen et al**
**D. YOUNG & CO. 10 Staple Inn**
**London WC1V 7RD(GB)**

**EP 0 206 741 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to compounds that act as antagonists of the leukotrienes and inhibitors of their biosynthesis.

The leukotrienes are a group of biologically active mediators derived from arachidonic acid through the action of lipoxygenase enzyme systems. There are two groups of leukotrienes derived from the common unstable precursor Leukotriene $A_4$. The first of these are the peptido-lipid leukotrienes, the most important being Leukotrienes $C_4$, $D_4$ and $E_4$. These compounds collectively account for the biologically active material known as the slow reacting substance of anaphylaxis (SRS-A).

The leukotrienes are potent smooth muscle contracting agents, particularly on respiratory smooth muscle but also on other tissues (e.g. gall bladder). In addition, they promote mucous production, modulate vascular permeability changes and are potent inflammatory agents in human skin. The most important compound in the second group of leukotrienes is Leukotriene $B_4$, a dihydroxy fatty acid. This compound is a potent chemotactic agent for neutrophils and eosinophils and in addition, may modulate a number of other functions of these cells. It also effects other cell types such as lymphocytes and for example may modulate the action of T-suppressor cells and natural killer cells. When injected in vivo, in addition to promoting the accumulation of leukocytes, Leukotriene $B_4$ is also a potent hyperalgesic agent and can modulate vascular permeability changes through a neutrophil dependent mechanism. Both groups of leukotrienes are formed following oxygenation of arachidonic acid through the action of a 5-lipoxygenase enzyme. See for example, D. M. Bailey et al , Ann. Rpts. Med. Chem. 17 203 (1982).

The leukotrienes are potent spasmogens of human trachea, bronchus and lung parenchymal strips, and when administered to normal volunteers as aerosols are 3,800 times more potent that histamine at inducing a 50% decrease in air flow at 30% of vital capacity. They mediate increases in vascular permeability in animals and promote mucous production in human bronchial explants. In addition, Leukotriene $B_4$ may also mediate mucous production and could be an important mediator of neutrophil and eosinophil accumulation in asthmatic lungs. 5-lipoxygenase products are also thought to be regulators of mast cell degranulation and recent studies with human lung mast cells have suggested that 5-lipoxygenase inhibitors, but not corticosteroids, may suppress antigen-induced mast cell degranulation. In vitro studies have shown that antigen challenge of human lung results in the release of leukotrienes and in addition purified human mast cells can produce substantial amount of leukotrienes. There is therefore good evidence that leukotrienes are important mediators of human asthma. Leukotriene antagonists or inhibitors would therefore be a new class of drugs for the treatment of asthma.

Psoriasis is a human skin disease which effects between two and six percent of the population. There is no adequate therapy for psoriasis and related skin conditions. The evidence for leukotriene involvement in these diseases is as follows. One of the earliest events in the development of prepapillary lesions is the recruitment of leukocytes to the skin site. Injection of Leukotriene $B_4$ into human skin results in a pronounced neutrophil accumulation. There are gross abnormalities in arachidonic acid metabolism in human psoriatic skin. In particular, highly elevated levels of free arachidonic acid can be measured as well as large amounts of lipoxygenase products. Leukotriene $B_4$ and 8- and 12-HETE have been detected in psoriatic lesions, but not in uninvolved skin, in biologically significant amounts.

Leukotrienes can be measured in nasal washings from patients with allergic rhinitis and are greatly elevated following antigen challenge. Leukotrienes may mediate this disease through their ability to regulate mast cell degranulation, by modulating mucous production and mucocillary clearance and by mediating the accumulation of inflammatory leukocytes.

Leukotrienes can also mediate other diseases. These include atopic dermatitis, allergic conjunctivitis, gouty arthritis, and gall bladder spasms. In addition, they may have a role in cardiovascular disease because Leukotrienes $C_4$, $D_4$ and $E_4$ act as coronary and cerebral arterial vasoconstrictors and these compounds may also have negative inotropic effects on the myocardium. In addition, the leukotrienes are important mediators of inflammatory diseases through their ability to modulate leukocyte and lymphocyte function. See for example, B. Samuelson, Science, 220 568(1983).

Several classes of compounds exhibit ability to antagonize the action of leukotrienes in mammals, especially humans. See for example: United Kingdom Patent Specifications GB-A-2,058,785 and 2,094,301; and European Patent Specifications EP-A-0,056,172, 0,061,800 and 0,068,739.

The compounds of the present invention have the formula I:

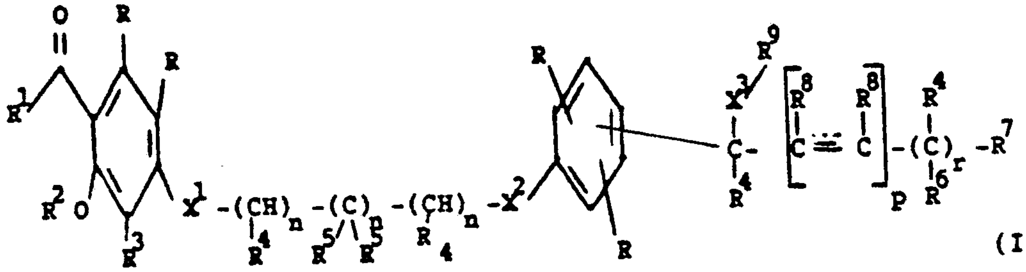

(I)

wherein:

the broken line represents an optional triple bond;

each R is independently H; OH; lower alkyl; lower alkenyl; trifluoromethyl; lower alkoxy; phenyl; phenyl substituted by alkyl of 1 to 3 carbon atoms or by halogen; benzyl; phenethyl; halogen; $N(R^4)_2$; $-(C=O)R^1$; $CH_2OR^4$; CN; $SR^{10}$; $SOR^{10}$; $SO_2R^{10}$; or nitro;

$R^1$ is H; lower alkyl; or lower alkoxy;

$R^2$ is H; lower alkyl; $R^4CO$; or $R^4OCH_2$;

each $R^3$ is independently lower alkyl or lower alkenyl;

each $R^4$ is independently H or lower alkyl;

each $R^5$ is independently H; $OR^2$; lower alkyl; or both $R^5$'s may be combined to create a doubly bonded oxygen (=O) or a $=C(R^4)_2$ group;

each $R^6$ is independently H; OH; or lower alkyl;

each $R^7$ is independently $COOR^4$; CHO; $CH_2OH$; tetrazole; $CONHSO_2R^{10}$; $NHSO_2R^{10}$; hydroxymethylketone; acetoxymethylketone; $CON(R^4)_2$; CN; Het; or

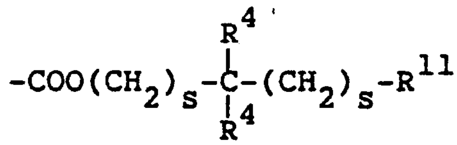

each $R^8$ is independently H or lower alkyl, and is absent when a triple bond is present;

$R^9$ is $R^3$,

$(CH_2)_a$-Het,

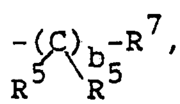

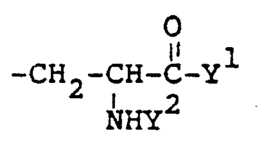

,

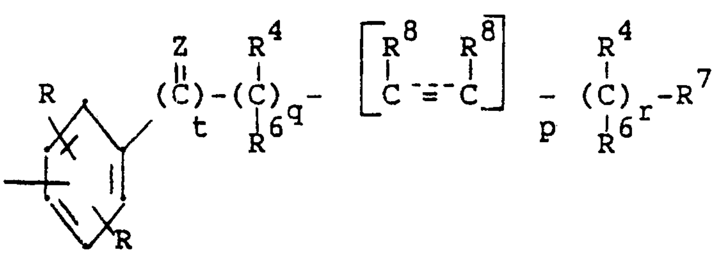

wherein the broken line represents an optional triple bond,

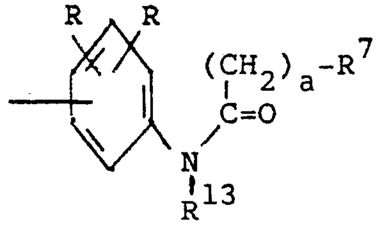
,

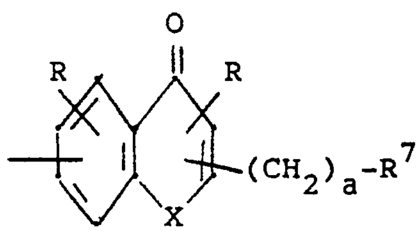
,

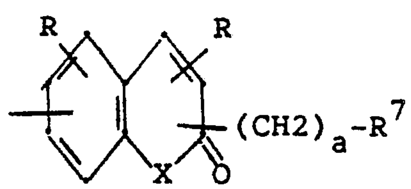
,

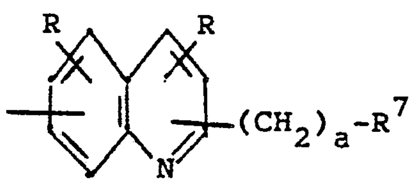
,

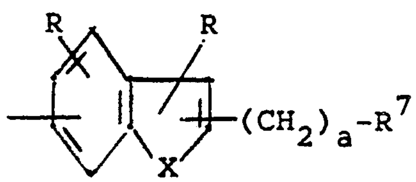
, or

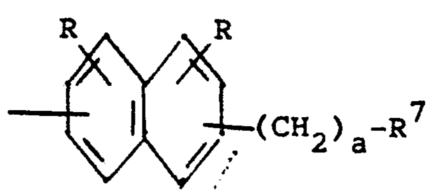
;

each $R^{10}$ is independently OH; $N(R^4)_2$; $CF_3$; lower alkyl; lower alkoxy; phenyl; or phenyl substituted by one or more alkyl or alkoxy groups of 1 to 3 carbon atoms, halogen, hydroxy, $COOR^4$, CN, formyl or lower alklacyl;

each $R^{11}$ is independently

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical $W-R^{12}$;

each $R^{12}$ independently contains up to 20 carbon atoms and is (1) an alkyl radical or (2) an alkylacyl radical of an organic acyclic or monocyclic carboxylic acid containing not more than one heteroatom in the ring;

each $R^{13}$ is independently H or $R^{10}$.
each W is independently O, S or NH;
each X is independently O, S or $NR^{13}$;
$X^1$ and $X^2$ are each independently O, S, SO, $SO_2$, $S(O)=NR^4$, $NR^4$, $NCOR^1$, NCN, or $NCONHR^4$;
$X^3$ is O, S, SO or $SO_2$;
$Y^1$ is OH or the N-terminus of an amino acid such that $Y^1H$ is an essential amino acid:
$Y^2$ is H or the C-terminus of an amino acid such that $Y^2OH$ is an essential amino acid;
Z is O, H and OH, or H and $R^4$;
each a is independently 0 to 4;
each b is independently 1 to 6;
each n is independently 0 to 6;
each p is independently 0 to 2;
each q is independently 0 to 4;
each r is independently 0 to 4;
each s is independently 0 to 3;
each t is independently 0 to 1;
each Het is independently a heterocyclic or heterobicyclic ring of 5 or 6 atoms each, containing one or more heteroatoms selected from O, N or S, said heterocyclic or heterobicyclic ring containing an acidic proton;
or the pharmaceutically acceptable salts thereof.

Compounds of the present invention have activity as leukotriene and SRS-A antagonists or inhibitors. Because of their activity as leukotriene antagonists or inhibitors, the compounds of the present invention are useful as anti-asthmatic, anti-allergic, anti-psoriatic, and anti-inflammatory agents and are useful in treating allergic conjunctivitis, allergic rhinitis, and chronic bronchitis and for amelioration of skin diseases like psoriasis and atopic eczema. These compounds are also useful to antagonize or inhibit the pathologic actions of leukotrienes on the cardiovascular and vascular systems for example, actions such as result in angina. The compounds are also useful as cytoprotective agents.

Thus, the compounds of the present invention may also be used to treat or prevent mammalian (especially, human) disease states such as erosive gastritis, erosive esophagitis, inflammatory bowel disease; ethanol-induced hemorrhagic erosions; hepatic ischemia; noxious agent induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile salt induced pancreatic or gastric damage; trauma- or stress-induced cell damage; and glycerol-induced renal failure.

Examples of useful heterocyclic rings (represented by Het above) include:

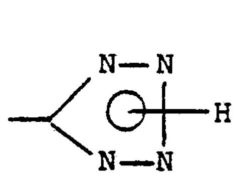 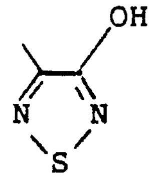 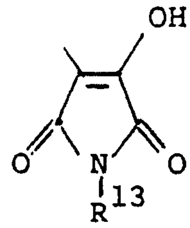

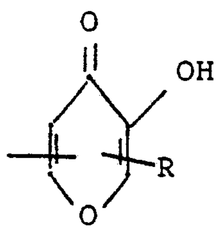 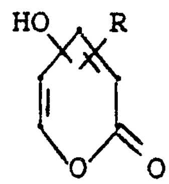 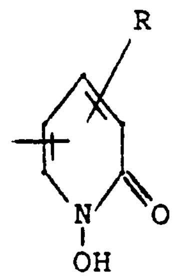

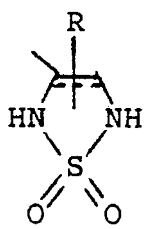 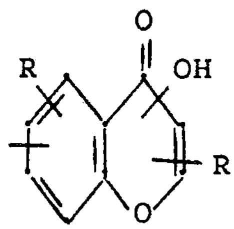 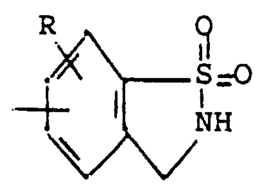

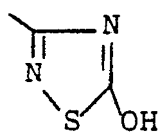 ;

wherein the broken line represents an optional double bond.

As used herein, the term "lower alkyl" means those alkyl groups of from 1 to 7 carbon atoms of either a straight, branched or cyclic structure. Examples of lower alkyl fragments include methyl, ethyl, propyl, isopropyl, butyl sec- and tert-butyl, pentyl, hexyl, heptyl, cyclopropyl, cyclobutyl, cyclopentyl, cyclohexyl, cycloheptyl and norbornyl.

As used herein, the term alkyl includes lower alkyl and extends to cover carbon fragments having up to 20 carbon atoms in straight, branched or cyclic structures. Examples of alkyl groups include octyl, nonyl, undecyl, dodecyl, tridecyl, tetradecyl, pentadecyl, eicosyl, 3,7-ethyl-2,2-methyl-4-propylnonyl, cyclododecyl and amantyl. The terms "lower alkyl" and "alkyl" also include groups having both straight chain and cyclic structures or both branched chain and cyclic structures.

As used herein, the term aryl includes the carbon containing aromatic structures such as phenyl, naphthyl, anthracentyl, phenanthrenyl, pyrenyl, phenyl substituted with one or more alkyls, naphthyl substituted with one or more alkyls, anthracenyl substituted with one or more alkyls and phenanthrenyl substituted with one as more alkyls.

As used herein, the term halogen includes F, Cl, Br and I.

As used herein, the term "lower alkenyl" means those alkenyl groups of from 2 to 7 carbon atoms of either a straight, branched or cyclic configuration. Examples of lower alkenyl groups include vinyl, allyl, isopropenyl, pentenyl, hexenyl, heptenyl, cyclopropenyl, cyclobutenyl, cyclopentenyl, cyclohexenyl, cycloheptenyl, 1-propenyl, 2-butenyl, 2-methyl-2-butenyl and norbornenyl.

As used herein, the term "lower alkoxy" means those alkoxy groups of from 1 to 7 carbon atoms of either a straight, branched or cyclic configuration. Examples of lower alkoxy groups include methoxy, ethoxy, propoxy, isopropoxy, butoxy sec- and tert-butoxy, pentyloxy, hexyloxy, heptyloxy, cyclopropyloxy, cylobutyloxy, cyclopentyloxy, cyclohexyloxy, cycloheptyloxy and norbornyloxy.

As used herein, the term "alkoxy" includes "lower alkoxy" and extends to cover groups having up to 20 carbon atoms in straight, branched or cyclic configurations. Examples of alkoxy groups include octyloxy, nonyloxy, undecyloxy, dodecyloxy, tridecyloxy, tetradecyloxy, pentadecyloxy, eicosyloxy, 3,7-ethyl-2,2-methyl-4-propylnonyloxy, cyclododecyloxy, adamantyloxy and the like.

As used herein, the term "acyl" refers to the carbonyl radical of a carboxylic acid. It will usually be further specified as "alkylacyl"

$$(\text{alkyl-}\overset{O}{\overset{\|}{C}}\text{-}),$$

"lower alkylacyl"

$$(\text{lower alkyl-}\overset{O}{\overset{\|}{C}}\text{-}),$$

"arylacyl"

$$(\text{aryl-}\overset{O}{\overset{\|}{C}}\text{-}),$$

etc., wherein the terms alkyl, lower alkyl and aryl have the meaning given above.

The term essential amino acid is employed to include the following amino acids; alanine, asparagine, aspartic acid, arginine, cysteine, glutamic acid, glutamine, glycine, histidine, isoleucine, leucine, lysine, methionine, phenylalanine, proline, serine, threonine, tryptophan, tyrosine and valine.

In those instances when asymmetric centers are present, more than one stereoisomer is possible, and all possible isomeric forms are deemed to be included within the planar structural representations shown. Optically active (R) and (S) isomers may be resolved using conventional techniques known to the skilled artisan.

A preferred embodiment of the present invention relates to compound of the formula I wherein:

each R, each $R^4$, $R^6$, $R^9$, each $R^{10}$, each $R^{13}$, each

X, $Y^1$, $Y^2$, Z, each a, each b, each q, each r, t and each Het is as defined above for formula I;

$R^1$ is H or lower alkyl;

$R^2$ is H;

each $R^3$ is independently lower alkyl, lower alkenyl;

each $R^5$ is independently H; $OR^2$; or both $R^5$'s may be combined to create a doubly bonded oxygen (=O);

each $R^7$ is independently $COOR^4$; CHO; $CH_2OH$; tetrazole; or Het;

$X^1$, $X^2$ and $X^3$ are each independently O, S, SO or $SO_2$;

each n is independently 1 or 2; and each p is O; or the pharmaceutically acceptable salts thereof.

A more preferred embodiment of the present invention relates to compounds of the formula I wherein:

each R is independently H; OH; lower alkyl; trifluoromethyl; lower alkoxy; halogen; $N(R^4)_2$; $-(C=O)R^1$; $CH_2OR^4$; CN; $SR^{10}$; $SOR^{10}$; or $SO_2R^{10}$;

$R^1$ is lower alkyl;

each n is 1; q=O; and

each of the other substituents is as defined for the aforementioned preferred embodiment, or the pharmaceutically acceptable salts thereof.

A still more preferred embodiment of the present invention are compounds of the formula II:

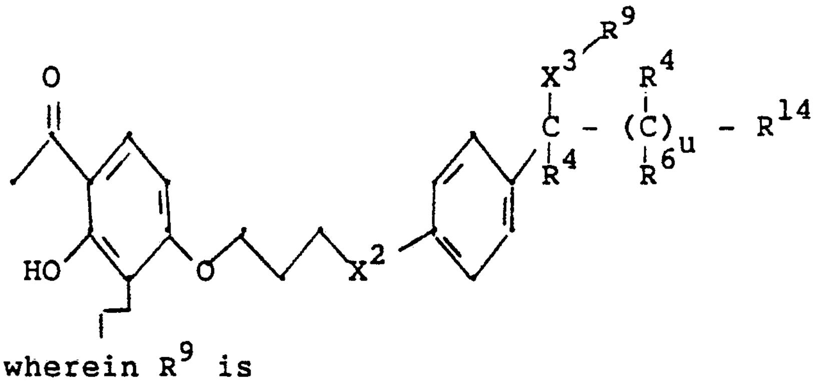

(II)

wherein $R^9$ is

$$(CH_2)_a\text{-Het},$$

where Het is

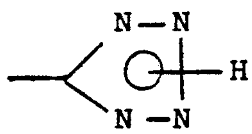

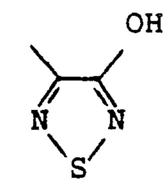

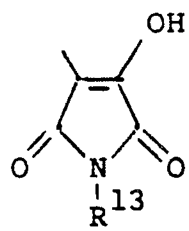

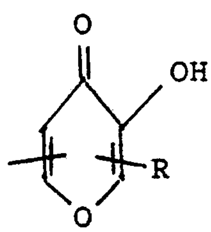

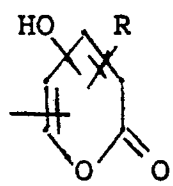

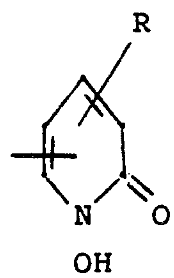

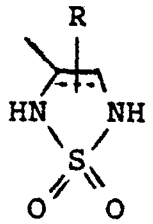 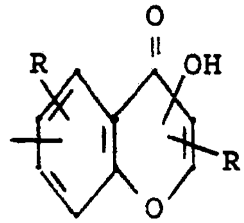 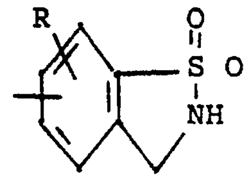

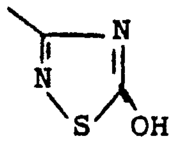 ; wherein the broken line

represents an optional double bond,

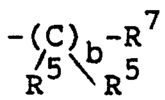 , 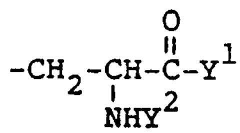 ,

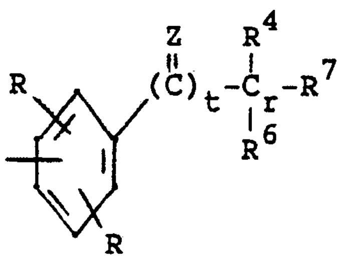 , 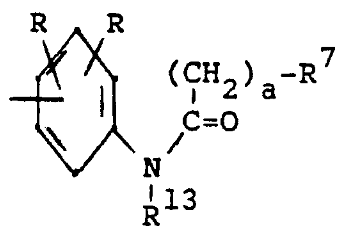 ,

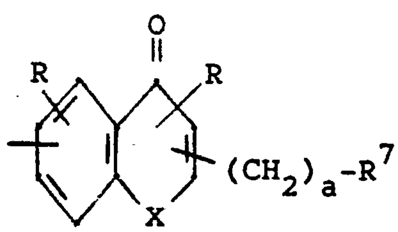 , 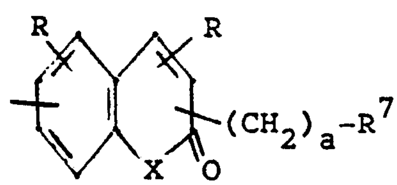 ,

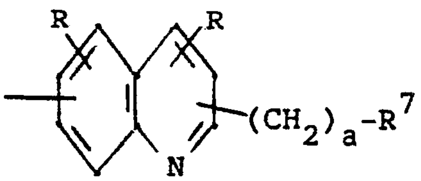 , 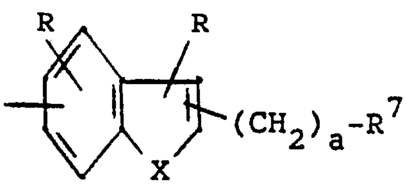 , or

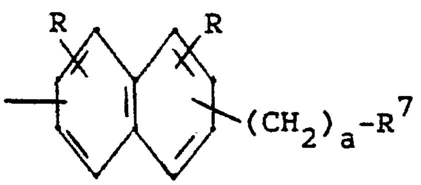 ;

$R^{14}$ is $COOR^4$, u is 0 to 4, and each of the other substituents is as defined for the aforementioned more preferred embodiment,
or the pharmaceutically acceptable salts thereof.

Preferred definitions of Het for compounds of Formula II are:

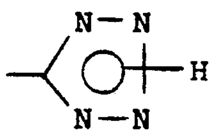 , 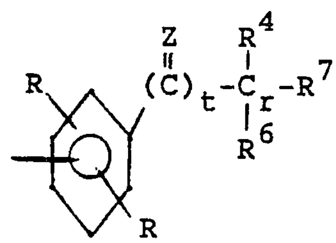 ,

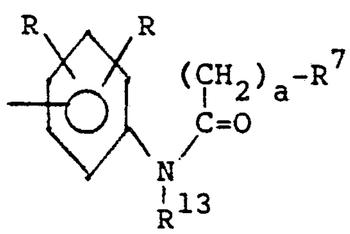 , or 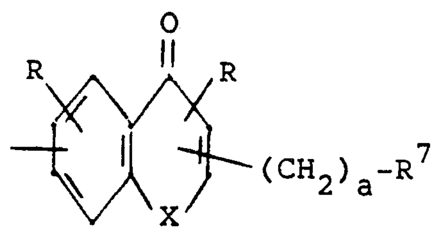 .

The compounds of the present invention may be prepared by several different routes. According to one method, a hydroxy acid salt of formula X, as described in EP 104,885 (April 4, 1984), converted to the free acid form by acidification and extraction into an organic solvent such as ethyl acetate or ether, and then treated with excess diazomethane to form the ester of formula XI. The ester (XI) is treated with a thiol in 1,2-dichloroethane, or similar inert solvent, in the presence of zinc iodide, or similar Lewis acid catalysts, to form the sulfides of formula XII. Hydrolysis with aqueous base provides the diacids of formula XIII. Hydrolysis to the diacids of formula XIII will cause a conversion to a carboxylic acid or a hydroxycarboxylic acid if $SR^9$ of formula XII contains an ester or a lactone ring. This synthetic route is illustrated below:

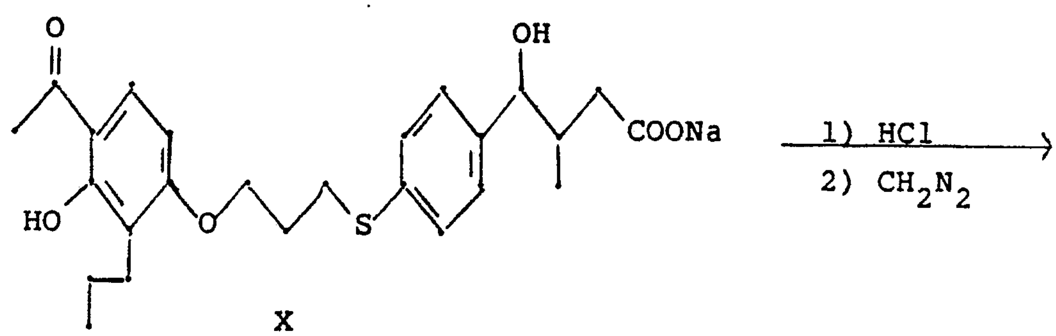


X

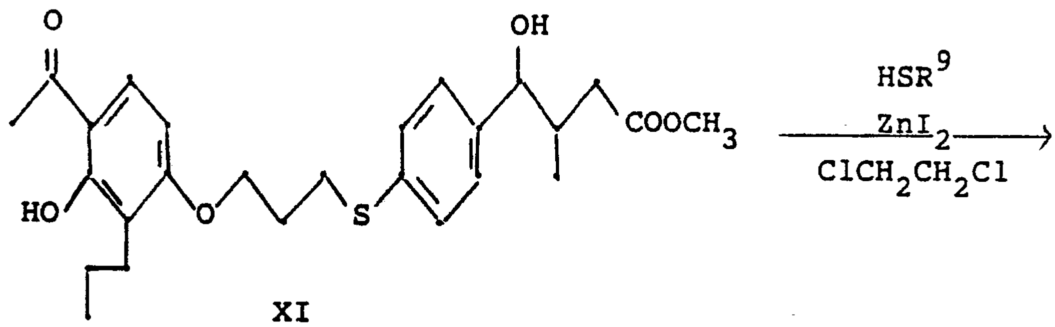


XI

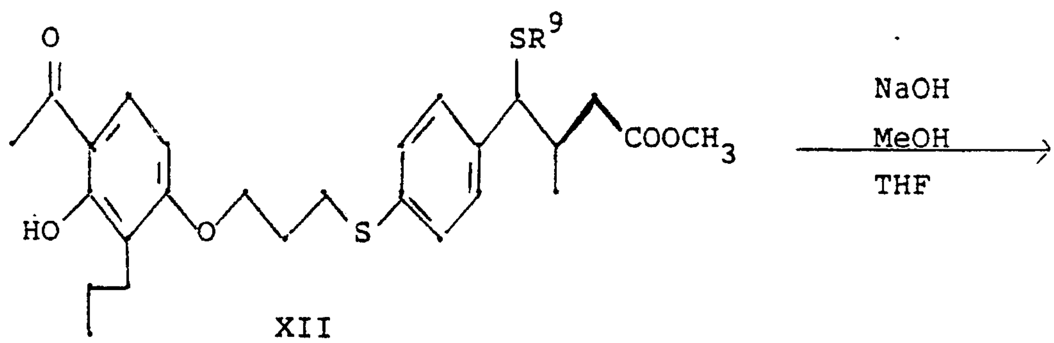


XII

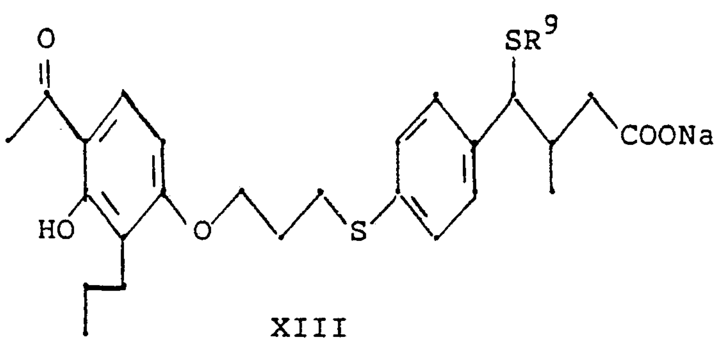

XIII

An alternative procedure is provided whereby the ester of formula XI is reacted with thiolacetic acid and zinc iodide (or similar Lewis acid catalysts) in dichloroethane or a similar inert solvent to provide the thiolacetate of formula XV. Reaction of XV with sodium methoxide in methanol followed by acidification gives the ester of formula XVI. Reaction of XVI with a strong base such as sodium hydride in an inert solvent such as THF at low temperature provides the thiolactones (XVIIa, b) which are separated by chromatography on silica gel or the like. Treatment of one isomeric thiolactone, for example XVIIb, with sodium methoxide followed by reaction of the thiolate anion with a reactive ω-halo-alkanoic acid ester or an alpha,beta-unsaturated alkenoic acid ester, provides the adduct XII which is converted to the diacid salts (XIII) by aqueous alcoholic hydrolysis. Alternatively, the lactone (e.g. XVIIb) can be oxidized with m-chloroperbenzoic acid (mCPBA), or other peracids, or hydrogen peroxide, to provide the sulfones of formula

XVIII. Treatment of XVIII, as shown above for XVIIb, provides the diacid salts of formula XX. This procedure is illustrated below:

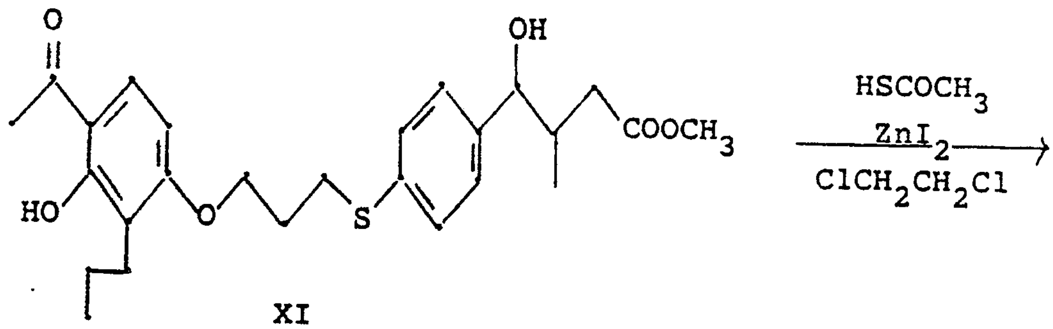


XI

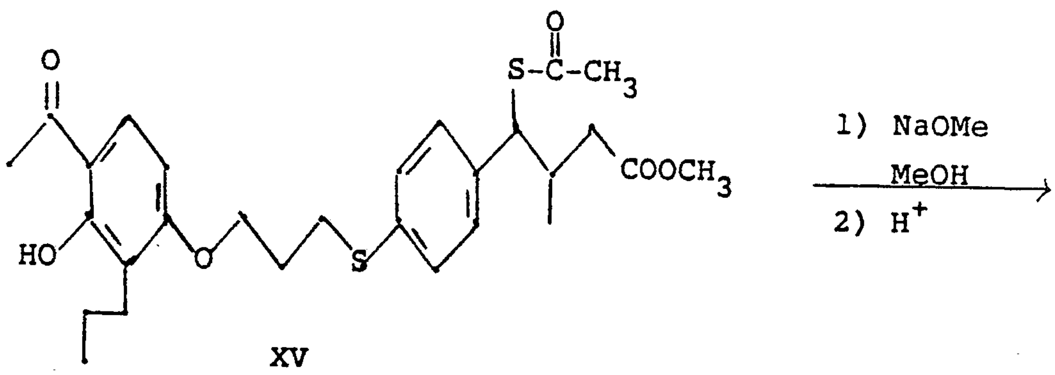


XV

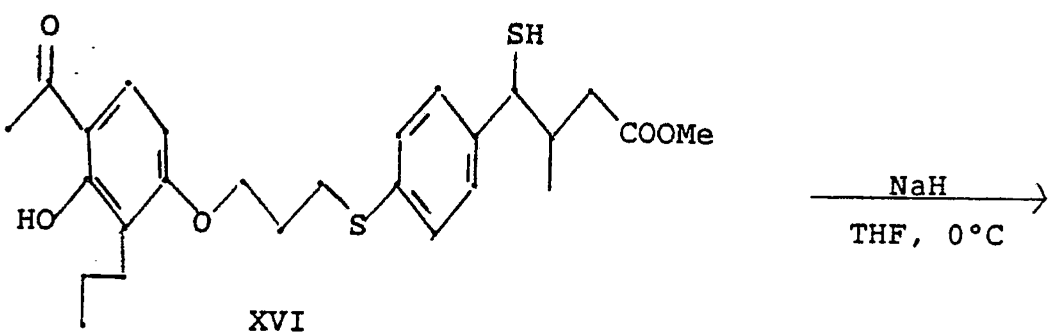


XVI

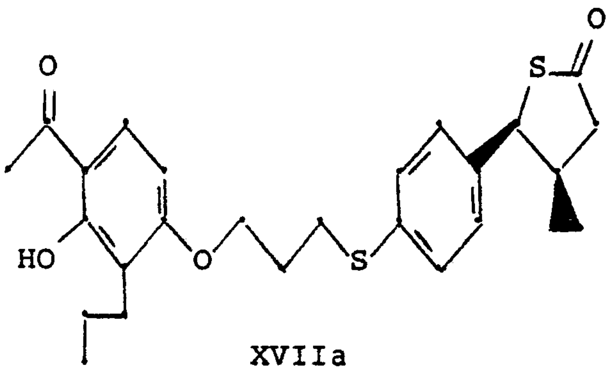

XVIIa

+

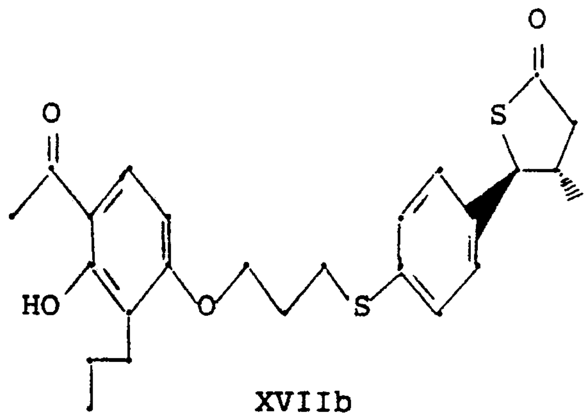

XVIIb

XVIIa
& XVIIb

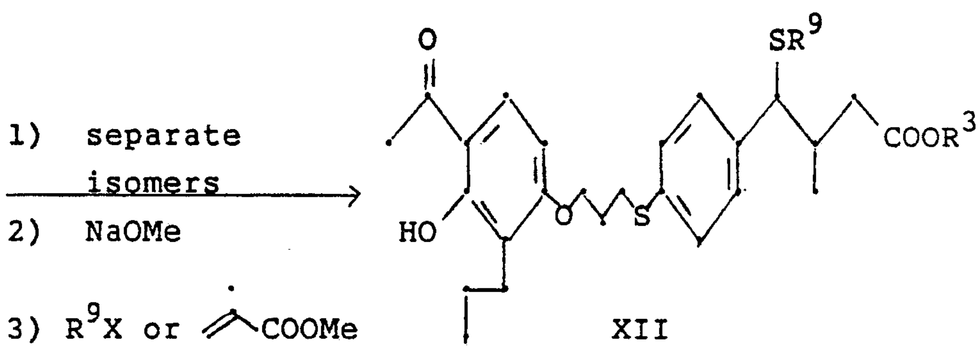


XII

where X is a good leaving group, such as Br, I, etc.

XII XIII
hydrolysis

XVIIb 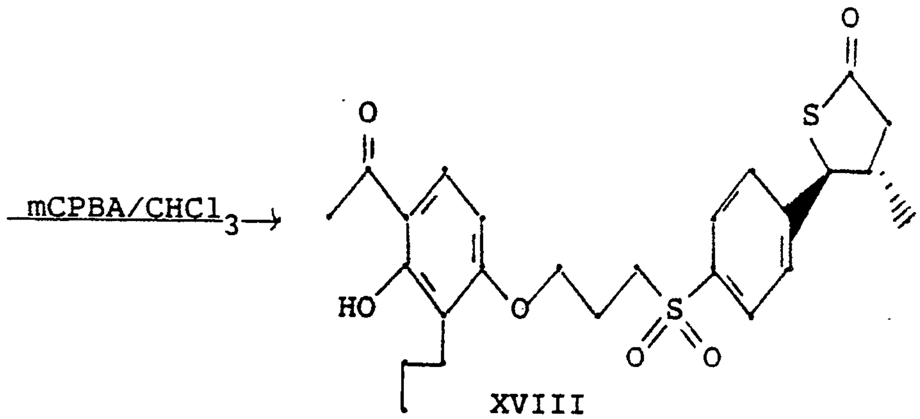

XVIII

XVIII 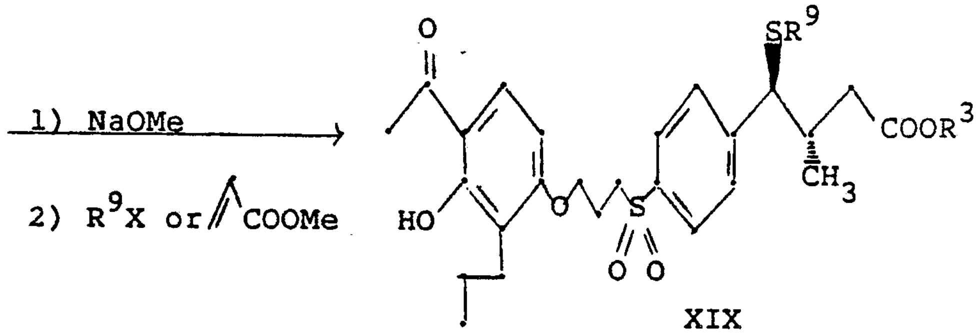

XIX

XIX 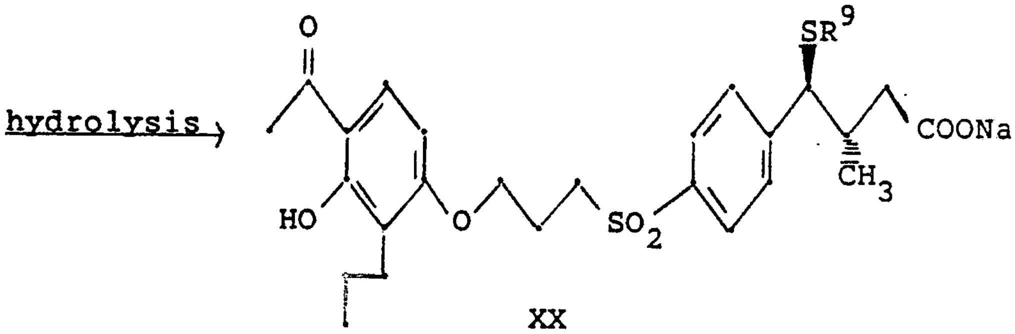

XX

The compounds of Formula I are active as antagonists of SRS-A and the leukotrienes $C_4$, $D_4$ and $E_4$. These compounds also have modest inhibitory activity on leukotriene biosynthesis but are primarily of therapeutic interest as antagonists. The activity of the compounds of Formula I can be detected and evaluated by methods known in the art. See for example, Kadin , U.S. Patent No. 4,296,129.

The ability of the compounds of Formula I to antagonize the effects of the leukotrienes makes them useful for inhibiting the symptoms induced by the leukotrienes in a human subject. The compounds are valuable therefore in the prevention and treatment of such disease states in which the leukotrienes are the causative factor, e.g. skin disorders, allergic rhinitis, and obstructive airway diseases. The compounds are particularly valuable in the prevention and treatment of allergic bronchial asthma. It will be understood that in this paragraph and in the discussion of methods of treatment which follows, references to the compounds of Formula I are meant to include the pharmaceutically acceptable salts and lactone forms.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay.

A. Ethanol-Induced Gastric Ulcer Assay

Twenty-four hour fasted Sprague-Dawley (S.D.) rats are perorally (p.o.) dosed with 1.0 ml absolute ethanol. Fifteen to thirty minutes prior to ethanol administration, groups of rats each receive either an aqueous vehicle (aqueous methylcellulose 5% wt.) or the test compound at various doses perorally. One hour later, the animals are sacrificed and stomach mucosae are examined for resulting lesions.

B. Indomethacin-Induced Ulcer Assay

Indomethacin, 10 mg/kg p.o., is used to induce ulcers in 24 hour fasted S.D. rats. Fifteen minutes prior to indomethacin administration, groups of rats each receive either an aqueous vehicle (5% by weight methylcellulose) or the test compound at various doses perorally. Four hours later the animals are sacrificed and stomach mucosae are examined for resulting ulcers.

The magnitude of a prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range for anti-asthmatic, anti-allergic or anti-inflammatory use and generally, uses other than cytoprotection, lie within the range of from about 0.1 mg to about 40 mg per kg body weight of a mammal, preferably 0.2 mg to about 20 mg per kg, and most preferably 1 to 10 mg per kg, in single or divided doses. On the other hand, it may be necessary to use dosages outside these limits in some cases.

The exact amount of a compound of the Formula I to be used as a cytoprotective agent will depend on, inter alia, whether it is being administered to heal damaged cells or to avoid future damage, on the nature of the damaged cells (e.g., gastrointestinal ulcerations vs. nephrotic necrosis), and on the nature of the causative agent. An example of the use of a compound of the Formula I in avoiding future damage would be co-administration of a compound of the Formula I with a non-steroidal antiinflammatory drug (NSAID) that might otherwise cause such damage (for example, indomethacin). For such use, the compound of Formula I is administered from 30 minutes prior up to 30 minutes after administration of the NSAID. Preferably, it is administered prior to or simultaneously with the NSAID (for example, in a combination dosage form).

The effective daily dosage level for compounds of Formula I inducing cytoprotection in mammals, especially humans, will generally range from about 0.02 mg/kg to about 100 mg/kg, preferably from about 0.2 mg/kg to about 20 mg/kg. The dosage may be administered in single or divided individual doses.

Any suitable route of administration may be employed for providing a mammal, especially a human with an effective dosage of a leukotriene antagonist. For example, oral, rectal, transdermal, parenteral, intramuscular, intravenous and the like may be employed. Dosage forms include tablets, troches, dispersions, suspensions, solutions, capsules and the like.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, N-ethyl morpholine, N-ethyl piperadine, hydrabamine, morpholine, procaine, 2-dimethylaminoethanol, 2-diethylaminoethanol, tromethamine, lysine, arginine, N,N'-dibenzylethylenediamine, histidine, caffeine, procaine, hydrabamine, choline, betaine, ethylenediamine, glucosamine, methylglucamine, theobromine, purines, piperazine, piperidine, N-ethylpiperidine and polyamine resins.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, panoic, pantothenic, phosphoric, succinic, sulfuric, tataric, p-tolnenesulfonic and the like. Particularly preferred are citric, hydrobromic, hydrochloric, maleic, phosphoric, sulfuric and tartaric acids.

The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range for anti-asthmatic, anti-inflammatory or anti-allergic use is from about 0.1 mg to about 20 mg (preferably from about 0.1 mg to about 10 mg) of a compound of formula I per kg of body weight per day and for cytoprotective use from about 0.02 mg to about 40 mg (preferably from about 0.2 mg to about 20 mg and more preferably from about 1 mg to about 10 mg) of a compound of Formula per kg of body weight per day. In the case where an oral composition is employed, a suitable dosage range for anti-asthmatic, anti-inflammatory or anti-allergic use is, e.g. from about 1 mg to about 40 mg of a compound of formula I per kg of body weight per day, preferably from about 5 mg to about 20 mg per kg and for cytoprotective use from about 0.2 mg to about 40 mg (preferably from about 0.2 mg to about 20 mg and more preferably from about 0.2 mg to about 10 mg) of a compound of Formula I per kg of body weight per day.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

In practical use, the compounds of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or intravenous. In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the compounds of Formula I may also be administered by controlled release means and/or delivery device such as those described in U.S. Patent Specifications US-A-3,845,770; 3,916,899; 3,536,809; 3,598,123; 3,630,200 and 4,008,719.

Pharmaceutical compositions of the present invention suitable for oral administration and by inhalation in the case of asthma therapy may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Molded tablets may be made by molding in a suitable machine, a mixture of the powdered compound moistened with an inert liquid diluent. Desirably, each tablet contains from about 25 mg to about 500 mg of the active ingredient and each cachet or capsule contains from about 25 mg to about 500 mg of the active ingredient.

The following are examples of representative pharmaceutical dosage forms for the compounds of Formula I:

| Injectable Suspension | mg/ml |
|---|---|
| Compound of Formula I | 2.0 |
| Methylcellulose | 5.0 |
| Polysorbate | 0.5 |
| Benzyl alcohol | 9.0 |
| Methyl paraben | 1.8 |
| Propyl paraben | 0.2 |
| Water for injection to a total volume of 1 ml | |

| Tablet | mg/tablet |
|---|---|
| Compound of Formula I | 25.0 |
| Microcrystalline Cellulose | 325.0 |
| Polyvinylpyrrolidone | 14.0 |
| Microcrystalline Cellulose | 90.0 |
| Pregelatinized Starch | 43.5 |
| Magnesium Stearate | 2-2.5 |
| | 500 |

| Capsule | mg/capsule |
|---|---|
| Compound of Formula I | 25.0 |
| Lactose Powder | 573.5 |
| Magnesium Stearate | 1.5 |
| | 600 |

In addition to the compounds of Formula I, the pharmaceutical compositions of the present invention can also contain other active ingredients, such as cyclooxygenase inhibitors, non-steroidal anti-inflammatory drugs (NSAIDs), peripheral analgesic agents such as zomepirac diflunisal and the like. The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I or XII to the NSAID will generally range from about 200:1 to about 1:200. Combinations of a compound of the Formula I and other active ingredients will generally also be within the aforementioned range, but in each case, an effective dose of each active ingredient should be used.

NSAIDs can be characterized into five groups:

(1) the propionic acid derivatives;

(2) the acetic acid derivatives;

(3) the fenamic acid derivatives;

(4) the biphenylcarboxylic acid derivatives;

and

(5) the oxicams

or a pharmaceutically acceptable salt thereof.

NSAIDS which are within the scope of this invention are those disclosed in EP-A-0,140,684.

Pharmaceutical compositions comprising the Formula I compounds may also contain inhibitors of the biosynthesis of the leukotrienes such as are disclosed in European Patent Specification EP-A-0,138,481, EP-A-O,115,394, EP-A-0,136,893 and EP-A-0,140,709.

The compounds of the Formula I may also be used in combination with leukotriene antagonists such as those disclosed in EP-A-0,106,565, EP-A-0,104,885, EP-A-0,056,172 and 0,061,800; and in UK Patent

Specification GB-A-2,058,785.

Pharmaceutical compositions comprising the Formula I compounds may also contain as the second active ingredient, antihistaminic agents such as benadryl, dramamine, histadyl and phenergan. Alternatively, they may include prostaglandin antagonists such as those disclosed in European Patent Application EP-A-0,011,067 or thromboxane antagonists such as those disclosed in US-A-4,237,160. They may also contain histidine decarboxyase inhibitors such as α-fluoromethyl-histidine, described in US-A-4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in EP-A-0,040,696 and like compounds, such as those disclosed in US Patent Specification US-A-4,283,408; 4,362,736; and 4,394,508. The pharmaceutical compositions may also contain a $K^+/H^+$ ATPase inhibitor such as omeprazole, disclosed in US-A-4,255,431.

The following examples are provided to illustrate and aid in the interpretation of the present invention. They are not limiting.

Infrared (IR) spectra were measured as KBr disks or as thin films and absorption bands are reported in reciprocal centimeters ($cm^{-1}$). Nuclear magnetic resonance (NMR) spectra (90 MHz) were measured in deuterochloroform ($CDCl_3$), perdeuterodimethyl sulfoxide (DMSO-$d_6$), perdeuteromethanol ($CD_3OD$), deuterium oxide ($D_2O$) or deuterated trifluoroacetic acid ($CF_3COOD$) and peak positions are expressed in parts per million (ppm) downfield from an internal reference, tetramethylsilane. The following abbreviations are used for peak shapes: s, singlet; d, doublet; t, triplet; q, quartet; and m, multiplet. All melting and boiling points are reported in degrees Centigrade (° C) and are uncorrected. Standard abbreviations are used for chemical compounds. For example: THF, tetrahydrofuran; MeOH, methanol; DMF, dimethylformamide; $Et_2O$, diethyl ether; and EtOAc, ethyl acetate.

In the following Examples, R* and S* represent a racemic mixture of RS:SR in the ratio of 1:1.

EXAMPLE 1

Preparation of αR*, βR* and αR*, βS* 7-((α-(4-((3 -(4-acetyl-3-hydroxy-2-propylphenoxy)propyl)thio) phenyl)-γ-carboxy-β-methylpropyl)thio)-4-oxo-4-H-1 -benzopyran-2-carboxylate disodium salt mixture of diastereomers

Step A : Preparation of Methyl 4-((3-(4-acetyl-3 -hydroxy-2-propylphenoxy)propyl)thio)-beta-methyl-gamma-hydroxybenzenebutanoate

To the 4-((3-(4-acetyl-3-hydroxy-2-propyl-phenoxy)propyl)thio)-beta-methyl-gamma-hydroxybenzene butanoic acid γ lactone (8.84 g, 20 mM) described in EP 104,885 (April 4, 1984) was added a mixture of 2N NaOH (12 ml, 24 mM), THF (50 ml) and MeOH (20 ml) and the mixture was stirred 12 hours under $N_2$. The reaction mixture was evaporated to dryness and dissolved in water (75 ml) and cooled to 0° C; IN HCl was added dropwise until the pH was lower than 6 and the organic compound was extracted into EtOAc (200 ml). The organic layer was washed with brine (100 ml) and dried with $Na_2SO_4$. The solvent was removed in vacuo with no heating and the residue was dissolved in $Et_2O$ (100 ml) and cooled to 0° C. Diazomethane (in $Et_2O$) was added in excess and the reaction mixture was evaporated to dryness with no heat and dried under high vacuum to yield the title compound as an oil slightly contaminated (by less than 10 percent of the starting lactone).

$^1H$-250-MHz-NMR/$CDCl_3$:

| Delta (ppm) | Number | m |
|---|---|---|
| 12.7 | 1H | s |
| 7.6 | 1H | d |
| 7.35 | 2H | d |
| 7.25 | 2H | d |
| 6.42 | 1H | d |
| 4.6 | 1H | d |
| 4.15 | 2H | t |
| 3.68 | 3H | s |
| 3.1 | 2H | t |
| 2.8-2.95 | 1H | bs |
| 2.65 | 2H | t |
| 2.55 | 3H | s |
| 2.5-2 | 5H | m |
| 1.55 | 2H | m |
| 0.85-1 | 6H | m |

Step B : Preparation of methyl 7-((1-(4-((3-(4-acetyl -3-hydroxy-2-propylphenoxy)propyl)thio)phenyl) -3-methoxycarbonyl)-2-methylpropyl)thio)-4 -oxo-4H-1-benzopyran-2-carboxylate

To a solution of the ester obtained in Step A (950 mg, 2 mM) of this Example in dry dichloroethane (5 ml) was added dry $ZnI_2$ (20 mM, 6.4 g) and the Methyl 7-mercapto-4-oxo-4H-1-benzopyran-2-carboxylate described in EP 123,543 (April 19, 1984) (566 mg) and the suspension was efficiently stirred for 3 hours. To the reaction was added IN HCl (20 ml) and methylenedichloride (25 ml). The organic layer was washed with brine (15 ml) and dried with $Na_2SO_4$. Solvents were removed in vacuo and the residue purified by flash chromatography on silica gel (1:1, hexane:ethyl acetate) and by passing over basic alumina (activity I) with $CH_2Cl_2$. Removal of the solvent yielded the title compound as an oil.

$^1$H-250-MHz-NMR:

| Delta (ppm) | Number | m |
|---|---|---|
| 12.7 | 1H | s |
| 7.85-8 | 1H | dd |
| 7.55 | 1H | d |
| 7.4 | 1H | d |
| 7.0-7.35 | 6H | m, |
| 6.4 | 1H | d |
| 4.5 | 1H | t |
| 4.15 | 2H | t |
| 4 | 3H | s |
| 3.6 | 3H | d |
| 3.1 | 2H | t |
| 2.6 | 7H | m |
| 2-2.4 | 3H | m |
| 1.5 | 2H | m |
| 1 and 1.1 | 3H | 2d |
| 0.9 | 3H | t |

Step C : Preparation of αR*, βR* and αR*, βS* 7-((α-(4-((3-(4-acetyl-3-hydroxy-2-propylphenoxy) propyl) thio) phenyl)-γ-carboxy-β-methylpropyl) thio)-4-oxo-4-H-1-benzopyran-2-carboxylic acid disodium salt mixture of diastereomers

To a solution of the diester obtained in Step B of this Example (188 mg), MeOH (2 ml), and $H_2O$ (2 ml) was added IM $Na_2CO_3$ (2 ml) and the mixture was stirred for 96 hours at room temperature. The mixture was then cooled to 0° C and IM NaOH (235 £1) was added. The reaction was then stirred for another 48 hours at 5° C. The reaction mixture was evaporated to dryness and absorbed on XAD-8 neutral resin in water, washed with water and then eluted off with ethanol. Evaporation of the solvent *in vacuo* yielded the title compound.

$^{1}H$-250 MHz-NMR/DMSO-$d_6$:

| Delta (ppm) | Number | m |
|---|---|---|
| 12.9 | 1 | broad s |
| 6.6-7.9 | 10 | m |
| 4.8-4.9 | 1 | t |
| 4.1-4.3 | 2 | t |
| 3.1 | 2 | t |
| 0.8-2.9 | 18 | m |

EXAMPLE 2

Preparation of d,1 4-((1-(4((3-(4-acetyl-3-hydroxy-2 -propylphenoxy)propyl)thio)phenyl)-3-carboxypropyl) thio)-gamma-oxobenzenebutanoic acid

Step A : Preparation of Methyl 4(3-bromopropylthio) -gamma-hydroxy-benzenebutanoate

To methyl 4(3-bromopropylthio)-gamma-oxo-benzene butanoate (described in EP 104,885 (April 4, 1984)) (17.26 g, 50 mM) in 1,2-dimethoxy ethane (100 ml) and MeOH (100 ml) cooled to 0° C, was added $CeCl_3$ (10 mg) and $NaBH_4$ (945 mg) portion-wise, over 1/2 hours and the reaction mixture was maintained at 0° for an additional 1/2 hour. Thereafter, $NaBH_4$ (300 mg) was added and reaction stirred for another 1/2 hour at 0° C. The reaction was slowly poured on cold IN HCl (200 ml) and extracted with ethyl acetate (300 ml). The organic layer was then washed with brine, dried with $Na_2SO_4$ and solvents were removed in vacuo at less than 30° C to yield the title compound as an oil which as used immediately in Step D.

Step B : Preparation of Methyl 4-(methylthio)-gamma -oxo-benzenebutanoate

Thioanisole (12.4 g) and 1,4-dioxo-4-methoxy-butyl chloride (16.5 g) in dichloroethane (100 ml) were cooled to 0° C and aluminum chloride (16 g) was added, followed by another equivalent (16 g) and the reaction mixture was stirred at 0° C for 2 hours. Ice was added, followed by IN HCl. Dichloromethane (100 ml) was then added and the organic layer was separated, washed with water and then brine and dried with $Na_2SO_4$. Removal of the solvents yielded the title compound as an oil.

Analysis calculated: C, 60.48; H, 5.92; S, 13.45.

Found: C, 60.50; H, 5.99; S, 13.27.

Step C : Preparation of Methyl (4-mercapto)-gamma -oxo-benzenebutanoate

To a solution of Methyl (4-methylthio)-gamma-ketobenzene-butanoate (476 mg) in chloroform (15 ml), cooled to 0° C, was added m-chloroperoxy-benzoic acid (345 mg) and the mixture was stirred at 0° C for 1

hour. Excess $Ca(OH)_2$ was added. The mixture was stirred 0.5 hours at room temperature. Thereafter, filtration through Celite and removal of the solvent left an oily residue of the sulfoxide. Trifluoroacetic anhydride (20 ml) was then added and the mixture was warmed to 40° C for 15 minutes. Removal of the volatiles left a residue to which was added 50 ml of a 1:1 mixture of MeOH and triethylamine. Solvents were removed in vacuo and further methanol-triethylamine was added and this process was repeated two times. Ethyl acetate (25 ml) was added to the resulting residue. The solution was washed with 1N HCl (10 ml) and then with brine (20 ml) and the organic layer was dried with $Na_2SO_4$. Removal of the solvents yielded the title compound which was immediately used in the coupling reaction described in the following step (to avoid formation of the disulfide).

Step D : Preparation of methyl 4((1-(4-(3-bromo -propylthio)phenyl)-3-(methoxy carbonyl) propyl)thio)-gamma-oxo-benzenebutanoate

To a well stirred suspension of the alcohol obtained in Step A of this Example (694 mg) and $ZnI_2$ (6.4 g) in dichloroethane (10 ml), was added the thiol obtained in Step C of this Example (448 mg) and the mixture was stirred for 3 hours. It was then quenched with IN HCl (10 ml) and diluted with $CH_2Cl_2$ (20 ml). The organic layer was then washed with IN HCl (10 ml) and then with brine (10 ml) and dried with $Na_2SO_4$. Removal of the solvent yielded an oily residue which was purified by flash chromatography (toluene:ethylacetate, 10:1) to yield the pure title compound.

$^1H$-250-MHz-NMR/$CDCl_3$:

| Delta (ppm) | Number | m |
|---|---|---|
| 7.9 | 2H | d |
| 7.3 | 2H | d |
| 7.25 | 4H | s |
| 4.4 | 1H | m |
| 3.75 | 3H | s |
| 3.7 | 3H | s |
| 3.52 | 2H | t |
| 3.25 | 2H | t |
| 3.1 | 2H | t |
| 2.75 | 2H | t |
| 2-2.7 | 6H | m |

Step E : Preparation of methyl 4-((1-(4-(3 -(4-acetyl-3-hydroxy-2-propylphenoxy)propyl) thio)phenyl)-3-(methoxycarbonyl)propyl) thio)-gamma-oxo-benzenebutanoate

A suspension of the bromide obtained in Step D of this Example (553 mg), 2,4-dihydroxy-3-propyl acetophenone (235 mg), potassium carbonate (400 mg, milled) and methyl ethyl ketone (20 ml) was refluxed for 6 hours. The reaction mixture was then cooled to room temperature and the insolubles removed by filtration through Celite. Solvents were removed in vacuo and the residue was purified on Prep-500 Waters apparatus using toluene: ethyl acetate, 10:1, to yield the title compound.

$^1$H-250 MHz-NMR:

| Delta (ppm) | Number | m |
|---|---|---|
| 12.75 | 1H | s |
| 7.80 | 2H | d |
| 7.55 | 1H | d |
| 7.35 | 2H | d |
| 7.2-7.3 | 4H | m |
| 6.4 | 1H | d |
| 4.4 | 1H | m |
| 4.15 | 2H | t |
| 3.6-3.7 | 6H | 2s |
| 3.25 | 2H | t |
| 3.15 | 2H | t |
| 2.7-2.8 | 2H | t |
| 2.6-2.7 | 2H | t |
| 2.58 | 3H | s |
| 2-2.45 | 6H | m |
| 1.45-1.55 | 2H | m |
| 0.95 | 3H | t |

Step F : Preparation of 4-((1-(4-((3-(4-acetyl-3 -hydroxy-2-propylphenoxy)propyl)thio)phenyl) -3-carboxypropyl)thio)-gamma-oxo-benzene -butanoic acid

A solution of the diester obtained in Step E of this Example (922 mg), IN NaOH (4.2 ml), MeOH (4 ml) and THF (4 ml) was stirred, under $N_2$, for 4 hours, at room temperature. The reaction mixture was then evaporated to dryness and the residue mixed with water (20 ml), acidified with IN HCL and extracted with diethyl ether (50 ml). The organic layer was washed with brine and dried with $Na_2SO_4$. The solvent was removed in vacuo to yield the title compound as a white solid which was purified by trituration with ethyl acetate hexane, 1:5.

Analysis calculated: C, 63.93; H, 6.00; S, 10.04.

Found: C, 64.10; H, 5.99; S, 9.73.

EXAMPLE 3

Preparation of $\beta R^*$, $\gamma R^*$ and $\beta S^*$, $\gamma R^*$4-((3-(4-acetyl -3-hydroxy-2-propylphenoxy)propyl)thio)-gamma-(3-((carboxyacetyl)amino)phenyl)thio-Beta-methylbenzene -butanoic acid

Step A : Preparation of ethyl 3-oxo-3-(3-mercapto -phenylamino)propanoate

A mixture of 3-aminothiophenol (5.0 g) and diethyl malonate (6.41 g) was heated under a nitrogen atmosphere for 2 hours at from 165° to 170° C. The mixture was chromatographed on silica gel to yield the title compound, m.p. 52-54°.

Analysis calculated: C, 55.21; H, 5.47; N, 5.85; S, 13.39.

Found: C, 54.64; H, 5.41; N, 5.80; S, 13.02.

Step B : Preparation of Methyl 4-((3-(4-acetyl-3 -hydroxy-2-propylphenoxy)propyl)thio)-gamma -((3-ethoxy-1,3-dioxopropylamino)phenyl)thio) -beta-methylbenzenebutanoate

To a well stirred suspension of the alcohol obtained in Step A, Example 1 (1.422 g) and $ZnI_2$ (6 g) in dichloroethane (25 ml) was added the thiol obtained in step A, Example 3 (550 mg) and the mixture was stirred for 3 hours. Thereafter, 1N HCl (20 ml) was added, followed by dichloromethane (50 ml). The organic layer was separated, washed with brine and then dried with $Na_2SO_4$. The solvents were removed in vacuo to give a residue which was purified by flash chromatography using 33% ethyl acetate in hexane to yield the title compound as an oil (1.76 g, 84%).

$^1H$-250 MHz-NMR/$CDCl_3$:

| Delta (ppm) | Number | m |
|---|---|---|
| 12.75 | 1H | s |
| 9.2 | 1H | bs |
| 7.5 | 1H | d |
| 7.52 | 1H | s |
| 6.8-7.4 | 7H | m |
| 6.4 | 1H | d |
| 4.2-4.35 | 3H | m |
| 4.15 | 2H | t |
| 3.65 | 3H | 2s |
| 3.45 | 2H | s |
| 3.1 | 2H | t |
| 2-2.85 | 10H | m |
| 1.55 | 2H | m |
| 1.3 | 3H | t |
| 0.9-1.15 | 6H | m |

Step C : Preparation of 4-((3-(4-acetyl-3-hydroxy-2 -propylphenoxy)propyl)thio)-gamma-((3-carboxy -acetyl)-amino)phenyl)thio)beta-methylbenzene -butanoic acid

To a cooled (0° C) solution of the ester obtained in Step B of this Example (615 mg), MeOH (5 ml) and THF (5 ml) was added IN NaOH (2.6 ml) and the reaction mixture was allowed to return to room temperature and was stirred for 12 hours. Thereafter, the solvents were removed in vacuo and the residue was diluted with water (10 ml), acidified with IN HCL (5 ml) and extracted with ether (30 ml). The organic layer was washed with brine, dried with $Na_2SO_4$ and evaporated to dryness in vacuo to yield the title compound as a beige foam (520 mg, 90%).

Analysis calculated: C, 62.46; H, 6.01; N, 2.14; S, 9.81.

Found: C, 62.00; H, 6.24; N, 2.07; S, 9.98.

EXAMPLE 4

Preparation of IR*, γR* and IR*, γS*4-((1-(4 -((3-(4-acetyl-3-hydroxy-2-propylphenoxy)propyl)thio) phenyl)-3-carboxypropyl)thio)-beta-methyl-gamma -oxobenzenebutanoic acid (mixture of diastereoisomers)

Step A : Preparation of Methyl (1((4(3-bromopropyl -phenyl)-3-methoxycarbonyl-propyl)thio)-β-methyl-gamma-oxobenzene-butanoate

To a solution of the alcohol obtained in Step A of Example 2 (1.041 g), $ZnI_2$ (9.6 g) and dichloroethane (15 ml), was added methyl 4-mercapto-beta-methyl-gamma-oxobenzenebutanoate from Example 9, Step E (714 mg) under efficient stirring and the reaction mixture was stirred for 3 hours. The reaction mixture was then quenched with water (10 ml) and then diluted with $CH_2Cl_2$ (25 ml). The organic layer was then washed with IN HCl (25 ml) and then with brine and was then dried with $Na_2SO_4$. Removal of the solvents in vacuo yielded a residue which was purified by flash chromatography (10:1, toluene:ethylacetate) to yield the title compound as an oil (1.6 g, 94%).

$^1$H-250 MHz-NMR/$CDCl_3$:

| Delta (ppm) | Number | m |
|---|---|---|
| 7.84 | 2H | d |
| 7.29 | 2H | d |
| 7.24 | 4H | s |
| 4.4 | 1H | m |
| 3.86 | 1H | m |
| 3.64 | 6H | 2s |
| 3.51 | 2H | t |
| 3.07 | 2H | t |
| 2.94 | 1H | m |
| 2.05-2.54 | 7H | m |
| 1.19 | 3H | d |

Step B : Preparation of Methyl 4-((1-(4-((3-(4-acetyl -3-hydroxy-2-propylphenoxy)propyl)thio)phenyl) -3-methoxycarbonylpropyl)thio)-beta-methyl -gamma-oxobenzene butanoate

A solution made of the bromide obtained in Step A of this Example (1.55 g), 2,4-dihydroxy-3-propyl acetophenone (652 mg), and milled $K_2CO_3$ (1.16 g) in MEK (methyl ethyl ketone) (25 ml) was refluxed for 6 hours and then cooled to room temperature. The mixture was filtered and removal of the solvent left a residue which was purified by flash chromatography with 30% ethyl acetate in hexane to yield the title compound as an oil (1.05 g, 55%).

$^1$H-250 MHz-NMR($CDCl_3$):

| Delta (ppm) | Number | m |
|---|---|---|
| 12.75 | 1H | s |
| 7.83 | 2H | d |
| 7.6 | 1H | d |
| 7.45 | 2H | d |
| 7.3 | 4H | s |
| 6.42 | 1H | s |
| 4.4 | 1H | m |
| 4.15 | 2H | t |
| 3.85 | 1H | m |
| 3.65 | 6H | 2s |
| 3.12 | 2H | t |
| 2.65 | 2H | AB |
| 2.1-2.7 | 11H | m |
| 1.55 | 2H | m |
| 1.2 | 3H | d |
| 0.95 | 3H | t |

Step C : Preparation of IR*γR*, IR*γS*4-((1 -(4-((3-(4-acetyl-3-hydroxy-propylphenoxy) propyl)thio)phenyl)-3-carboxypropyl)thio) -beta-methyl-gamma-oxobenzenebutanoic acid (mixture of diastereoisomers)

To a solution of the diester from Step B of this Example (940 mg) in MeOH (4 ml) and THF (4 ml) was added IN NaOH (4.2 ml) and the reaction mixture was stirred for 3 hours at room temperature. Solvents were removed *in vacuo* and the residue mixed with water (20 ml) and acidified with IN NCI (10 ml). The aqueous layer was extracted with ether (50 ml) and the organic layer was washed with brine (10 ml) and then dried with $Na_2SO_4$. The solvents were removed *in vacuo* to give the title compound as a beige foam (740 mg, 82%).

Analysis calculated: C, 64.40; H, 6.18; S, 9.82.

Found: C, 64.34; H, 6.12; S, 9.82.

EXAMPLE 5

Preparation of αR*, βR* and αR*, βS*N(S(α-(4 -((3-(4-acetyl-3-hydroxy-2-propylphenoxy)propyl)thio) phenyl)-γ-carboxy-β-methylpropyl)-cysteinyl)glycine (mixture of diastereoisomers)disodium salt

Step A : Preparation of N(S-(α(4-((3-(4-acetyl-3 -hydroxy-2-propylphenoxy)propyl)thio)phenyl) -γ-methoxycarbonyl-β-methylpropyl)N-trifluoro -acetylcysteinyl)glycine methyl ester (mixture of diastereoisomers)

To a well stirred suspension of the ester obtained in Step A, Example 1 (474 mg), $ZnI_2$ (3.2 g) and dichloroethane (3 ml) was added cysteinyl-glycine methyl ester and the mixture was stirred for 3 hours. Thereafter, water (5 ml), IN HCL (5 ml) and dichloromethane (25 ml) were added. The organic layer was washed with brine and then dried with $Na_2SO_4$. The solvents were removed to yield a residue which was chromatographed to yield the title compound as an oil (436 mg, 59%).

$^1H$-250 MHz/$CDCl_3$:

| Delta (ppm) | Number of H | m |
|---|---|---|
| 12.9 | 1 | s |
| 7.6 | 1 | d |
| 7.4-7.55 | 1 | m |
| 7.15-7.35 | 4 | m |
| 6.6-6.7 | 1 | m |
| 6.4 | 1 | d |
| 4.7-3.5 | 13 | m |
| 3.1-3.2 | 2 | t |
| 2.9-2 | 11 | m |
| 1.5 | 2 | m |
| 0.8-1.1 | 6 | m |

Step B : Preparation of αR*, βS* and αR*, βR* N-(S-(α-(4-( (3-(4-acetyl-3-hydroxy-2-propylphenoxy) propyl)-thio)phenyl)-γ-carboxy-β-methylpropyl) cysteinyl)glycine (mixture of diastereoisomers) disodium salt

To a solution made of the ester obtained in Step A of this Example (390 mg), MeOH (3 ml) and THF (3 ml) was added IN NaOH (2 ml) and the mixture was stirred for 12 hours. Thereafter, solvents were removed and the residue passed on a XAD-8 neutral resin to yield the title compound as a beige foam (275 mg, 80%).

Analysis calculated: C, 51.64; H, 5.63; S, 9.19.

Found: C, 51.44; H, 5.97; S, 8.54.

EXAMPLE 6

Preparation of βR*, αR* and βR*, αS* 4-((3-(4-acetyl-3-hydroxy-2-propylphenoxy)propyl)thio)-gamma-((2-carboxyethyl)thio)-beta-methylbenzenebutanoic acid disodium salt (mixture of diastereoisomers)

Step A : Preparation of Methyl 4-((3-(4-acetyl-3 -hydroxy-2-propylphenoxy)propyl)thio)-gamma -((2-methoxycarbonylethyl)thio)-beta-methyl -benzenebutanoate (mixture of diastereoisomers)

To an efficiently stirred suspension made of the alcohol obtained from Step A of Example 1 (1.42 g), $ZnI_2$ (4.8 g) in dichloroethane (20 ml) was added methyl 3-mercaptopropionate (360 mg) and the suspension was stirred for 3 hours. Thereafter, IN HCl (20 ml) and dichloromethane (50 ml) were added. The organic layer was then washed with brine and dried with $Na_2SO_4$. The solvents were removed in vacuo to yield a residue which was chromatographed on silica gel to yield the title compound as an oil (1.37 g, 80%).

NMR $^1H$-250 MHz/$CDCl_3$:

| Delta (ppm) | Number of H | m |
|---|---|---|
| 12.9 | 1 | s |
| 7.55 | 1 | d |
| 7.2-7.4 | 4 | m |
| 6.45 | 1 | d |
| 4.15 | 2 | t |
| 3.6-3.8 | 1 | m |
| 3.65 | 3 | 2s |
| 3.65 | 3 | s |
| 3.15 | 2 | t |
| 2.8-2.0 | 14 | m |
| 1.55 | 2 | m |
| 0.95 | 6 | m |

Step B : Preparation of βR*, αR* and βR*, αS*4-((3 -(4-Acetyl-3-hydroxy-2-propylphenoxy)propyl) thio-gamma-((2-carboxyethyl)thio)-beta -methylbenzenebutanoic acid disodium salt tetrahydrate (mixture of diastereoisomers)

To a solution made of the ester obtained in Step A of this Example (1.3 g), MeOH (10 ml), and THF (10 ml) was added IN NaOH (6.7 ml) and the mixture was stirred for 12 hours. Thereafter, the solvents were removed in vacuo . The residue was then mixed with water and passed over XAD-8 neutral resin to yield the title compound as a beige foam (780 mg, 60%).

Analysis calculated: C, 50.59; H, 6.37; S, 9.65; Na, 6.92.

Found: C, 50.40; H, 6.31; S, 9.69; Na, 6.36.

EXAMPLE 7

Preparation of βR*, γR* and βR*, γS*4-((3-(4-Acetyl -3-hydroxy-2-propylphenoxy)propyl)thio)-gamma-(butyl - thio)-beta-methylbenzenebutanoic acid (mixture of diastereoisomers)

Step A: Preparation of Methyl 4-((3-(4-acetyl-3 -hydroxy-2-propylphenoxy)propyl)thio)-gamma -(thioacetyl)-beta-methylbenzenebutanoate (mixture of diastereoisomers)

To a solution of the alcohol obtained in Step A, of Example 1 (5.69 g), $ZnI_2$ (19 g) in dichloroethane (50 ml) was added thioacetic acid (1.005 g) with efficient stirring. After 3 hours, IN HCl (25 ml), water (25 ml) and dichloromethane (150 ml) were added. The organic layer was washed with brine and dried with $Na_2SO_4$. The solvents were then removed in vacuo to yield a residue which was chromatographed to yield the title compound as an oil (5.43 g, 85%).

Analysis calculated: C, 63.13; H, 6.81; S, 12.04.

Found: C, 63.01; H, 6.77; S, 11.77.

NMR $^1H$-250 MHz/$CDCl_3$:

| Delta (ppm) | Number of H | m |
|---|---|---|
| 12.9 | 1 | s |
| 7.6 | 1 | d |
| 7.1-7.3 | 4 | m |
| 6.4 | 1 | d |
| 4.5 | 1 | m |
| 4.1 | 2 | t |
| 3.6-3.7 | 3 | 2s |
| 3.15 | 2 | t |
| 2.75-2 | 13 | m |
| 1.5 | 2 | m |
| 0.8-1.1 | 6 | m |

Step B : Preparation of βR*, γR* and βR*, γS* Methyl 4-((3-(4-acetyl-3-hydroxy-2-propylphenoxy) propyl)-

thio)-gamma-(butylthio)-beta-methyl -benzenebutanoate (mixture of diastereoisomers)

To a cooled (0 °C) solution of the thioacetate obtained in Step A of this Example (532 mg) in MeOH (5 ml) was added 2N NaOMe (650 microliters) followed, 1/2 hour later, by l-iodobutane (148 microliters, 240 mg) and the reaction mixture was stirred for 1 hour at 0 °C. IN HCl (5 ml) was added followed by ethyl acetate (25 ml). The organic layer was collected, washed with brine and dried. Solvents were removed *in vacuo* and the residue purified by chromatography to yield the title compound as an oil (465 mg, 85%).

NMR $^1H$-250 MHz/$CDCl_3$:

| Delta (ppm) | Number of H | m |
|---|---|---|
| 12.9 | 1 | s |
| 7.65 | 1 | d |
| 7.1-7.4 | 4 | m |
| 6.4-6.5 | 1 | d |
| 4.15 | 2 | t |
| 3.5-3.8 | 4 | m |
| 3.1-3.2 | 2 | t |
| 2-2.8 | 12 | m |
| 1.2-1.7 | 6 | m |
| 0.75-1.15 | 9 | m |

Step C : Preparation of βR*, γR* and βR*, γS*4-((3 -(4-Acetyl-3-hydroxy-2-propylphenoxy)propyl) thio)-gamma-(butylthio)-beta-methylbenzenebutanoic acid (mixture of isomers)

To a solution of the ester from Step B of this Example (340 mg) in MeOH (3 ml) and THF (3 ml) was added IN NaOH (1.6 ml) and the mixture was stirred for 12 hours. Thereafter, the solvents were removed and the residue mixed with water (10 ml), acidified with IN HCl (5 ml) and extracted with ethyl acetate (25 ml). The organic layer was washed with brine and dried with $Na_2SO_4$. The solvents were removed *in vacuo* to give a residue which was chromatographed to yield the title compound (210 mg, 63%).

Analysis calculated: C, 65.38; H, 7.57; S, 12.04.

Found: C, 65.25; H, 7.68; S, 12.11.

## EXAMPLE 8

Preparation of βR*, γR* and βR*, γS*4-((3-(4 -Acetyl-3-hydroxy-2-propylphenoxy)propyl)thio)-beta -methyl-gamma-((lH-tetrazol-5-ylmethyl)thio)benzene -butanoic acid disodium salt monohydrate
and
Methyl βR*, γR* and βR*, γS*4-((3-(4-acetyl-3 -hydroxy-2-propylphenoxy)propyl)thio)-beta-methyl -gamma-(-(lH-tetrazole-5-yl-methyl)thio)benzenebutanoate (diastereoisomer mixture) sodium salt

Step A : Preparation of Methyl βR*, γR* and βR*, γs * 4-((3-(4-acetyl-3-hydroxy-2-propylphenoxy) propyl)-thio)-beta-methyl-gamma-(cyanomethyl) thio)benzenebutanoate (mixture of isomers)

To a cooled (0° C) solution of the acetate obtained in Step A of Example 7 (2.66 g) in MeOH (30 ml) was added 2N NaOMe (3.25 ml) and the reaction mixture was stirred for 1/2 hour. Thereafter, chloroacetonitrile (491 mg) was added and the mixture stirred for another hour. The mixture was slowly poured on ice cold IN HCl (100 c.c.). Ethyl acetate (100 ml) was then added and the organic layer was separated. The aqueous layer was extracted with ethyl acetate (50 ml) and the combined organic layers were washed with brine and dried with $Na_2SO_4$. The solvents were removed to yield a residue which was chromatographed to yield the title compound as an oil.

NMR $^1H$-250 MHz/$CDCl_3$:

| Delta (ppm) | Number of H | m |
|---|---|---|
| 12.9 | 1 | s |
| 7.6 | 1 | d |
| 7.15-7.35 | 4 | m |
| 6.45 | 1 | d |
| 4.15 | 2 | t |
| 3.85-4.05 | 1 | m |
| 3.5-3.6 | 3 | 2s |
| 3.1-3.2 | 2 | t |
| 2.9-2.0 | 12 | m |
| 1.45-1.6 | 2 | m |
| 0.85-1.2 | 6 | m |

Step B : Preparation of βR*, γR* and βR*, γS*4-((3 -(4-Acetyl-3-hydroxy-2-propylphenoxy)propyl) thio)-beta-methyl-gamma-((IH-tetrazole-5-yl -methyl)thio)benzenebutanoic acid sodium salt monohydrate
and
Methyl βR*, γR* and βR*, γS* 4-((3-(4 -acetyl-3-hydroxy-2-propylphenoxy)propyl) thio)-beta-methyl-gamma-((1H-tetrazole-5-yl -methyl)thio)benzenebutanoate (diastereoisomer mixture) sodium salt

The ester obtained in Step A (2.54 g) and tri-n-butyltin azide (1.75 g) were heated, neat, under $N_2$, to 80° C for 16 hours. Ether (100 ml) saturated with HCl gas was added and the mixture was stirred at room temperature for 3 hours. It was then diluted with ether (100 ml) and stirred with (100 ml) 2N NaOH at room temperature for 30 minutes. The aqueous layer was separated and the ether layer was extracted back with 2N NaOH (100 ml). The combined aqueous layers were acidified and extracted with ethyl acetate. The ethyl acetate solution was dried with brine and sodium sulfate (anhydrous). The solvent was removed to yield an oil which was purified by flash chromatography. This yielded the tetrazole/acid and the tetrazole/ester. Both were converted to their respective sodium salts with sodium hydroxide and purified on XAD-8 neutral resin.

Analysis calculated for $C_{27}H_{29}O_5S_2N_4Na_2\ H_2O$:
C, 52.50; H, 5.06; N, 9.07; S, 10.38; Na, 7.44.
Found: C, 52.39; H, 5.45; N, 8.72; S, 10.54; Na, 7.03.

Analysis calculated for $C_{28}H_{32}O_5S_2N_4Na\ H_2O$:
C, 55.16; H, 5.62; S, 10.52.
Found: C, 55.18; H, 6.10; S, 10.71.

EXAMPLE 9

Preparation of 1R, 2R, βR, γs; 1R, 2S, βR, γS; 1S, 2R, βR, γS; 1S, 2S, βR, γs; 1R, 2R, βS, γR; 1R, 2S, βS, γR; 1S, 2R, βS, γR; 1S, 2S, βS, γR 4-((1-(4-((3-(4-Acetyl-3-hydroxy-2-propylphenoxy)propyl)thio)phenyl)-3-carboxy-2-methylpropyl)thio)gamma-hydroxy-beta-methylbenzenebutanoic acid disodium salt monohydrate

Step A : Preparation of 4-(Methylthio)phenyl-propan -1-one

To a solution of thioanisole (5 g) and propionyl chloride (3.9 ml) in dichloroethane (80 ml) at 0 °C was added, in portions, aluminum chloride (6.4 g). The mixture was stirred overnight at room temperature. The reaction mixture was poured onto a mixture of ice and water (200 ml) and concentrated HCl (2 ml) and extracted with $CH_2Cl_2$. The combined organic layers were washed with water, dried over $Na_2SO_4$ and purified on a silica gel column (140 g) using hexane/EtOAc (10:1) as eluant to afford the title compound as a white solid, m.p. 60-61 °C.

Step B : Preparation of Methyl 4-(methylthio)-beta -methyl-gamma-oxobenzenebutanoate

To a solution of potassium hexamethylsilazane (0.158 mole) in toluene (254 ml) and THF (160 ml) at -78 °C was added dropwise the ketone from Step A of this Example (25.8 g) in THF (90 ml). The reaction mixture was stirred 30 minutes at -78 °C. Methyl bromoacetate (16.2 ml) in THF (25 ml) was added dropwise. After stirring 1.5 hours at -78 °C the reaction mixture was poured into IN HCl (400 ml). The organic layer was separated and the aqueous layer was further extracted with ethyl acetate. The combined organic layers were washed with water, dried over $Na_2SO_4$ and evaporated to give a yellow oil which was purified on a flash silica gel column (1 kg) using hexane/EtOAc (10:2) as eluant to give the title compound as an oil.

$^1$H NMR ($CDCl_3$) delta: 1.2 (3H, d, J=6Hz), 2.45 (3H, s), 2.3-3.1 (2H, m), 3.6 (3H, s), 3.85 (IH, quintuplet, J=6Hz), 7.25 (2H, d, J=7Hz), 7.9 (2N, d, J=7Hz).

Step C : Preparation of Methyl 4-(methylsulfinyl) -beta-methyl-gamma-oxobenzenebutanoate

To a solution of the sulfide from Step 2 (14.6 g) in $CH_2Cl_2$ (75 ml) at 0 °C was added dropwise a solution of 85% m-CPBA (11.7 g) in $CH_2Cl_2$ (225 ml). The reaction mixture was stirred for 2 hours at 0 °C and solid calcium hydroxide (6.4 g) was added and stirred at room temperature for 15 minutes and filtered through a bed of Celite. The filtrate was evaporated to give an oil which was purified on a flash silica gel column (175 g) using $CH_2Cl_2$/acetone (10:1) as eluant to afford the title compound as a colorless oil (13.5 g, 87%).

$^1$H NMR ($CDCl_3$) delta: 1.23 (3H, d, J=6Hz), 2.45-2.57 and 2.95-3.1 (2H, m), 2.78 (3H, s), 3.65 (3H, s), 3.95 (IH, quintuplet), 7.78 (2H, d, J=7Hz), 8.15 (2H, d, J=7Hz).

Step D : Preparation of Methyl 4-(trifluoroacetoxy -methylthio)-beta-methyl-gamma-oxobenzene -butanoate

A solution of the sulfoxide from Step C of this Example (10 g) in trifluoroacetic anhydride (50 ml) was heated at 45° C for 25 minutes and the mixture was evaporated, and then coevaporated with toluene, to dryness to give the title compound as an oil (15 g, crude) which was used directly in the following step.

$^1$H NMR ($CDCl_3$) delta: 1.23 (3H, d, J=6Hz), 1.45-1.52 (lH, dd), 1.93-2.05 (lH, m), 3.65 (3H, s), 3.85-4.0 (lH, m), 5.7 (2H, s), 7.55 (2H, d, J=7Hz), 7.97 (2H, d, J=7Hz).

Step E: Preparation of Methyl 4-mercapto-beta -methvl-gamma-oxobenzenebutanoate

To the neat trifluoroacetate from Step D of this Example (12.3 g) was added a mixture of 1:1 MeOH-$NEt_3$ (600 ml) and the resulting reaction mixture was evaporated under vacuum. The procedure was repeated twice more. The residue was dissolved in $CH_2Cl_2$, washed with IN HCl and brine and dried over $Na_2SO_4$. Evaporation of the solvent gave the title compound as an oil (7.8 g, 97%).

$^1$H NMR ($CDC1_3$) delta: 1.23 (3N, d, J=6Hz), 2-.45-2.52 (lH, dd), 2.93-3.05 (lH, m), 3.65 (3H, s), 3.85-4.0 (lH, m), 7.32 (2H, d, J=7Hz), 7.85 (2N, d, J=7hz).

Step F: Preparation of Methyl 4-(S-dimethylthio -carbamoyl)-beta-methyl-gamma-oxobenzenebutanoate

To a solution of the thiol from Step E of this Example (7.5 g) in DMF (100 ml) at 0° C was added in two portions 99% NaH (835 mg) and stirred for 30 minutes at 0° C. To this mixture was added dimethylcarbamoyl chloride (3.8 ml) and stirred for 15 minutes at 0° C and 30 minutes at room temperature. The reaction mixture was poured onto a mixture of ice and water (300 ml) and extracted with EtOAc. The combined organic layers were washed with brine, dried over $Na_2SO_4$ and evaporated to give a yellow oil which was purified on a flash silica gel column (500 g) using hexane/EtOAc (2:1) as eluant to give the title compound as an oil (7.8 g, 77%).

$^1$H NMR ($CDCl_3$) delta: 1.23 (3H, d, J=6Hz), 2.45-2.55 (lH, dd), 2.93-3.05 (lH, m), 3.0-3.2 (6H, d(b)), 3.65 (3H, s), 3.85-4.0 (lH, m), 7.62 (2H, d, J=7Hz), 7.97 (2H, d, J=7Hz).

Step G : Preparation of 4-(S-Dimethylthiocarbamoyl) -beta-methyl-gamma-oxobenzenebutanoic acid

To a solution of the ester from Step F of this Example (7.6 g) in MeOH (110 ml) at 0° C was added 2N NaOH (37 ml) and the mixture was stirred at 0° C for 1 hour. The reaction mixture was poured into a mixture of ice and water (300 ml), acidified with concentrated HCl and extracted with EtOAc. The combined organic layers were washed with water, dried over $Na_2SO_4$ and evaporated to give the title compound as an oil (7.3 g, 100%).

$^1$N NMR ($CDCl_3$) delta: 1.23 (d, 3H, J=6Hz), 2.4-2.55 (lH, dd), 2.85-3.05 (lH, m), 3.0-3.2 (6H, d(b)), 3.8-4.0 (1H, m), 7.6 (2H, d, J=7Hz), 7.95 (2H, d, J=7Hz).

Step H: Preparation of $\beta R^*$, $\gamma S^*$4-(S-Dimethylthio -carbamoyl)-beta-methyl-gamma-hydroxybenzenebutanoic acid gamma-lactone

To a solution of the ketone from Step G of this Example (6.85 g) in dry THF (190 ml) at -78° C was added dropwise, under nitrogen, a solution of 1.5M diisobutyl aluminum hydride (DIBAL-H) in toluene (37 ml) and stirred at -78° C for 1.5 hours. The reaction mixture was poured into cold IN HCl (600 ml) and extracted with EtOAc. The combined organic layers were washed with brine, dried over $Na_2SO_4$ and evaporated to give an oil which was dissolved in $CH_2Cl_2$ (200 ml) and trifluoroacetic acid (70 drops) was added and the mixture stirred at room temperature for 2 hours. The reaction mixture was diluted with toluene and evaporated to give an oil which was purified on flash silica gel column (350 g) using hexane/EtOAc (1:1) as eluant to give the title compound as a white solid (3.4 g, 52%): m.p. 113-115° C.

$^1$H NMR ($CDCl_3$) delta: 0.73 (cis) and 1.2 (trans) (3H, d, J=7Hz), 2.27-2.45 (lH, dd), 2.75-3.0 (2H, m), 3.0-3.2 (6H, d(b)), 4.97 (trans) and 5.6 (cis) (lH, d, J=5.6Hz), 7.27 (2H, d, J=7Hz), 7.55 (2H, d, J=7Hz).

Unreacted starting material was recovered from the column by eluting with toluene-dioxaneacetic acid (10:2:0.1) to recover 2.2 g (32%) as an oil.

Step I : Preparation of βR*, γS* 4-mercapto-beta -methyl-gamma-hydroxybenzenebutanoic acid gamma-lactone

To a suspension of the thiocarbamate from Step H of this Example (3 g) in MeOH (160 ml) was added 2N NaOH (54 ml) and the mixture refluxed under $N_2$ for 1.5 hours. The reaction mixture was cooled to room temperature, diluted with water (500 ml), acidified with 2N HCl and extracted with ethyl acetate. The combined organic layers were washed with water, dried over $Na_2SO_4$ and evaporated to give an oil which was dissolved in $CH_2Cl_2$ (100 ml) and treated with trifluoroacetic acid (30 drops) from 3 hours at room temperature. The reaction mixture was evaporated and coevaporated with toluene to give the title compound as an oil (2.39 g).

$^1H$ NMR ($CDCl_3$) delta: 0.73 and 1.2 (3H, d, J=7Hz), 2.27-2.45 (lH, dd), 2.75-2.95 (2H, m), 2.5 (lH, s), 4.9 and 5.55 (lH, d, J=5.6 Hz), 7.1 (2H, d, J=7Hz), 7.3 (2H, d, J=7Hz).

Step J : Preparation of 1R*, 2R*, βR*, γS* and 1R*, 2S*, βR*, γS*4-((1-(4-((3-(4-Acetyl-3 -hydroxy-2-propylphenoxy)propyl)thio)phenyl) -3-carbomethoxy-2-methylpropyl)thio)-gamma -hydroxy-beta-methylbenzenebutanoic acid gamma lactone

To a solution of the alcohol from Step A, Example 1 (930 mg) and the thiol from Step I of this Example (410 mg) in anhydrous $CH_2Cl_2$ (75 ml) was added dry zinc iodide (3.13 g) and the mixture was stirred at room temperature under $N_2$ for 4.5 hours. The reaction mixture was washed with 0.1N HCl, brine, dried over $Na_2SO_4$ and evaporated to dryness to give an oil which was purified on a PREP-500 Waters HPLC using hexane/EtOAc (10:8) as eluant to give the title compound as an oil (700 mg), 54%.

$^1H$ NMR ($CDCl_3$) delta: 0.62 (3H, d, J=6Hz), 0.85-1.0 (6H, m), 1.15 (2H, d, J=6Hz), 1.45-1.65 (2H, m), 2.0-2.2 (2H, m), 2.2-2.4 (2H, m), 2.5-2.7 (7H, m, containing a sharp singlet), 2.7-2.9 (2H, m), 3.1 (2H, t, J=6Hz), 3.65 and 3.7 (3H, 2 singlets), 4.05-4.2 (3H, m), 5.5 (lH, d, J=5.6Hz), 6.45 (lH, d, J=7Hz), 7.0-7.3 (8H, m), 7.6 (lH, d, J=7hz), 12.7 (lH, s).

Step K : Preparation of 1R, 2R, βR, γs; 1R, 2S, βR , γS; 1S, 2R, βR, γS; 1S, 2S, βR, γS; 1R, 2R , βS, γR; 1R, 2S, βS, γR; 1S, 2R, βS, γR; 1S , , 2S, βS, γR 4-((l-(4-((3-(4-Acetyl-3-hydroxy -2-propylphenoxy)propyl)-thio)phenyl)-3 -carboxy-2-methylpropyl)thio)-gamma-hydroxy -betamethylbenzenebutanoic acid disodium salt monohydrate

To a solution of the ester lactone from Step J of this Example (664 mg) in THF (10 ml) and MeOH (1 ml) was added 2N NaOH (1.2 ml) and stirred overnight at room temperature. The reaction mixture was evaporated to dryness and passed through a column of neutral XAD-8 resin eluting with water (250 ml) and then with 95% EtOH to give, after evaporation of the ethanol, the title compound as a foam (690 mg, 100%).

| | |
|---|---|
| | Analysis for: $C_{36}H_{42}O_8S_2Na_2.H_2O$: |
| Calc'd: | C, 59.16; H, 6.07; S, 8.77; Na, 6.29. |
| Found: | C, 59.15; H, 6.28; S, 8.98; Na, 5.40. |

EXAMPLE 10

Preparation of 1R*, 2R*, βR*; 1R*, 2R*, βS*; 1R* , 25*, βR*; and 1R*, 2S*, βS* 4-((1-(4-((3-(4 -Acetyl-3-hydroxy-2-propylphenoxy)propyl)thio) phenyl)-3-carboxy-2-methylpropyl)thio)-beta-methyl -gamma-oxobenzenebutanoic acid monohydrate

Step A : Preparation of Methyl 4-((1-(4-((3-(4-acetyl -3-hydroxy-2-propylphenoxy)propyl)thio)phenyl) -4-methoxy-2-methyl-4-oxobutyl)thio)-beta -methyl-gamma-oxobenzenebutanoate

To a solution of alcohol from Example 1, Step A (1.6 g) and the thiol from Example 9, Step E (800 mg) in anhydrous $CH_2Cl_2$ (170 ml) was added dry zinc iodide (10.7 g) and stirred at room temperature under $N_2$ for 3.5 hours. The reaction mixture was washed with 0.IN HCl, brine and dried over $Na_2SO_4$ to give an oil which was purified on PREP-500 Waters HPLC using hexane-EtOAc (2:1) as eluant to give an oil (1.06 g, 45%).

$^1$H NMR $CDCl_3$ delta: 0.85-1.25 (9H, m), 1.45-1.65 (2H, m), 2.0-2.2 (2H, m), 2.2-2.5 (2H, m), 2.5-2.7 (7H, m, containing a sharp singlet), 2.85-3.0 (IH, q), 3.15 (2H, t, J=6Hz), 3.63 (3H, s), 3.66 and 3.7 (3H, 2s) , 3.8-3.9 (IH, m) , 4.15 (2H, t, J=6Hz), 4.35-4.45 (IH, m), 6.45 (IH, d, J=7Hz), 7.2-7.4 (6H, m), 7.58 (IH, d, J=7Hz), 7.75-7.85 (2H, m).

Analysis for: $C_{38}H_{46}O_8S_2$:
Calc'd: C, 65.68; H, 6.67; S, 9.23.
Found: C, 65.48; H, 6.59; S, 9.51.

Step B : Preparation of 4-((1-(4-((3-(4-Acetyl-3 -hydroxy-2-propylphenoxy)propyl)thio)phenyl) -3-carboxy-2-methylpropyl)thio)-beta-methyl -gamma-oxo-benzenebutanoic acid monohydrate

To a solution of ester from Step A of this Example (760 mg) in THF (15 ml) and MeOH (1 ml) was added 2N NaOH (2.4 ml) and stirred overnight at room temperature. The reaction mixture was diluted with $N_2O$ (50 ml) and acidified with 2N HCl and extracted with $CH_2Cl_2$ (2x50 ml). The combined organic layers were washed with brine, dried over $Na_2SO_4$ and purified on a column of flash silica gel 230-400 mesh using toluene-dioxane-acetic acid (10:2:0.1) as eluant to give the title compound as a foam (580 mg, 79%).

Analysis for: $C_{36}H_{42}O_8S_2.H_2O$:
Calc'd: C, 63.13; H, 6.47; S, 9.36.
Found: C, 62.64; H. 6.25; S, 9.46.

EXAMPLE 11

Preparation of 4-((3-(4-acetyl-3-hydroxy-2-propyl -phenoxy)propyl)thio)-γ-((4-(3-carboxy-1-hydroxy -propyl)-phenyl)thio-benzene butanoic acid, disodium salt, monohydrate, mixture of isomers

Step A: Methyl 4(3-bromopropylthio)-γ-((4-(3 -methoxycarbonyl-1-hydroxy-propyl)phenyl) -thio)-benzenebutanoate, mixture of isomers

To a cooled (0° C) solution made of the ketone obtained in Step D of Example 2 (2.72 g) in DME (1,2-dimethoxyethane) (25 c.c.) and MeOH (5 c.c.) containing $CeCl_3$ (5 mg), was added portion-wise $NaBH_4$ (138 mg) and the mixture was kept at this temperature until the starting ketone had disappeared. The mixture was then poured into ice-cold water, acidified with IN HCl and the organic material was extracted into ethyl acetate; the organic layer was washed with brine, dried with $Na_2SO_4$ and the solvents were removed *in vacuo* to yield an oil which was purified on silica gel to yield the title compound as an oil.

Analysis Calc'd: C, 54.05; H, 5.62; S, 11.54.
Found: C, 53.83; H, 5.70; S, 11.55.

Step B : Preparation of 4-((1-(4-((3-(4-acetyl-3 -hydroxy-2-propylphenoxy)propyl)thio)phenyl) -3-(methoxycarbonyl)propyl)thio)-γ-hydroxybenzenebutanoic acid, -γ-lactone, mixture of isomers

A suspension of the bromide from the previous step (2.05 g), 2,4-dihydroxy-3-propyl-acetophenone (933 mg), and milled potassium carbonate (765 mg) in MEK (methylethylketone) (20 c.c.) was refluxed for 6 hours. Thereafter it was cooled to room temperature, the solids were filtered off and the solvent was removed in vacuo to yield an oil which was purified on silica gel to yield the title compound as an oil.

NMR $^1H$-250 MHz/$CDCl_3$

| δ(ppm) | No. of H | m | J(Hz) |
|---|---|---|---|
| 12.75 | 1 | s | - |
| 7.58-7.62 | 1 | d | 8.3 |
| 7.15-7.3 | 8 | m | - |
| 6.4-6.48 | 1 | d | 8.3 |
| 5.4-5.5 | 1 | t | 6.0 |
| 4.1-4.25 | 3 | m | - |
| 3.65 | 3 | s | - |
| 3.1-3.18 | 2 | t | 5.0 |
| 2.05-2.7 | 15 | m | - |
| 1.5-1.6 | 2 | m | - |
| 0.9-1.0 | 3 | t | 5.0 |

Step C : Preparation of 4-((3-(4-acetyl-3-hydroxy-2-propylphenoxy)propyl)thio)-γ-((4-(3-carboxy-1-hydroxypropyl)phenyl)thio)benzene butanoic acid, disodium salt, monohydrate, mixture of isomers

To a solution of the lactone from the previous step (1.64 g) in MeOH (5 c.c.) and THF (20 c.c.) was added 2N NaOH (3.9 c.c.) and the mixture was stirred for 2 hours. The reaction mixture was evaporated to dryness in vacuo and absorbed onto XAD-8 neutral resin in water, washed with water and then eluted off with ethanol. Evaporation of the solvent in vacuo yielded the title compound.

| | | | | | |
|---|---|---|---|---|---|
| Analysis | Calc'd: | C, 58.11; | H, 5.74; | S, 9.12; | Na, 6.54 |
| | Found: | C, 58.57; | H, 5.97; | S, 9.31; | Na, 5.16 |

EXAMPLE 12

Preparation of βR*, γS* and βS*, γR* 4-((3-(4 -acetyl-3-hydroxy-2-propylphenoxy)propyl)thio)-γ-((2-carboxymethyl)thio)-β-methylbenzenebutanoic acid disodium salt (mixture of diastereoisomers) , hemihydrate

Step A: Preparation of Methyl 4-((3-(4-acetyl-3 -hydroxy-2-propylphenoxy)propylthio)-γ-((-2 -methoxycarbonylmethyl)thio)-β-methylbenzenebutanoate (mixture of diastereoisomers , monohydrate

To an efficiently stirred solution of the alcohol (948 mg) from Step A of Example 1 and methyl thioglycolate (233 mg) in dichloroethane (10 c.c.) was added $ZnI_2$ (1.92 g) and the suspension was stirred for 3 hours. Thereafter, in HCl (20 c.c.), $H_2O$ (20 c.c.) and dichloromethane (50 c.c.) were added. The organic layer was separated, washed with brine, and dried with $Na_2SO_4$. The solvents were removed in vacuo to yield a residue which was chromatographed cn silica gel to yield the title compound as an oil.

Analysis Calc'd: C, 59.97; H, 6.94; S, 11.04.
Found: C, 59.91; H, 7.09; S, 10.94.

Step B : Preparation of βR*, γS* and βS*, γR * 4-((3-(4-acetyl-3-hydroxy-2-propylphenoxy) -propyl)thio)-γ-((2-carboxymethyl)thio)-β-methylbenzenebutanoic acid disodium salt , hemihydrate (mixture of diastereoisomers)

A solution of the diester from the previous step (790 mg) in 2N NaOH (2.1 cc.), MeOH (2 c.c.) and THF (10 c.c.) was stirred for 4 hours. The reaction mixture was evaporated to dryness and absorbed onto XAD-8 neutral resin in water, washed with water and then eluted off with ethanol. Evaporation of the solvent *in vacuo* yielded the title compound.

Analysis Calc'd: C, 55.18; H, 5.66; S, 10.91; Na, 7.82.
Found: C, 55.60; H, 5.66; S, 9.96; Na, 7.17.

EXAMPLE 13

Preparation of βR*, γR* 4-((3-(4-acetyl-3-hydroxy-2 -propylphenoxy)propyl)thio)-γ-((2-carboxyethyl)thio)-β-methylbenzenebutanoic acid, disodium salt, mixture of enantiomers
and
preparation of βR*, γS* 4-((3-(4-acetyl-3-hydroxy-2 -propylphenoxy)propyl)thio)-γ-((2-carboxyethyl)-thio) -β-methylbenzenebutanoic acid, disodium salt, mixture of enantiomers

Step A: Preparation of βR*, γR* 4-((3-(4-acetyl-3 -hydroxy-2-propylphenoxy)propyl)thio)-γ -(thio)-β-methylbenzenebutanoic acid-γ-thiolactone, mixture of enantiomers

A solution of the thioacetate obtained from Step A of Example 7 (42 g) in THF (250 c.c.) was cooled to 0°C, a solution of NaOMe (6.5 g) in MeOH (100 c.c.) was added, and the mixture was kept at 0°C for 30 minutes. Thereafter, it was poured into ice-cold IN HCl (200 c.c.); the organic material was extracted into ethyl acetate and the organic layer successively washed with 10% $NaHCO_3$ and brine, dried with $Na_2SO_4$ and concentrated *in vacuo* to yield a residue which was then dissolved in dry THF (250 c.c.) and cooled to 0°C. Thereafter, a suspension of NaH (1.6 g) in THF (50 c.c.) was added dropwise and the mixture kept at 0°C for 6 hours. It was then poured into ice-cold IN HCl (300 c.c.) and extracted into ethyl acetate. The organic layer was washed with 10% $NaHCO_3$, brine and then dried with $Na_2SO_4$ and concentrated *in vacuo* to yield a residue which was purified on silica gel to yield the mixture of the βR*, γR* and the βR*, γS* thiolactones from which the βR*, γR* lactone was purified by repetitive preparative HPLC.

NMR $^1H$-250 MHz/$CDCl_3$ for βR*, γR*

| δ(ppm) | No. of H | M | J(Hz) |
|---|---|---|---|
| 4.50 | 1 | d | 6.6 |
| 1.09 | 3 | d | 6.6 |

Analysis Calc'd: C, 65.47; H, 6.59; S, 13.98.
Found: C, 64.72; H, 6.32; S, 13.18.

Step B: Preparation of βR", γS* 4-((3-(4-acetyl-3 -hydroxy-2-propylphenoxy)propyl)thio-γ-(thio)-β-methylbenzenebutanoic acid-γ-thiolactone, mixture of enantiomers

This lactone was isolated by repetitive preparative HPLC purification of the mixture of the βR*, γR* and βR*, γS* thiolactones obtained in the previous step.

Analysis Calc'd: C, 65.47; H, 6.59; S, 13.98.
Found: C, 64.69; H, 6.03; S, 13.48.

NMR $^1$H-250 MHz/$CDCl_3$ for βR*, γS*

| δ(ppm) | No. of H | M | J(Hz) |
|---|---|---|---|
| 5.0 | 1 | d | 6.6 |
| 0.8 | 3 | d | 6.6 |

Step C: Preparation of βR*, γR*methyl 4-((3-(4 -acetyl-3-hydroxy-2-propylphenoxy)propyl)thio) -γ-((2-methoxycarbonylethyl)thio)-β-methyl -benzenebutanoate, mixture of enantiomers

To an ice cold solution of Na (19 mg) in MeOH (5 c.c.) was added a THF (5 c.c.) solution of the lactone (290 mg) obtained in Step A of this Example and the mixture was reacted at 0° C for 1 hour. Methyl 3-bromopropionate (127 mg) was then added and reacted for 2 hours at 0° C. Dowex 50-Wx-8 resin was then added and stirred for 15 minutes and removed by filtration. The filtrate was concentrated in vacuo, then diluted with $CH_2Cl_2$ (15 c.c.), washed with brine and the organic layer was dried with $Na_2SO_4$. Removal of the solvents in vacuo, followed by purification of the residue on silica gel yielded the title compound as an oil.

Analysis Calc'd: C, 62.47; H, 6.99; S, 11.12.
Found: C, 62.44; H, 7.67; S, 10.70.

Step D: Preparation of βR", γS* methyl 4-((3-(4 -acetyl-3-hydroxy-2-propylphenoxy)propyl)thio) -γ-((2-methoxycarbonylethyl)thio)-β-methyl -benzenebutanoate, mixture of enantiomers

To an ice cold solution of Na (28 mg) in MeOH (10 c.c.) was added a THF (10 c.c.) solution of the lactone (430 mg) obtained in Step B of this Example and the mixture was reacted for 1 hour at 0° C. Methyl 3-bromopropionate (200 mg) was then added and reacted for 2 hours. Dowex 50-WK-8 resin was then added, stirred for 15 minutes and removed by filtration. The filtrate was concentrated in vacuo , diluted in $CH_2Cl_2$ (25 c.c.), washed with brine and the organic layer was dried with $Na_2SO_4$. Removal of the solvent in vacuo , followed by purification of the residue on silica gel yielded the title compound as an oil.

Analysis Calc'd: C, 62.47; H, 6.99; S, 11.12.
Found: C, 62.51; H, 7.04; S, 11.05.

Step E: Preparation of βR", γR*4-((3-(4-acetyl-3 -hydroxy-2-propylphenoxy)propyl)thio)-γ-((2 -carboxyethyl)-thio)-β-methylbenzenebutanoic acid, disodium salt, mixture of enantiomers

To a solution of the product obtained in Step C of this Example (231 mg) in MeOH (5 c.c.), THF (5 c.c.)

and $H_2O$ (2 c.c.) was added 2N NaOH (0.6 c.c.) and the mixture was stirred at room temperature for 12 hours. The solvents were then removed in vacuo and the residue absorbed on XAD-8 neutral resin in water, washed with water and then eluted off with ethanol. Evaporation of the solvent in vacuo yielded the title compound as a highly hygroscopic foam. NMR $^1H$-250 MHz/$CD_3OD$

NMR $^1H$-250 MHz/$CD_3OD$

| δ(ppm) | No. of H | m | J(Hz) |
|---|---|---|---|
| 7.59-7.64 | 1 | d | 8.3 |
| 7.12-7.21 | 4 | m | |
| 6.41-6.45 | 1 | d | 8.3 |
| 4.0-4.1 | 2 | t | 5.5 |
| 3.59-3.65 | 1 | d | 8.3 |
| 2.97-3.05 | 2 | t | 5.5 |
| 1.65-2.55 | 14 | m | |
| 1.35-1.45 | 2 | m | |
| 0.9-1.0 | 3 | d | 6.1 |
| 0.75-0.82 | 3 | t | 5.5 |

Step F: Preparation of βR*, γS* 4-((3(4-acetyl-3-hydroxy-2-propylphenoxy)propyl)thio)-γ-( (2-carboxyethyl)-thio)-β-methyl benzenebutanoic acid, disodium salt, mixture of enantiomers

To a solution of the product obtained in Step D of this Example (387 mg) in MeOH (5 c.c.), THF (5 c.c.) and $H_2O$ (2 c.c.) was added 2N NaOH (1.0 c.c.) and the mixture was stirred at room temperature for 12 hours. The solvents were then removed in vacuo and the residue absorbed on XAD-8 neutral resin in water, washed with water and then eluted off with ethanol. Evaporation of the solvent in vacuo yielded the title compound as a highly hygroscopic foam.

NMR $^1H$-250 MHz/$CD_3OD$

| δ(ppm) | No. of H | m | J(Hz) |
|---|---|---|---|
| 7.59-7.65 | 1 | d | 8.3 |
| 7.12-7.22 | 4 | m | |
| 6.41-6.45 | 1 | d | 8.3 |
| 4.0-4.1 | 2 | t | 5.5 |
| 3.59-3.65 | 1 | d | 8.3 |
| 2.95-3.05 | 2 | t | 5.5 |
| 1.65-2.55 | 14 | m | |
| 1.35-1.45 | 2 | m | |
| 0.75-0.85 | 3 | t | 5.5 |
| 0.69-0.75 | 3 | d | 6.1 |

EXAMPLE 14

Preparation of βR*, γS* 4-((3-(4-acetyl-3-hydroxy-2 -propylphenoxy)propyl)sulfonyl)-γ-((2-carboxyethyl) - thio)-β-methylbenzenebutanoic acid, disodium salt , mixture of enantiomers, monohydrate

Step A: Preparation of βR*, γS* 4-((3-(4-acetyl-3 -hydroxy-2-propylphenoxy)propyl)sulfonyl)-γ-(thio)-β-methylbenzene butanoic acid, -γ-thiolactone, mixture of enantiomers

To a cooled (0° C) solution of the lactone (230 mg) obtained in Step B of Example 13 in chloroform (5 c.c.) was added m-chloroperoxybenzoic acid (m-CPBA) (105 mg) and the suspension was stirred at 0° C for 45 minutes. Another portion (10.5 mg) of m-CPBA was then added and reacted for 1 hour at 0° C. The suspension was warmed up to room temperature and $Ca(OH)_2$ (111 mg) was added and the mixture stirred for 1 hour. Insolubles were then filtered off and the filtrate concentrated to dryness to yield the title compound.

| | | | | |
|---|---|---|---|---|
| Analysis Calc'd.1$H_2O$: | C, 59.22; | H, 6.34; | S, 12.61. |
| Found: | C, 59.24, | H, 5.87; | S, 12.43. |

Step B: Preparation of βR*, γS* methyl 4-((3-(4 -acetyl-3-hydroxy-2-propylphenoxy)propyl) -sulfonyl)-γ-((2-methoxycarbonyl ethyl) -thio)-β-methylbenzenebutanoate, mixture of enantiomers

To a cooled (0° C) solution of the lactone obtained in the previous step (800 mg) in MeOH (25 c.c.) was added NaOMe (110 mg) followed, one-half hour later, by methyl acrylate (215 mg). After 45 minutes at this temperature, IN HCl (25 c.c.) was added and most of the methanol removed in vacuo; organic materials were extracted into ethyl acetate (3 x 25 c.c.), the organic layer washed with brine, dried with $Na_2SO_4$ and concentrated to dryness to yield a residue which was purified by chromatography on silica gel, yielding the title compound as an oil.

| | | | |
|---|---|---|---|
| Analysis Calc'd: | C, 59.19; | H, 6.62; | S, 10.53. |
| Found: | C, 58.74; | H, 6.56; | S, 9.60. |

Step C: Preparation of βR*, γS* 4-((3-(4-acetyl-3 -hydroxy-2-propylphenoxy)propyl)sulfonyl)-y -((2-carboxyethyl) thio) -β-methylbenzenebutanoic acid, disodium salt, mixture of enantiomers , monohydrate

To a cooled (0° C) solution of the compound obtained in the previous step (925 mg) in THF (10 c.c.) and MeOH (5 c.c.) was added 2N NaOH (2.3 c.c.) and the mixture was stirred for 48 hours while being warmed up to room temperature. The solvents were then removed in vacuo and the residue absorbed on XAD-8 neutral resin in water, washed with water and then eluted off with ethanol. Removal of the solvent from the ethanolic fraction yielded the title compound as a foam.

| | | | |
|---|---|---|---|
| Analysis Calc'd.1$H_2O$: | C, 52.53; | H, 5.65; | S, 9.98 |
| Found: | C, 52.31; | H, 5.43; | S, 9.45. |

EXAMPLE 15

Preparation of βR*, γS* and βR*, γR* 4-((3-(4 -acetyl-3-hydroxy-2-propylphenoxy)propyl)thio)-γ-((2 -N,N-dimethylcarbonylethyl)thio)-β-methylbenzene -butanoic acid, sodium salt

Step A: Preparation of βR*, γS* and βR*, γR * methyl 4-((3-(4-acetyl-3-hydroxy-2-propyl -phenoxy)propyl)-thio)-γ-((2-N,N-dimethyl -carbonylethyl)thio)-β-methylbenzenebutanoate

To a cooled (0° C) solution of the mixture of the lactones prepared in Step A of Example 13 (916 mg) in MeOH (20 c.c.) and THF (10 c.c.) was added NaOMe (130 mg) followed 90 minutes later by N,N-dimethylacrylamide (300 mg) and this mixture was reacted for 2 hours at 0° C. It was then acidified with IN HCl and extracted with ethyl acetate (3 x 25 c.c.); the organic layer was washed with brine, dried with $Na_2SO_4$ and concentrated to yield a residue which was purified on silica gel to yield the title compound as an oil.

Analysis Calc'd: C, 63.13; H, 7.35; S, 10.87
Found: C, 62.86; H, 7.58; S, 10.83.

Step B: Preparation of βR*, γS* and βR*, γR * 4-((3-(4-acetyl-3-hydroxy-2-propylphenoxy) -propyl)thio)-γ-((2-N,N-dimethylcarbonyl -ethyl)thio)-β-methylbenzenebutanoic acid , sodium salt

To a solution of the ester obtained in the previous step (590 mg) in MeOH (10 c.c.) and $H_2O$ (2 c.c.) was added 2N NaOH (1.5 c.c.) and the mixture was reacted for 12 hours. It was then acidified and extracted with ethyl acetate (3 x 25 c.c.); the organic layer was washed with brine, dried with $Na_2SO_4$ and concentrated to dryness. To the residue (512 mg) dissolved in EtOH (5 c.c.) and MeOH (5 c.c.) was added IN NeOH (0.890 c.c.) and the obtained solution was evaporated to dryness in vacuo to yield the title compound as a foam.

Analysis Calc'd: C, 60.28; H, 6.75; S, 10.73.
Found: C, 60.02; H, 6.75; S, 10.78.

## Claims

1. A compound of the formula:

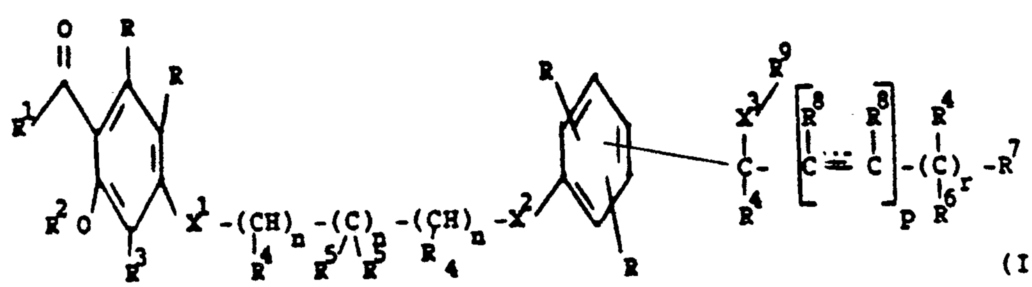

(I)

wherein:
the broken line represents an optional triple bond;
each R is independently H; OH; lower alkyl; lower alkenyl; trifluoromethyl; lower alkoxy; phenyl; phenyl substituted by alkyl of 1 to 3 carbon atoms or by halogen; benzyl; phenethyl; halogen; $N(R^4)_2$; -(C=O)-$R^1$; $CH_2OR^4$; CN; $SR^{10}$; $SOR^{10}$; $SO_2R^{10}$; or nitro; O
$R^1$ is H; lower alkyl; or lower alkoxy;
$R^2$ is H; lower alkyl; $R^4CO$; or $R^4OCH_2$;
each $R^3$ is independently lower alkyl or lower alkenyl;
each $R^4$ is independently H or lower alkyl;

each $R^5$ is independently H; $OR^2$; lower alkyl; or both $R^5$'s may be combined to create a doubly bonded oxygen (=O) or a $=C(R^4)_2$ group;
each $R^6$ is independently H; OH; or lower alkyl;
each $R^7$ is independently $COOR^4$; CHO; $CH_2OH$; tetrazole; $CONHSO_2R^{10}$; $NHSO_2R^{10}$; hydrozymethylketone; acetoxymethylketone; $CON(R^4)_2$; CN; Het; or

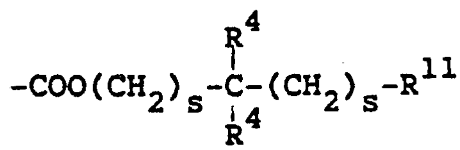

each $R^8$ is independently H or lower alkyl, and is absent when a triple bond is present;
$R^9$ is $R^3$,

$(CH_2)_a$-Het,

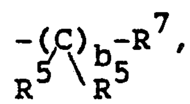

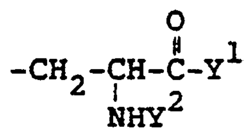

,

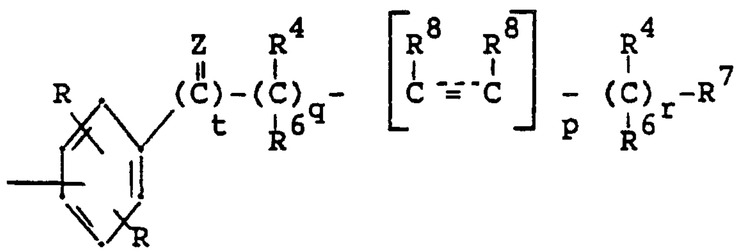

wherein the broken line represents an optional triple bond,

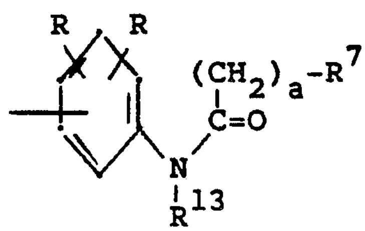

,

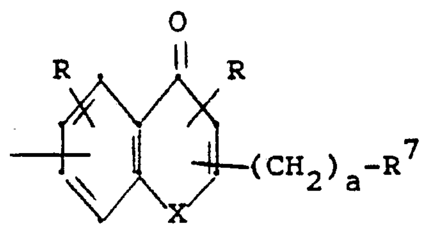

,

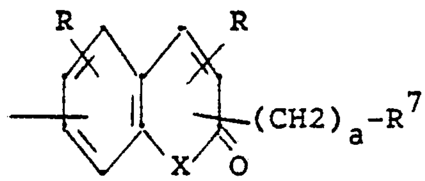

,

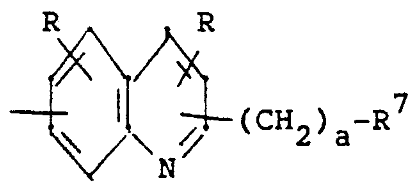

,

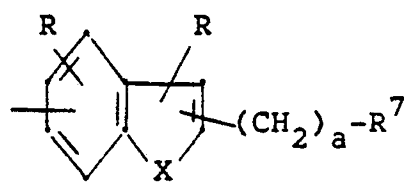

, or

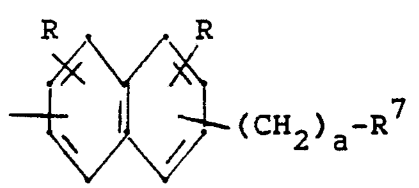

;

each $R^{10}$ is independently OH; $N(R^4)_2$; $CF_3$; lower alkyl; lower alkoxy; phenyl; or phenyl substituted by one or more alkyl or alkoxy groups of 1 to 3 carbon atoms, halogen, hydroxy, $COOR^4$, CN, formyl or lower alklacyl;

each $R^{11}$ is independently

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical $W\text{-}R^{12}$;

each $R^{12}$ independently contains up to 20 carbon atoms and is (1) an alkyl radical or (2) an alkylacyl radical of an organic acyclic or monocyclic carbozylic acid containing not more than one heteroatom in the ring;

each $R^{13}$ is independently H or $R^{10}$;

each W is independently O, S or NH;

each X is independently O, S or $NR^{13}$;

$X^1$ and $X^2$ are each independently 0,S,SO,$SO_2$, $S(O)=NR^4$, $NR^4$, $NCOR^1$, NCN, or $NCONHR^4$;

$X^3$ is 0,S,SO or $SO_2$;

$Y^1$ is OH or the N-terminus of an amino acid such that $Y^1H$ is an essential amino acid:

$Y^2$ is H or the C-terminus of an amino acid such that $Y^2OH$ is an essential amino acid;

z is O, H and OH, or H and $R^4$;

each a is independently 0 to 4;

each b is independently 1 to 6;
each n is independently 0 to 6;
each p is independently 0 to 2;
each q is independently 0 to 4;
each r is independently 0 to 4;
each s is independently 0 to 3;
each t is independently 0 to 1;
each Het is independently a heterocyclic or heterocyclic or heterobicyclic ring of 5 or 6 atoms each, containing one or more heteroatoms selected from O,N or S, said heterocyclic or heterobicyclic ring containing an acidic proton;
and the terms "lower alkyl", "lower alkenyl" and "lower alkoxy" mean $C_{1-7}$ alkyl, $C_{2-7}$ alkenyl and $C_{1-7}$ alkoxy respectively; or a pharmaceutically acceptable salt thereof.

**2.** A compound according to Claim 1, in which $R^1$ is H or lower alkyl;
$R_2$ is H;
each $R_3$ is independently lower alkyl or lower alkenyl;
each $R_5$ is independently H; $OR^2$; or both $R^5$s may be combined to create a doubly bonded oxygen (=O);
each $R^7$ is independently $COOR^4$; CHO; $CH_2OH$; tetrazole; or Het;
$x^1$, $x^2$ and $X^3$ are each independently O, S, SO or $SO_2$;
each n is independently 1 or 2; and each p is O; or a pharmaceutically acceptable salt thereof.

**3.** A compound according to Claim 2, in which each R is independently H; OH; lower alkyl; trifluoromethyl; lower alkoxy; halogen; $N(R^4)_2$;
$-(C=O)R^1$; $CH_2OR^4$; CN; $SR^{10}$; $SOR^{10}$; or $SO_2R^{10}$;
$R^1$ is lower alkyl;
each n is 1; q=0;
or a pharmaceutically acceptable salt thereof.

**4.** A compound according to Claim 3, of the formula:

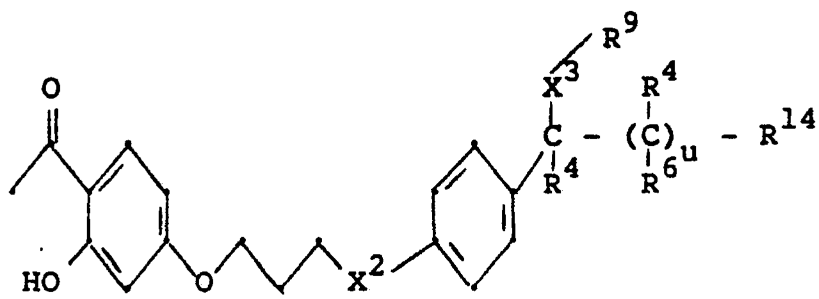

(II)

wherein $R^9$ is

$$(CH_2)_a\text{-Het},$$

where Het is

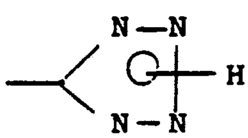

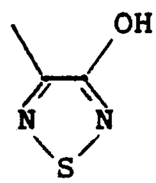

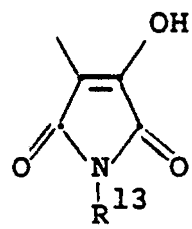

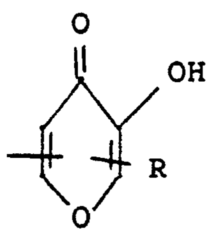

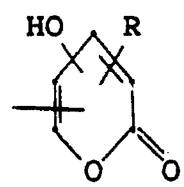

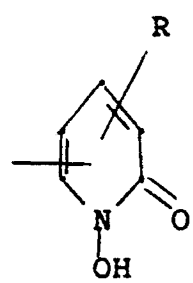

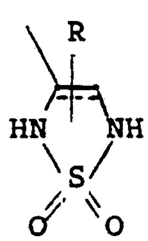

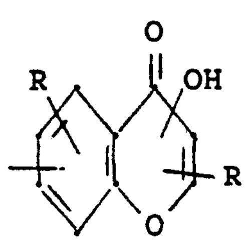

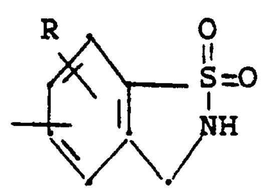

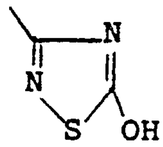 ; wherein the broken line represents an optional double bond.

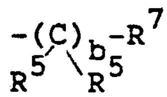 ,

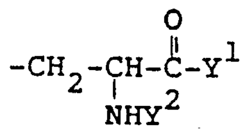 ,

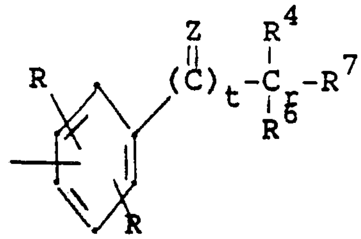 ,

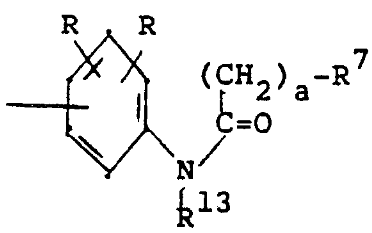 ,

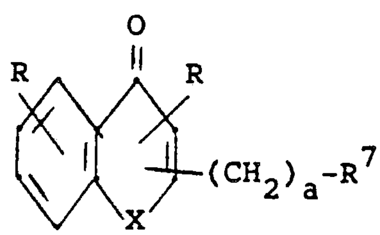 ,

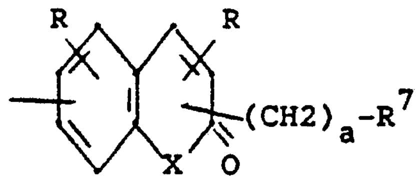

,

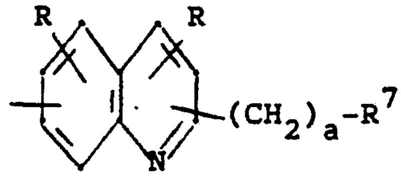

,

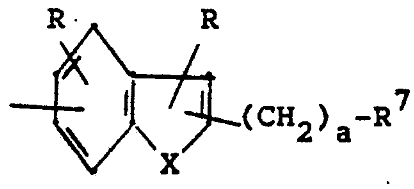

, or

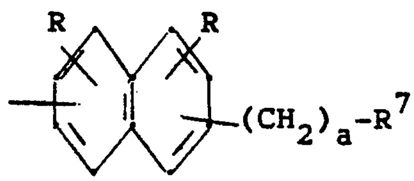

;

$R^{14}$ is $COOR^4$; u is 0 to 4;
or a pharmaceutically acceptable salt thereof.

**5.** A compound according to Claim 1, which is:
7-((γ-(4-((3-(4-acetyl-3-hydroxy-2-propylphenoxy)-propyl)thio)phenyl)-gamma-carboxy-β-methylpropyl) thio)-4-oxo-4-H-1-benzopyran-2-carboxylate disodium salt;
4-((1-(4-((3-(4-acetyl-3-hydroxy-2-propylphenoxy)-propyl)thio)phenyl)-3-carboxypropyl)thio)-gamma-oxobenzenebutanoic acid;
4-((3-(4-acetyl-3-hydroxy-2-propylphenoxy)propyl) thio)-gamma-((3-(carboxyacetyl)amino)phenyl)thio-beta-methylbenzenebutanoic acid;
4-((1-(4-((3-(3-acetyl-3-hydroxy-2-propylphenoxy) propyl)thio)phenyl)-3-carboxypropyl)thio)-beta-methyl-gamma-oxobenzenebutanoic acid;
N(S(γ-(4-((3-(4-acetyl-3-hydroxy-2-propylphenoxy) propyl) thio)phenyl) -gamma-carboxy-β-methyl-propyl)-cysteinyl)glycine disodium salt;
4-((3-)4-acetyl-3-hydroxy-2-propylphenoxy)propyl)-thio)-gamma-((2-carboxyethyl)thio)-beta-methyl-benzenebutanoic acid disodium salt;
4((3-(4-acetyl-3-hydroxy-2-propylphenoxy)propyl) thio)-gamma-(butylthio)-beta-methylbenzenebutanoic acid;
4-((3-(4-acetyl-3-hydroxy-2-propylphenoxy)propyl) thio)-beta-methyl-gamma-((1H-tetrazole-5-yl-methyl) thio)benzenebutanoic acid disodium salt monohydrate;
methyl 4-((3-(4-acetyl-3-hydroxy-2-propylphenoxy)-propyl)thio)-beta-methyl-gamma-((1H-tetrazole-5-yl-methyl)thio)benzenebutanoate sodium salt;
4-((1-(4-((3-(4-acetyl-3-hydroxy-2-propylphenoxy) propyl)thio)phenyl)-3-carboxy-2-methylpropyl)thio)-gamma-hydroxy-beta-methylbenzenebutanoic acid disodium salt monohydrate;
4-((1-(4-((3-(4-acetyl-3-hydroxy-2-propylphenoxy) propyl)thio)phenyl)-3-carboxy-2-methylpropyl) thio)-beta-methyl-gamma-oxobenzenebutanoic acid monohydrate;
4-((3-(4-acetyl-3-hydroxy-2-propylphenoxy)propyl)-thio)-γ-((4-(3-carboxy-1-hydroxypropyl)phenyl)-thio-benzene butanoic acid, disodium salt, monohydrate;
4-((3-(4-acetyl-3-hydroxy-2-propylphenoxy)propylthio)-γ-((2-carbozymethyl)thio)-β-methylbenzenebutanoic acid disodium salt, hemihydrate;
4-((3-(4-acetyl-3-hydroxy-2-propylphenoxy)propyl)thio)-γ-((2-carboxyethyl)thio)-β-methylbenzenebutanoic acid, disodium salt;
4-((3-(4-acetyl-3-hydroxy-2-propylphenoxy)propyl)-sulfonyl-γ-((2-carboxyethyl)thio)-β-methylbenzenebutanoic acid, disodium salt, monohydrate; or
4-((3-(4-acetyl-3-hydroxy-2-propylphenoxy)propyl)thio-γ-((2-N,N-dimethylcarbonylethyl)thio)-β-methylbenzenebutanoic acid, sodium salt.

6. A pharmaceutical composition useful in antagonizing leukotriene action in mammals comprising a compound as claimed in any one of Claims 1 to 5 and a pharmaceutically acceptable carrier.

7. A composition as claimed in Claim 6 additionally comprising an effective amount of a second active ingredient that is a non-steroidal anti-inflammatory drug; a peripheral analgesic agent; a cyclooxygenase inhibitor; a leukotriene antagonist; a leukotriene inhibitor; an $H_2$-receptor antagonist; an antihistaminic agent; a prostaglandin antagonist or a thromboxane antagonist.

8. A composition as claimed in Claim 7 in which the weight ratio of the compound as claimed in any one of Claims 1 to 5 to the second active ingredient ranges from 200:1 to 1:200.

9. A compound as claimed in any one of Claims 1 to 5 or a composition as claimed in any one of Claims 6 to 8, for use in preventing the synthesis, the action or the release of SRS-A and the leukotrienes $C_4$, $D_4$, $E_4$ and $B_4$ in mammals.

10. A compound as claimed in any one of Claims 1 to 5 or a composition as claimed in any one of Claims 6 to 8, for use in cytoprotection in mammals in need of cytoprotection.

Claims for the following Contracting State: AT

1. A method for preparing a compound of the formula:

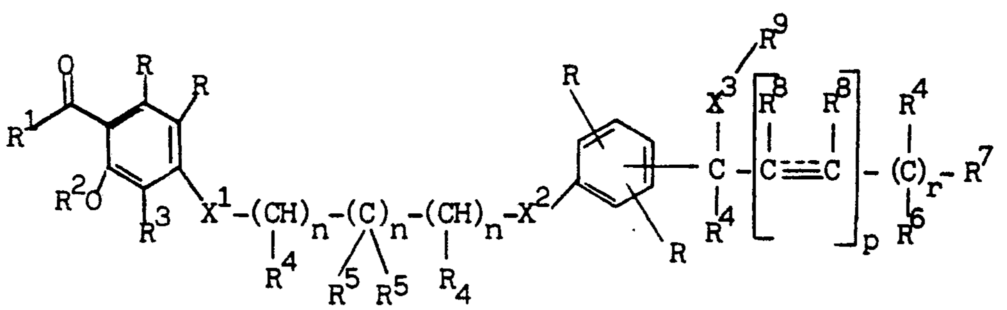

(I) ,

wherein:
the broken line represents an optional triple bond;
each R is independently H; OH; lower alkyl; lower alkenyl; trifluoromethyl; lower alkoxy; phenyl; phenyl substituted by alkyl of 1 to 3 carbon atoms or by halogen; benzyl; phenethyl; halogen; $N(R^4)_2$; $-(C=O)-R^1$; $CH_2OR^4$; CN; $SR^{10}$; $SOR^{10}$; $SO_2R^{10}$; or nitro; O
$R^1$ is H; lower alkyl; or lower alkoxy;
$R^2$ is H; lower alkyl; $R^4CO$; or $R^4OCH_2$;
each $R^3$ is independently lower alkyl or lower alkenyl;
each $R^4$ is independently H or lower alkyl;
each $R^5$ is independently H; $OR^2$; lower alkyl; or both $R^5$'s may be combined to create a doubly bonded oxygen (=O) or a $=C(R^4)_2$ group;
each $R^6$ is independently H; OH; or lower alkyl;
each $R^7$ is independently $COOR^4$; CHO; $CH_2OH$; tetrazole; $CONHSO_2R^{10}$; $NHSO_2R^{10}$; hydroxymethylketone; acetoxymethylketone; $CON(R^4)_2$; CN; Het; or

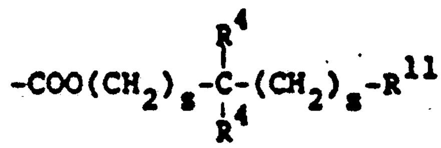

each $R^8$ is independently H or lower alkyl, and is absent when a triple bond is present; $R^9$ is $R^3$,

$(CH_2)_a$-Het,

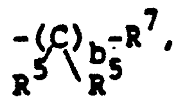

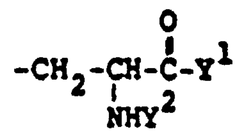
,

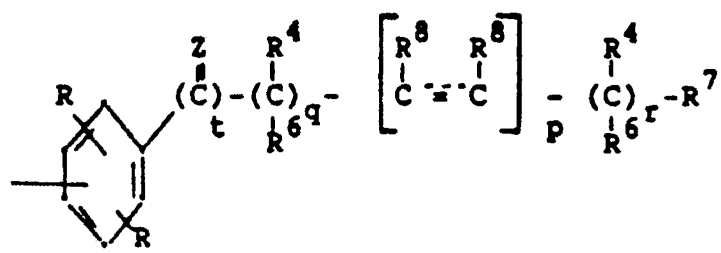

wherein the broken line represents an optional triple bond,

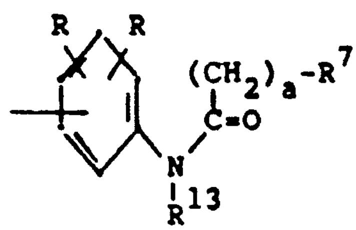
,

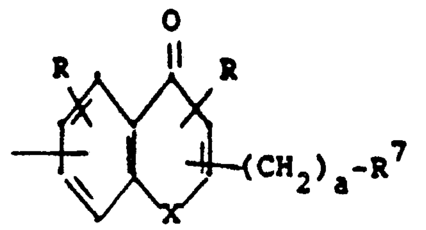
,

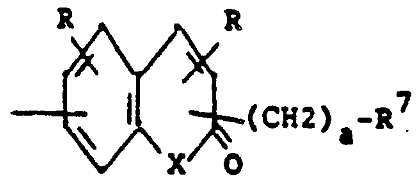
,

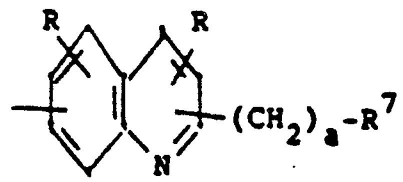
,

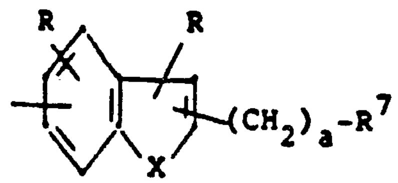
, or

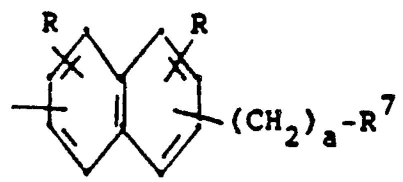
;

each $R^{10}$ is independently OH; $N(R^4)_2$; $CF_3$; lower alkyl; lower alkoxy; phenyl; or phenyl substituted by one or more alkyl or alkoxy groups of 1 to 3 carbon atoms, halogen, hydroxy, $COOR^4$, CN, formyl or lower alklacyl;

each $R^{11}$ is independently

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical $W\text{-}R^{12}$;

each $R^{12}$ independently contains up to 20 carbon atoms and is (1) an alkyl radical or (2) an alkylacyl radical of an organic acyclic or monocyclic carboxylic acid containing not more than one heteroatom in the ring;

each $R^{13}$ is independently H or $R^{10}$;

each W is independently O, S or NH;

each X is independently O, S or $NR^{13}$;

$X^1$ and $X^2$ are each independently O,S, so, $SO_2$, $S(O)=NR^4$, $NR^4$, $NCOR^1$, NCN, or $NCONHR^4$;

$x^3$ is O,S,SO or $SO_2$;

$Y^1$ is OH or the N-terminus of an amino acid such that $Y^1H$ is an essential amino acid:

$Y^2$ is H or the C-terminus of an amino acid such that $Y^2OH$ is an essential amino acid;

Z is O, H and OH, or H and $R^4$;

each a is independently 0 to 4;

each b is independently 1 to 6;
each n is independently 0 to 6;
each p is independently 0 to 2;
each q is independently 0 to 4;
each r is independently 0 to 4;
each s is independently 0 to 3;
each t is independently 0 to 1;
each Het is independently a heterocycylic or heterocyclic or heterobicyclic ring of 5 or 6 atoms each, containing one or more heteroatoms selected from O,N or S, said heterocyclic or heterobicyclic lc ring containing an acidic proton;
and the terms "lower alkyl", "lower alkenyl" and "lower alkoxy" mean $C_{1-7}$ alkyl, $C_{2-7}$, alkenyl and $C_{1-7}$ alkoxy respectively;
or a pharmaceutically acceptable salt thereof, comprising:
esterifying a compound of formula X

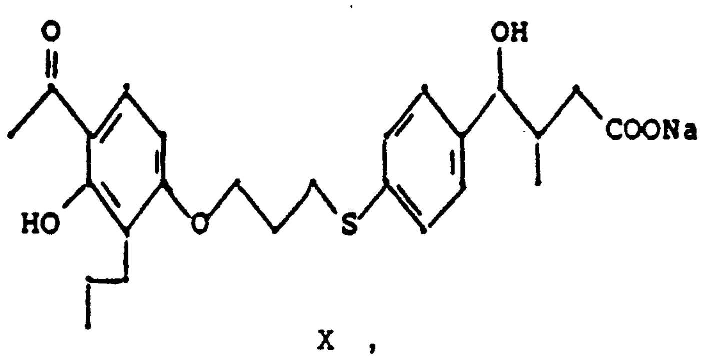

X ,

reacting the thus obtained compound of formula XI

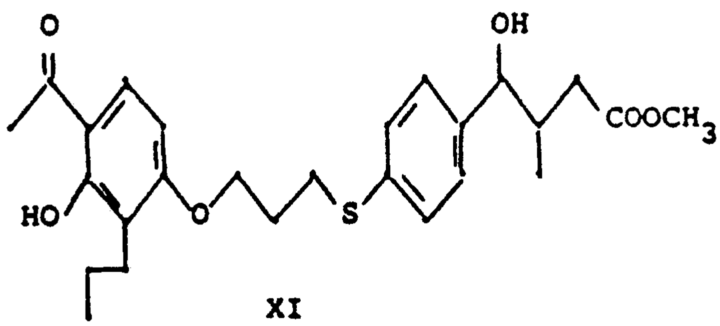

XI

with a thiol of the formula $HSR^9$, wherein $R^9$ is as defined above, in presence of a Lewis acid catalyst in an inert solvent;
hydrolysing the thus obtained compound of formula XII

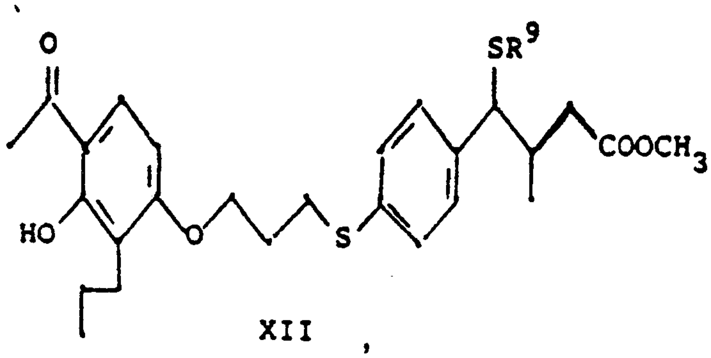

XII ,

wherein $R^9$ is as defined above, with aqueous base to provide the corresponding salt of an acid of formula XIII

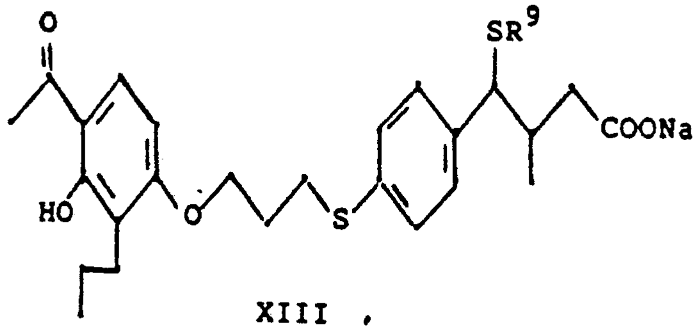

XIII ,

2. A method for preparing a compound as defined in claim 1, comprising reacting a compound of formula XI

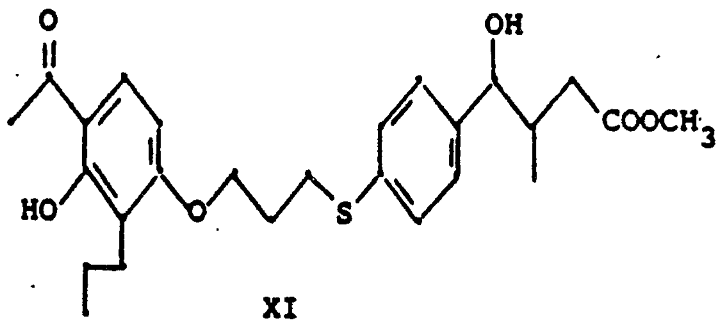

XI

with thiolacetic acid in presence of a Lewis acid catalyst in an inert solvent; reating the thus obtained compound of formula XV

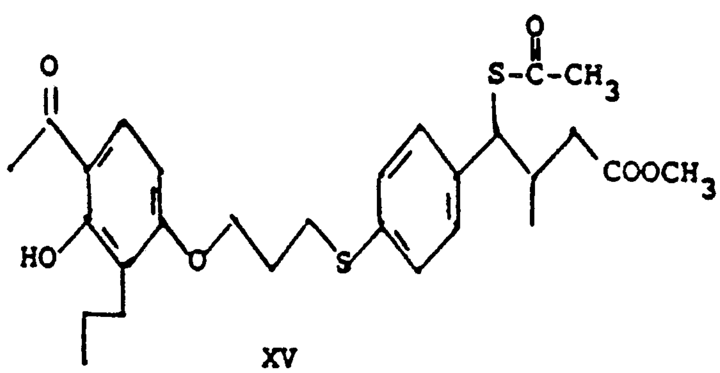

XV

with an alkaline metal alkoxide, followed by acidification; treating the thus obtained compound of formula XVI

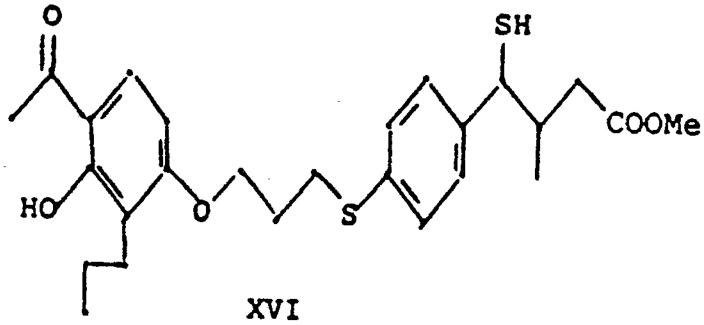

XVI

with a strong base in an inert solvent at low temperature to provide the corresponding thiolactones of the formula XVIIa and XVIIb, respectively,

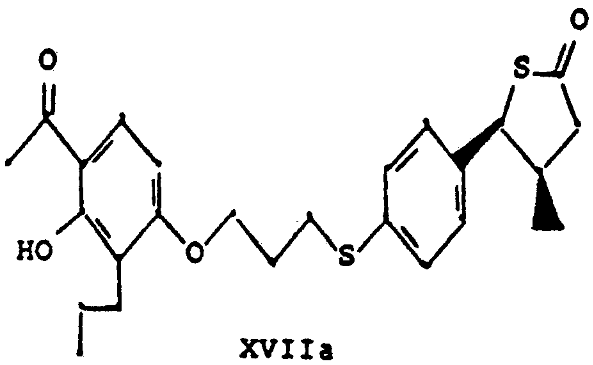

XVIIa

+

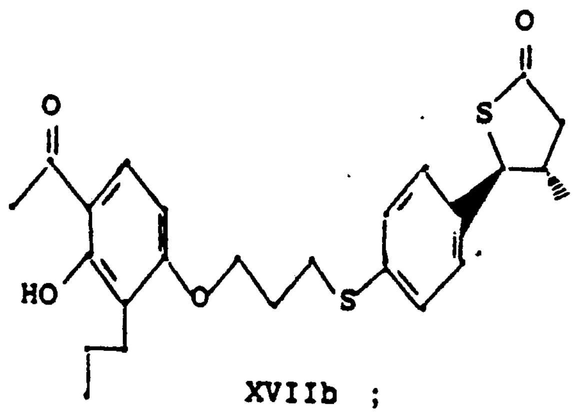

XVIIb ;

separating the isomers and treating the isomer XVIIa and XVIIIb, respectively, with a alkaline metal alkoxide, followed by a reaction with a $\omega$-haloalkanoic acid ester or an $\alpha,\beta$-unsaturated alkenoic acid ester;
hydrolysing the thus obtained compound of formula XII

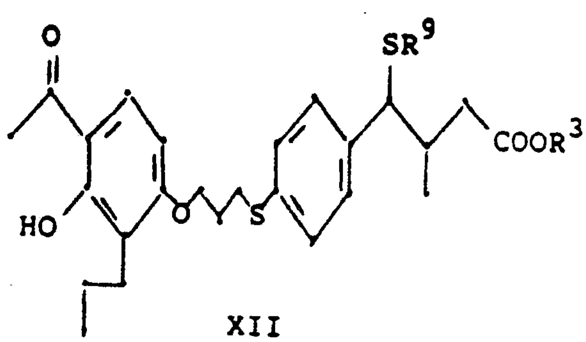

XII

to provide the corresponding diacid of formula XIII.

3. A method according to claim 2, wherein the compounds XVIIa and XVIIb, respectively, are oxidized with organic peracids or hydrogen peroxide to provide the sulfones of formula XVIII

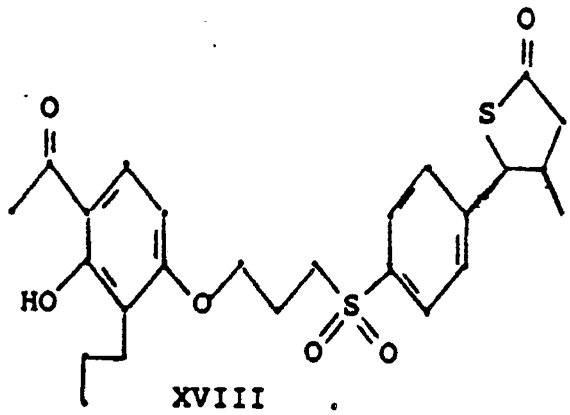

XVIII .

4. A method according to claims 1 to 3 as applied to the preparation of of a compound as defined in claim 1, in which $R^1$ is H or lower alkyl;
each $R_3$ is independently lower alkyl or lower alkenyl;
each $R_5$ is independently H; $OR^2$; or both $R^5$s may be combined to create a doubly bonded oxygen (=O);
each $R^7$ is independently $COOR^4$; CHO; $CH_2OH$; tetrazole; or Het;
$X^1$, $X^2$ and $X^3$ are each independently O, S, SO or $SO_2$;
each n is independently 1 or 2; and each p is O; or a pharmaceutically acceptable salt thereof.

5. A method according to claims 1 to 3 as applied to the preparation of a compound as defined in claim 4, in which each R is independently
H; OH; lower alkyl; trifluormethyl; lower alkoxy; halogen; $N(R^4)_2$;
$-(C=O)R^1$;$CH_2OR^4$; CN; $SR^{10}$; $SOR^{10}$; or $SO_2R^{10}$;
$R^1$ is lower alkyl;
each n is 1; q=O;
or a pharmaceutically acceptable salt thereof.

6. A method according to claims 1 to 3 as applied to the preparation of a compound as defined in claim 5, of the formula:

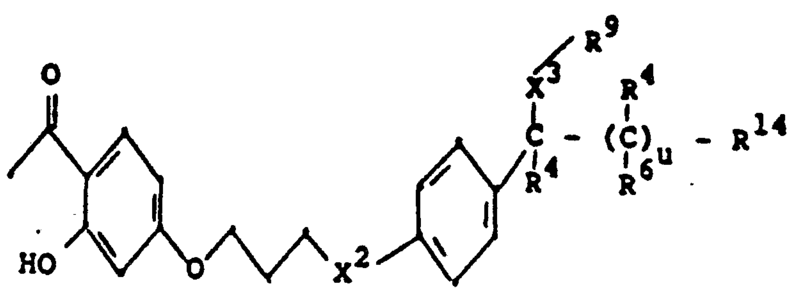

(II)

wherein $R^9$ is

$(CH_2)_a$-Het,

where Het is

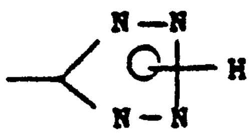
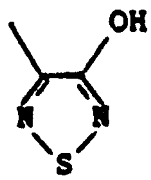
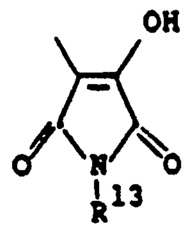

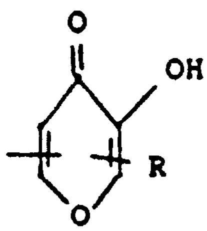
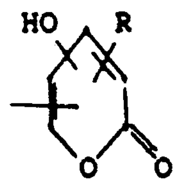
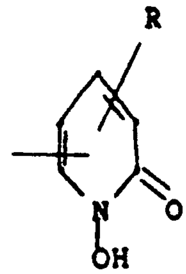

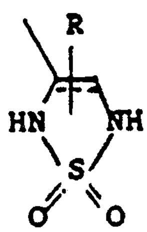
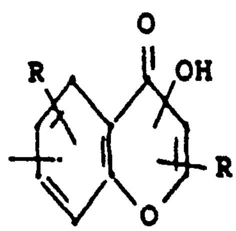
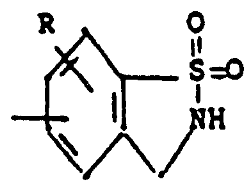

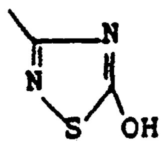 ; wherein the broken line represents an optional double bond.

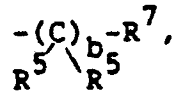

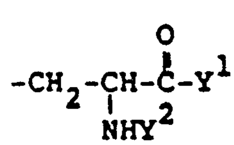 ,

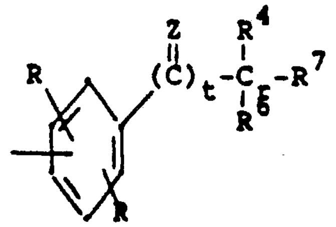

,

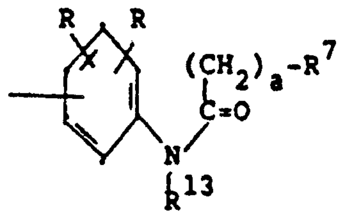

,

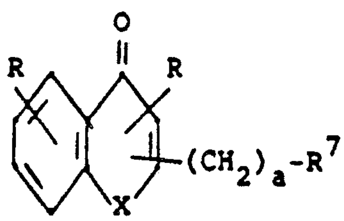

,

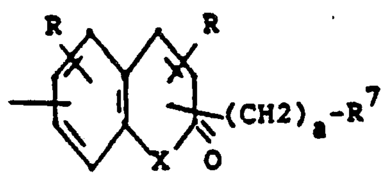

,

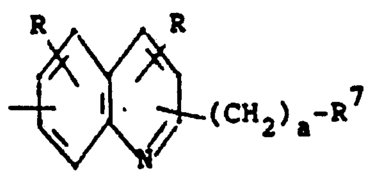

,

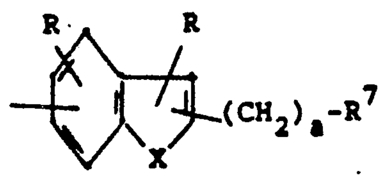

, or

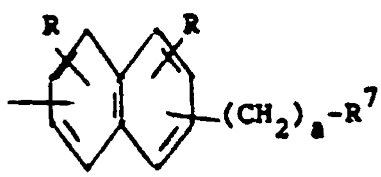

;

$R^{14}$ is $COOR^4$; u is 0 to 4;

or a pharmaceutically acceptable salt thereof.

7. A method according to claims 1 to 3 as applied to the preparation of one of the compounds
7-((γ-(4-((3-(4-acetyl-3-hydroxy-2-propylphenoxy)-propyl)thio)phenyl)-gamma-carboxy-β-methylpropyl) thio)-4-oxo-4-H-1-benzopyran-2-carboxylate disodium salt;
4-((1-(4-((3-(4-acetyl-3-hydroxy-2-propylphenoxy)-propyl)thio)phenyl)-3-carboxypropyl)thio)-gamma-oxobenzenebutanoic acid;
4-((3-(4-acetyl-3-hydroxy-2-propylphenoxy)propyl) thio)-gamma-((3-(carboxyacetyl)amino)phenyl)thio-beta-methylbenzenebutanoic acid;
4-((1-(4-((3-(4-acetyl-3-hydroxy-2-propylphenoxy) propyl)thio)phenyl)-3-carboxypropyl)thio)-beta-methyl-gamma-ozobenzenebutanoic acid;
N(S(γ-(4-((3-(4-acetyl-3-hydroxy-2-propylphenoxy) propyl)thio)phenyl)-gamma-carboxy-β-methylpropyl)-cysteinyl)glycine disodium salt;
4-((3-(4-acetyl-3-hydroxy-2-propylphenoxy)propyl)-thio)-gamma-((2-carboxyethyl)thio)-beta-methyl-benzenebutanoic acid disodium salt;
4-((3-(4-acetyl-3-hydroxy-2-propylphenoxy)propyl) thio)-gamma-(butylthio)-beta-methylbenzenebutanoic acid;
4-((3-(4-acetyl-3-hydroxy-2-propylphenoxy)propyl) thio)-beta-methyl-gamma-((1H-tetrazole-5-yl-methyl) thio)bensenebutanoic acid disodium salt monohydrate;
methyl 4-((3-(4-acetyl-3-hydroxy-2-propylphenoxy)-propyl)thio)-beta-methyl-gamma-((1H-tetrazole-5-yl-methyl)thio)benzenebutanoate sodium salt;
4-((1-(4-((3-(4-acetyl-3-hydroxy-2-propylphenoxy) propyl)thio)phenyl)-3-carboxy-2-methylpropyl)thio)-gamma-hydroxy-beta-methylbenzenebutanoic acid disodium salt monohydrate;
4-((1-(4-((3-(4-acetyl-3-hydroxy-2-propylphenoxy) propyl)thio)phenyl)-3-carboxy-2-methylpropyl) thio)-beta-methyl-gamma-oxobenzenebutanoic acid monohydrate;
4-((3-(4-acetyl-3-hydroxy-2-propylphenoxy)propyl)-thio)-γ-( (4-(3-carboxy-1-hydroxypropyl)phenyl)-thio-benzene butanoic acid, disodium salt, monohydrate;
4-((3-(4-acetyl-3-hydcoxy-2-propylphenoxy)propylthio)-γ-( (2-carboxymethyl)thio)-β-methylbenzenebutanoic acid disodium salt, hemihydrate;
4-((3-(4-acetyl-3-hydroxy-2-propylphenoxy)propyl)thio)-γ-((2-carboxyethyl)thio)-β-methylbenzenebutanoic acid, disodium salt;
4-((3-(4-acetyl-3-hydroxy-2-propylphenoxy)propyl)-sulfonyl-γ-((2-carboxyethyl)thio)-β-methylbenzene-butanoic acid, disodium salt, monohydrate; or
4-((3-(4-acetyl-3-hydroxy-2-propylphenoxy)propyl)thio-γ-((2-N,N-dimethylcacbonylethyl)thio)-β-methyl-benzenebutanoic acid, sodium salt.

8. A process for preparing a pharmaceutical composition useful in antagonizing leukotriene action in mammals, comprising admixing of a compound as prepared in one of the claims 1 to 7 with a pharmaceutically acceptable carrier.

9. A process for preparing a pharmaceutical composition according to claim 8, which additionally comprises admixing with an effective amount of a second active ingredient that is a non-steroidal anti-inflammatory drug; a peripheral analgesic agent; a cyclooxygenase inhibitor; a leukotriene antagonist; a leukotriene inhibitor; an $H_2$-receptor antagonist; an antihistaminic agent; a prostaglandin antagonist or a thromboxane antagonist.

10. A process for preparing a pharmaceutical composition according to claim 9, in which the weight ratio of the compound as prepared in one of the claims 1 to 7 to the second active ingredient ranges from 200:1 to 1:200.

11. The use of a compound as prepared in one of claims 1 to 7 for preparing a composition for use in preventing the synthesis, the action or the release of SRS-A and the leukotrienes $C_4$, $D_4$, $E_4$ and $B_4$ in mammals.

12. The use of a compound as prepared in one of claims 1 to 7 for preparing a composition for use in cytoprotection in mammals in need of cytoprotection.

## Revendications

1. Composé de formule:

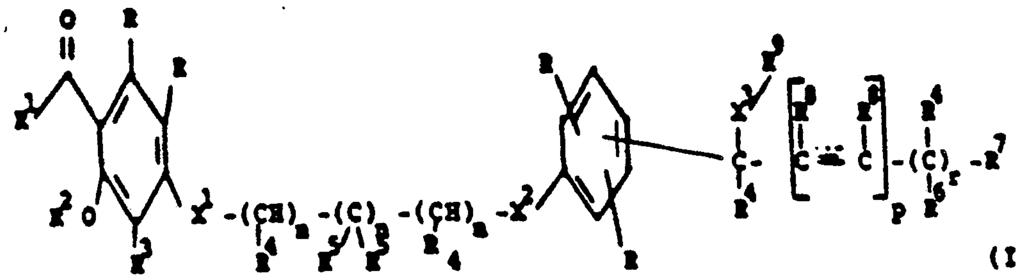

(I)

dans laquelle:
le trait discontinu représente une triple liaison facultative;
chaque R est indépendamment H; OH; un alkyle inférieur; un alcényle inférieur; un trifluorométhyle; un alcoxy inférieur; un phényle; un phényle substitué par un alkyle de 1 à 3 atomes de carbone ou par un halogéne; un benzyle; un phénéthyle; un halogène; $N(R^4)_2$; $-(C=O)R^1$; $CH_2OR^4$; CN; $SR^{10}$; $SOR^{10}$; $SO_2R^{10}$; ou un nitro;
$R^1$ est H ; un alkyle inférieur ; ou un alcoxy inférieur ;
$R^2$ est H ; un alkyle inférieur $R^4CO$ ; ou $R^4OCH_2$ ;
chaque $R^3$ est indépendamment un alkyle inférieur ou un alcényle inférieur ; O
chaque $R^4$ est indépendamment H ou un alkyle inférieur ;
chaque $R^5$ est indépendamment H ; $OR^2$ un alkyle inférieur ; ou les deux $R^5$ peuvent être combinés pour créer un oxygène à double liaison (=O) ou un groupe $=C(R^4)_2$ ;
chaque $R^6$ est indépendamment H ; OH ; ou un alkyle inférieur ;
chaque $R^7$ est indépendamment $COOR^4$ ; CHO ; $CH_2OH$ ; un tétrazole ; $CONHSO_2R^{10}$ ; $NHSO_2R^{10}$ ; une hydroxyméthylcétone ; une acétoxyméthylcétone ; $CON(R^4)_2$ ; CN ; Het ; ou

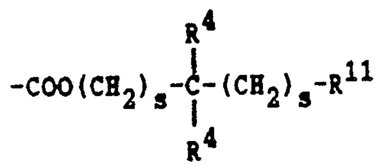

chaque $R^8$ est indépendamment H ou un alkyle inférieur et est absent lorsqu'une triple liaison est présente ;

$R^9$ est $R^3$,

$(CH_2)_a$-Het,

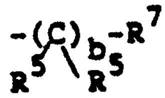,

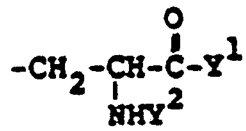,

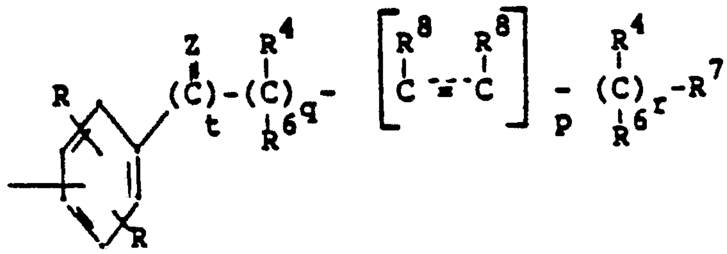

où le pointillé représente une triple liaison facultative,

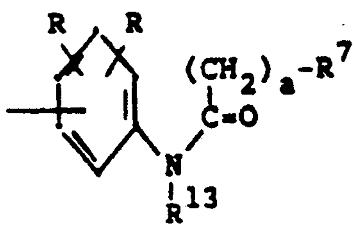,

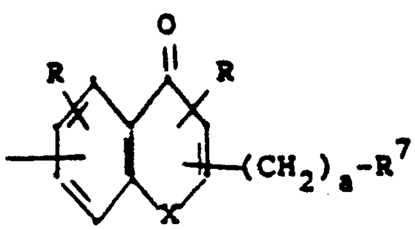,

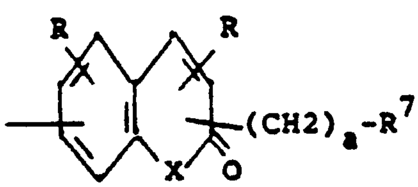,

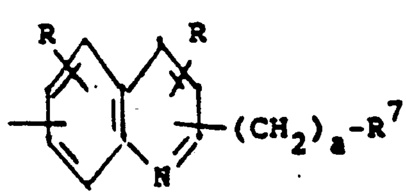,

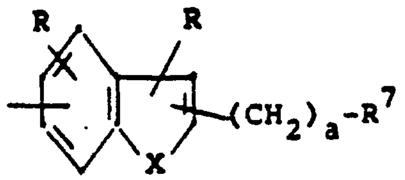

, ou

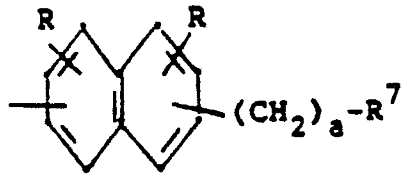

;

chaque $R^{10}$ est indépendamment OH ; $N(R^4)_2$ ; $CF_3$ ; un alkyle inférieur ; un alcoxy inférieur ; un phényle ; ou un phényle substitué par un ou plusieurs groupes alkyles ou alcoxy de 1 à 3 atomes de carbone, halogène, hydroxy, $COOR^4$, CN, formyle ou alkylacyle inférieur ;
chaque $R^{11}$ est indépendamment
A) un radical hétérocyclique monocyclique ou bicyclique contenant 3 à 12 atomes de carbone nucléaires et 1 ou 2 hétéroatomes nucléaires choisis parmi N et S, au moins l'un d'entre eux étant N, et chaque cycle du radical hétérocyclique étant formé de 5 ou 6 atomes, ou
B) le radical $W\text{-}R^{12}$;
chaque $R^{12}$ indépendamment contient jusqu'à 20 atomes de carbone et est (1) un radical alkyle ou (2) un radical alkylacyle d'un acide organique carboxylique acyclique ou monocyclique ne contenant pas plus d'un hétéroatome dans le cycle ;
chaque $R^{13}$ est indépendamment H ou $R^{10}$ ;
chaque W est indépendamment O, S ou NH ;
chaque X est indépendamment O, S ou $NR^{13}$ ;
$X^1$ et $X^2$ sont chacun indépendamment O, S, $SO_2$, $S(O)=NR^4$,
$NR^4$, $NCOR^1$, NCN ou $NCONHR^4$ ;
$X^3$ est O, S, SO ou $SO_2$ ;
$Y^1$ est OH ou l'extrémité N-terminale d'un amino-acide telle que $Y^1H$ soit un amino-acide essentiel ;
$Y^2$ est H ou l'extrémité C-terminale d'un amino-acide telle que $Y^2OH$ soit un amino-acide essentiel ;
Z est O, H et OH ou H et $R^4$ ;
chaque a vaut indépendamment 0 à 4 ;
chaque b vaut indépendamment 1 à 6 ;
chaque n vaut indépendamment 0 à 6 ;
chaque p vaut indépendamment 0 à 2 ;
chaque q vaut indépendamment 0 à 4 ;
chaque r vaut indépendamment 0 à 4 ;
chaque s vaut indépendamment 0 à 3 ;
chaque t vaut indépendamment 0 à 1 ;
chaque Het est indépendamment un noyau hétérocyclique ou hétérobicyclique ayant 5 ou 6 atomes par cycle, contenant un ou plusieurs hétéroatomes choisis parmi O, N ou S, ce noyau hétérocyclique ou hétérobicyclique contenant un proton acide ;
et les termes "alkyle inférieur", "alcényle inférieur" et "alcoxy inférieur" désignent respectivement des groupes alkyles en $C_{1-7}$, alcényles en $C_{2-7}$ et alcoxy en $C_{1-7}$ ;
ou un sel pharmaceutiquement acceptable de celui-ci.

2. Composé selon la revendication 1, où
$R^1$ est H ou un alkyle inférieur ;
$R^2$ est H ;
chaque $R^3$ est indépendamment un alkyle inférieur ou un alcényle inférieur ;
chaque $R^5$ est indépendamment H ; $OR^2$ ou les deux $R^5$ peuvent être combinés pour créer un oxygène à double liaison (=O);
chaque $R^7$ est indépendamment $COOR^4$ ; CHO ; $CH_2OH$ ; un tétrazole ; ou Het ;

$X^1$, $X^2$ et $X^3$ sont chacun indépendamment O, S, SO ou $SO_2$ ;
chaque n vaut indépendamment 1 ou 2 ; et chaque p vaut O ; ou un sel pharmaceutiquement acceptable de celui-ci.

**3.** Composé selon la revendication 2 où
chaque R est indépendamment H ; OH ; un alkyle inférieur ; un trifluorométhyle ; un alcoxy inférieur ; un halogène ; $N(R^4)_2$ ; $-(C=O)R^1$ ; $CH_2OR^4$ ; CN ; $SR^{10}$ ; $SOR^{10}$ ; ou $SO_2R^{10}$ ;
$R^1$ est un alkyle inférieur ;
chaque n vaut 1 ; q vaut 0 ;
ou un sel pharmaceutiquement acceptable de celui-ci.

**4.** Composé selon la revendication 3 de formule :

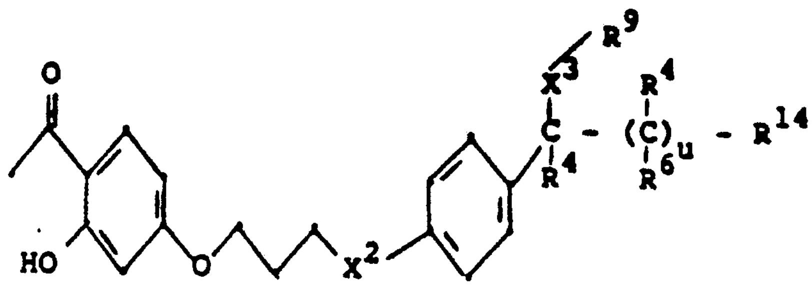

(II)

dans laquelle $R^9$ est

$(CH_2)_a$-Het

où Het est

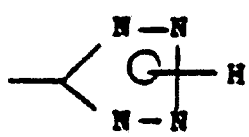
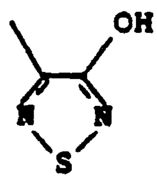
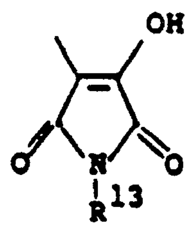

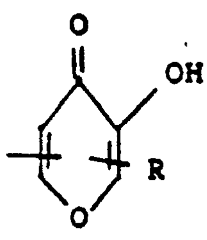
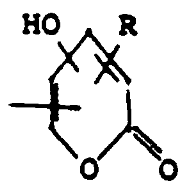
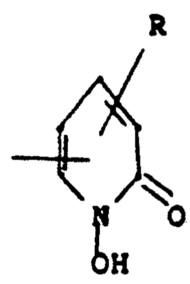

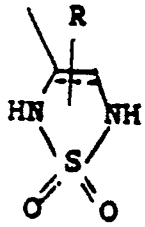
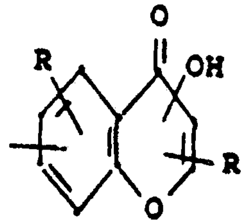
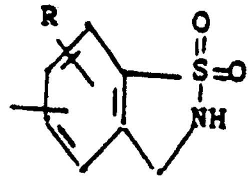

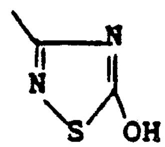 ; où le trait discontinu représente une double liaison facultative.

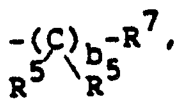

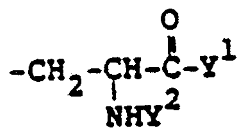 ,

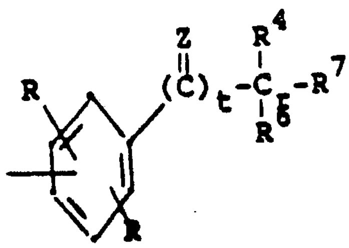 ,

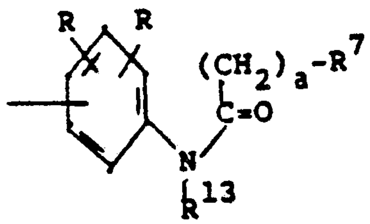 ,

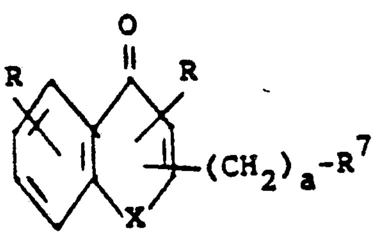 ,

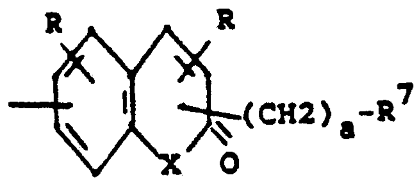 ,

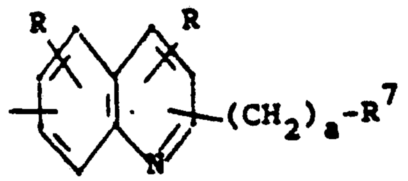 ,

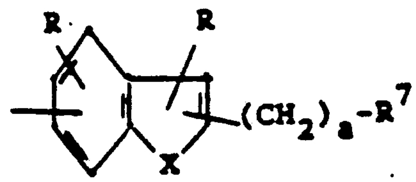 , ou

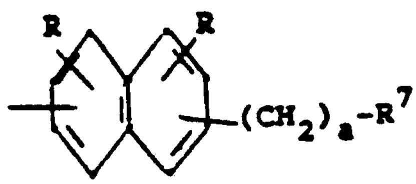 ;

$R^{14}$ est $COOR^4$ ; u vaut 0 à 4 ;
ou un sel pharmaceutiquement acceptable de celui-ci.

**5.** Composé selon la revendication 1 qui est ;
le sel disodique du 7((γ-(4-((3-(4-acétyl-3-hydroxy-2-propyl-phénoxy)propyl)thio)phényl)-γ-carboxy-β-méthylpropyl)thio)-4-oxo-4-H-1-benzopyranne-2-carboxylate ;
l'acide 4-((1-(4-((3-(4-acétyl-3-hydroxy-2-propylphénoxy)propyl) thio)phényl)-3-carboxypropyl)thio)-γ-oxobenzènebutanoïque ;
l'acide 4-((3-(4-acétyl-3-hydroxy-2-propylphénoxy)propyl)thio)-γ-((3-(carboxyacétyl)amino)phényl)thio-β-méthylbenzènebutanoïque ;
l'acide 4-((1-(4-((3-(4-acétyl-3-hydroxy-2-propylphénoxy)propyl) thio)phényl)-3-carboxypropyl)thio)-β-méthyl-γ-oxobenzènebutanoïque ;
le sel disodique de la N-(S-(γ-(4-((3-(4-acétyl-3-hydroxy-2-propyl-phénoxy)propyl)thio)phényl)-γ-carboxy-β-méthylpropyl)cystéinyl) glycine ;
le sel disodique de 1'acide 4-((3-(4-acétyl-3-hydroxy-2-propyl-phénoxy)propyl)thio)-γ-((2-carboxyéthyl)-thio)-β-méthylbenzène-butanoïque ;
l'acide 4-((3-(4-acétyl-3-hydroxy-2-propylphénoxy)propyl)thio)-γ-(butylthio)-β-méthylbenzènebutanoïque ;
le sel disodique monohydraté de l'acide 4-((3-(4-acétyl-3-hydroxy-2-propylphénoxy)propyl)thio)-β-méthyl-γ-((1H-tétrazole-5-ylméthyl) thio)benzènebutanoïque ;
le sel de sodium du 4-((3-(4-acétyl-3-hydroxy-2-propylphénoxy) propyl)thio)-β-méthyl-γ-((1H-tétrazole-5-ylméthyl)thio)benzène-butanoate de méthyle ;
le sel disodique monohydraté de l'acide 4-((1-(4-((3-(4-acétyl-3-hydroxy-2-propylphénoxy)propyl)thio)-phényl)-3-carboxy-2-méthyl-propyl)thio)-γ-hydroxy-β-méthylbenzènebutanoïque ;
l'acide 4-((1-(4-((3-(4-acétyl-3-hydroxy-2-propylphénoxy)propyl) thio)phényl)-3-carboxy-2-méthylpropyl)-β-méthyl-γ-oxobenzène-butanoïque monohydraté ;
le sel disodique monohydraté de l'acide 4-((3-(4-acétyl-3-hydroxy-2-propylphénoxy)propyl)thio)-γ-((4-(3-carboxy-1-hydroxypropyl)phényl) thio)benzènebutanoïque ;
le sel disodique hémihydraté de l'acide 4-((3-(4-acétyl-3-hydroxy-2-propylphénoxy)propyl)thio)-γ-((2-

carboxyméthyl)thio)-β-méthyl-benzènebutanoïque ;
le sel disodique de l'acide 4-((3-(4-acétyl-3-hydroxy-2-propyl-phénoxy)propyl)thio)-γ-((2-carboxyéthyl)-thio)-β-méthylbenzène-butanoïque ;
le sel disodique monohydraté de l'acide 4-((3-(4-acétyl-3-hydroxy-2-propylphénoxy)propyl)sulfonyl)-γ-(-(2-carboxyéthyl)thio)-β-méthyl-benzènebutanoïque ; ou
le sel de sodium de l'acide 4-((3-(4-acétyl-3-hydroxy-2-propyl-phénoxy)propyl)thio)-γ-((2-N,N-diméthyl-carbonyléthyl)thio)-β-méthylbenzènebutanoïque.

6. Composition pharmaceutique utile pour établir un effet d'antagonisme des leucotriènes chez les mammifères, comprenant un composé selon l'une quelconque des revendications 1 à 5 et un véhicule pharmaceutiquement acceptable.

7. Composition selon la revendication 6 comprenant de plus une quantité efficace d'un second ingrédient actif qui est un médicament anti-inflammatoire non stéroïdien ; un agent analqésique périphérique ; un inhibiteur de la cyclooxygénase ; un antaoniste des leucotriènes ; un inhibiteur des leucotriènes ; un antagoniste des récepteurs $H_2$ ; un agent antihistaminique ; un antagoniste des prostaglandines ou un antagoniste du thromboxane.

8. Composition selon la revendication 7, dans laquelle le rapport pondéral du composé selon l'une quelconque des revendications 1 à 5 au second ingrédient actif se situe entre 200/1 et 1/200.

9. Composé selon l'une quelconque des revendications 1 à 5 ou composition selon l'une quelconque des revendications 6 à 8 pour l'emploi dans la prévention de la synthèse, de l'action ou de la libération de la SRS-A et des leucotriènes $C_4$, $D_4$, $E_4$ et $B_4$ chez les mammifères.

10. Composé selon l'une quelconque des revendications 1 à 5 ou composition selon l'une quelconque des revendications 6 à 8 pour l'emploi dans la cytoprotection de mammifères nécessitant une cytoprotection.

Revendications pour l'Etat contractant suivant: AT

1. Procéde pour la préparation d'un composé de formule:

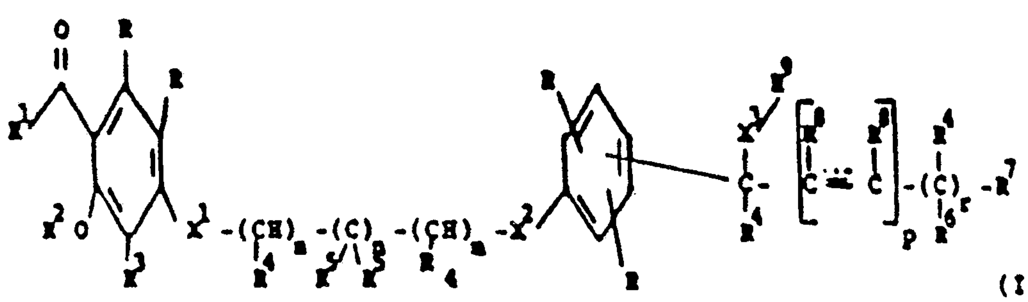

(I)

dans laquelle :
le trait discontinu représente une triple liaison facultative ;
chaque R est indépendamment H ; OH ; un alkyle inférieur ; un alcényle inférieur ; un trifluorométhyle ; un alcoxy inférieur ; un phényle ; un phényle substitué par un alkyle de 1 à 3 atomes de carbone ou par un halogène ; un benzyle ; un phénéthyle ; un halogène ; $N(R^4)_2$ ; $-(C=O)R^1$ ; $CH_2OR^4$ ; CN ; $SR^{10}$ ; $SOR^{10}$ ; $SO_2R^{10}$ ; ou un nitro ; O
$R^1$ est H ; un alkyle inférieur ; ou un alcoxy inférieur ;
$R^2$ est H ; un alkyle inférieur ; $R^4CO$ ; ou $R^4OCH_2$ ;
chaque $R^3$ est indépendamment un alkyle inférieur ou un alcényle inférieur ;
chaque $R^4$ est indépendamment H ou un alkyle inférieur ;
chaque $R^5$ est indépendamment H ; $OR^2$ ; un alkyle inférieur ; ou les deux $R^5$ peuvent être combinés pour créer un oxygène à double liaison (=O) ou un groupe $=C(R^4)_2$ ;
chaque $R^6$ est indépendamment H ; OH ; ou un alkyle inférieur ;
chaque $R^7$ est indépendamment $COOR^4$ ; CHO ; $CH_2OH$ ; un tétrazole ; $CONHSO_2R^{10}$ ; $NHSO_2R^{10}$ ;

une hydroxyméthylcétone ; une acétoxyméthylcétone ; $CON(R^4)_2$ ; CN ; Het ; ou

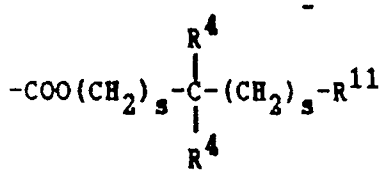

chaque $R^8$ est indépendamment H ou un alkyle inférieur et est absent lorsqu'une triple liaison est présente ;

$R^9$ est $R^3$,

$(CH_2)_a$-Het,

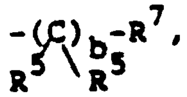

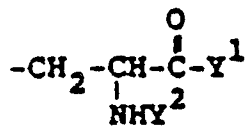 ,

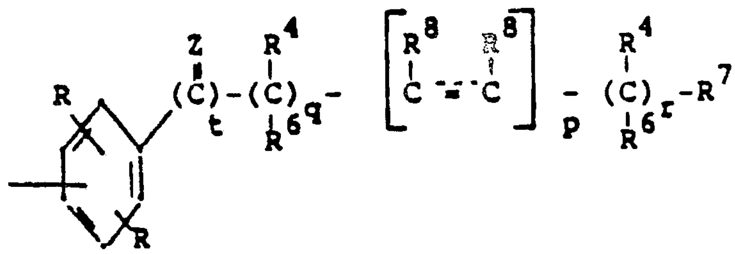

où le pointillé représente une triple liaison facultative,

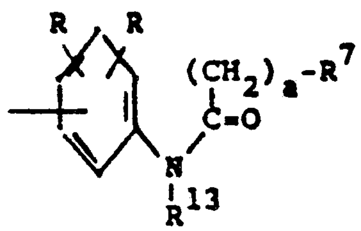 ,

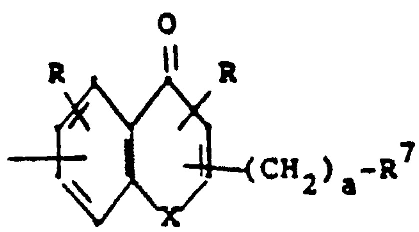 ,

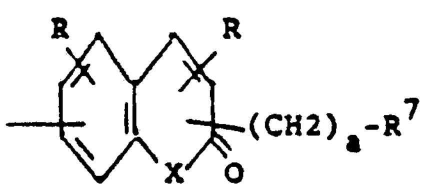 ,

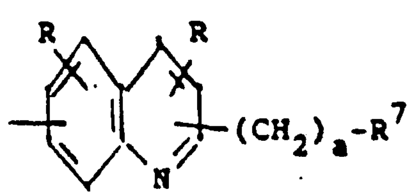 ,

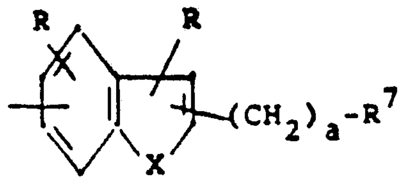

, ou

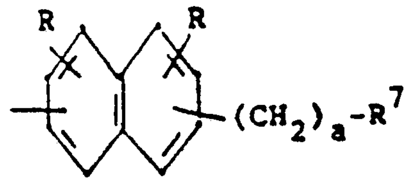

;

chaque $R^{10}$ est indépendamment OH ; $N(R^4)_2$ ; $CF_3$ ; un alkyle inférieur ; un alcoxy inférieur ; un phényle ; ou un phényle substitué par un ou plusieurs groupes alkyles ou alcoxy de 1 à 3 atomes de carbone, halogène, hydroxy, $COOR^4$, CN, formyle ou alkylacyle inférieur ;

chaque $R^{11}$ est indépendamment

A) un radical hétérocyclique monocyclique ou bicyclique contenant 3 à 12 atomes de carbone nucléaires et 1 ou 2 hétéroatomes nucléaires choisis parmi N et S, au moins l'un d'entre eux étant N, et chaque cycle du radical hétérocyclique étant formé de 5 ou 6 atomes, ou

B) le radical $W-R^{12}$ ;

chaque $R^{12}$ indépendamment contient jusqu'à 20 atomes de carbone et est (1) un radical alkyle ou (2) un radical alkylacyle d'un acide organique carboxylique acyclique ou monocyclique ne contenant pas plus d'un hétéroatome dans le cycle ;

chaque $R^{13}$ est indépendamment H ou $R^{10}$ ;

chaque W est indépendamment O, S ou NH ;

chaque X est indépendamment O, S ou $NR^{13}$ ;

$X^1$ et $X^2$ sont chacun indépendamment O, S, SO, $SO_2$, $S(O)=NR^4$, $NR^4$, $NCOR^1$, NCN ou $NCONHR^4$ ;

$X^3$ est O, S, SO ou $SO_2$ ;

$Y^1$ est OH ou l'extrémité N-terminale d'un amino-acide telle que $Y^1H$ soit un amino-acide essentiel ;

$Y^2$ est OH ou l'extrémité C-terminale d'un amino-acide telle que $Y^2OH$ soit un amino-acide essentiel ;

Z est O, H et OH ou H et $R^4$ ;

chaque a vaut indépendamment 0 à 4 ;

chaque b vaut indépendamment 1 à 6 ;

chaque n vaut indépendamment 0 à 6 ;

chaque p vaut indépendamment 0 à 2 ;

chaque q vaut indépendamment 0 à 4 ;

chaque r vaut indépendamment 0 à 4 ;

chaque s vaut indépendamment 0 à 3 ;

chaque t vaut indépendamment 0 à 1 ;

chaque Het est indépendamment un noyau hétérocyclique ou hétérobicyclique ayant 5 ou 6 atomes par cycle, contenant un ou plusieurs hétéroatomes choisis parmi O, N ou S, ce noyau hétérocyclique ou hétérobicyclique contenant un proton acide ;

et les termes "alkyle inférieur", "alcényle inférieur" et "alcoxy inférieur" désignent respectivement des groupes alkyles en $C_{1-7}$, alcényles en $C_{2-7}$ et alcoxy en $C_{1-7}$ ;

ou un sel pharmaceutiquement acceptable de celui-ci , qui comprend:

l'estérification d'un composé de formule X

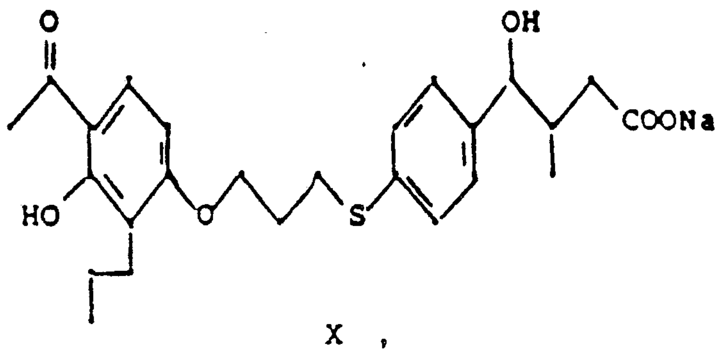

X ,

la réaction du composé ainsi obtenu de formule XI

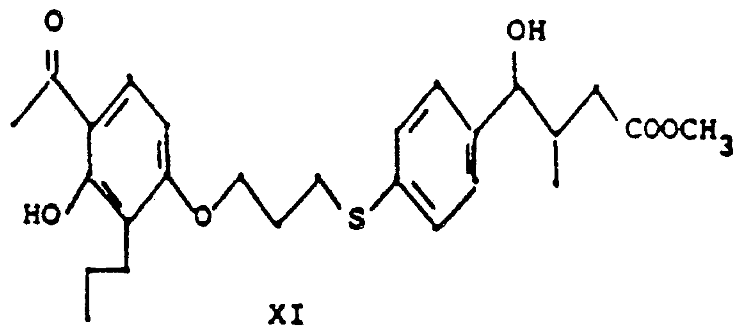

XI

avec un thiol de formule $HSR^9$, dans laquelle $R^9$ est tel que défini ci-dessus, en présence d'un catalyseur acide de Lewis dans un solvant inerte;
l'hydrolyse du composé ainsi obtenu de formule XII

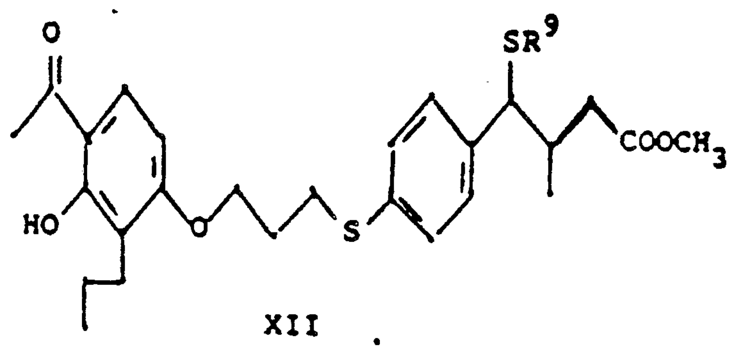

XII .

dans laquelle $R^9$ est tel que défini ci-dessus, avec une base aqueuse pour fournir le sel correspondant d'un acide de formule XIII

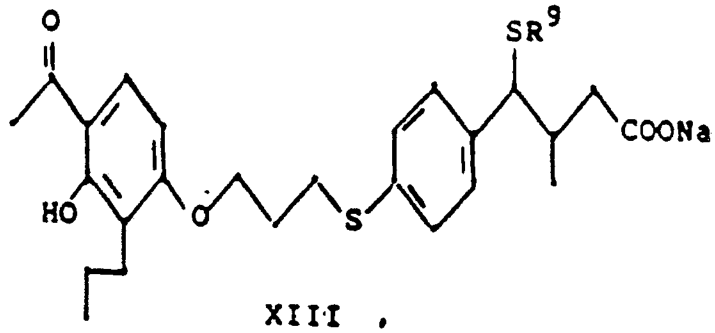

XIII ,

2. Procédé pour la préparation d'un composé selon la revendication 1, qui comprend la réaction d'un composé de formule XI

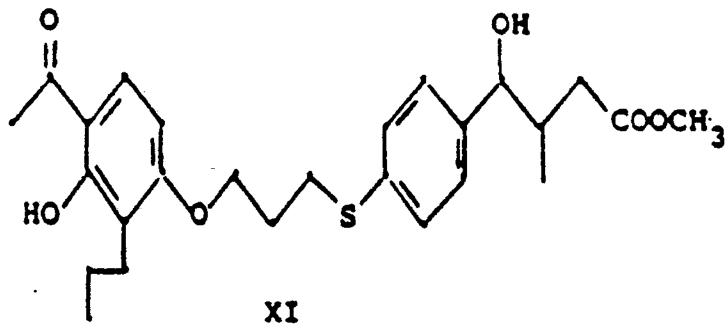

XI

avec l'acide thio-acétique en présence d'un catalyseur acide de Lewis dans un solvant inerte; la réaction du composé ainsi obtenu de formule XV

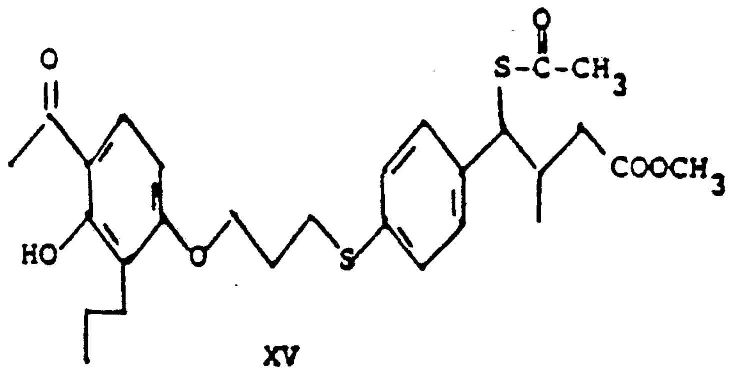

XV

avec un alcoolate de métal alcalin, suivie d'une acidification; le traitement du composé ainsi obtenu de formule XVI

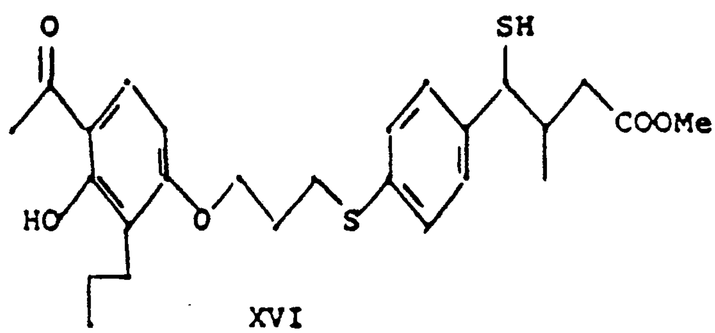

XVI

avec une base forte dans un solvant inerte à basse température pour fournir respectivement les thiolactones correspondantes de formules XVIIa et XVIIb,

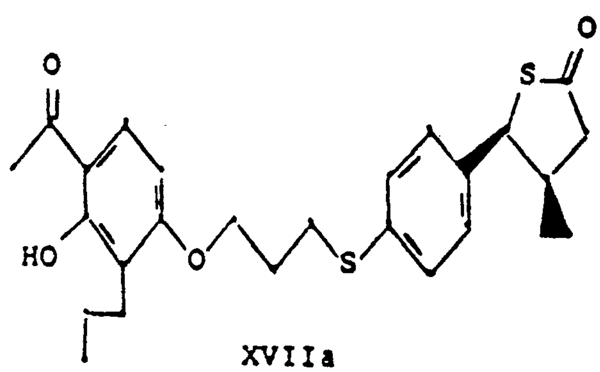

XVIIa

+

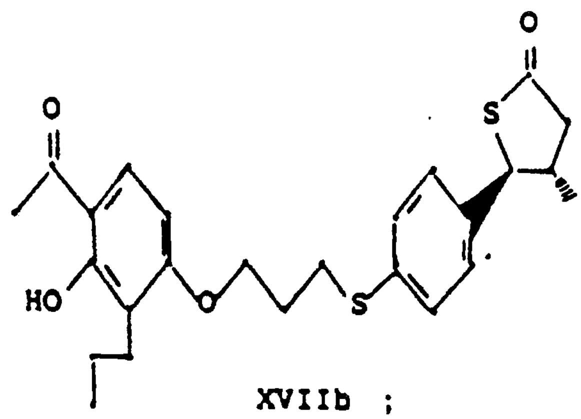

XVIIb ;

la séparation des isomères et le traitement de l'isomère XVIIa et XVIIb respectivement, avec un alcoolate de métal alcalin , celui-ci étant suivi par une réaction avec un ester d'un acide ω-halogéno-alcanoïque ou un ester d'un acide alcénoïque α,β-insaturé:
l'hydrolyse du composé ainsi obtenu de formule XII

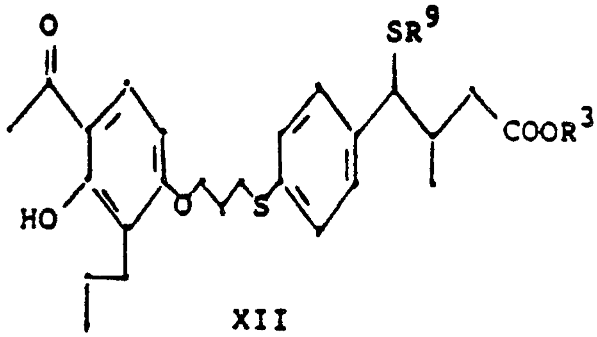

XII

pour fournir le diacide correspondant de formule XIII.

3. Procédé selon la revendication 2, dans lequel on oxyde les compposés XVIIa et XVIIb respectivement, avec des peracides organiques ou le peroxyde d'hydrogène pour fournir les sulfones de formule XVIII

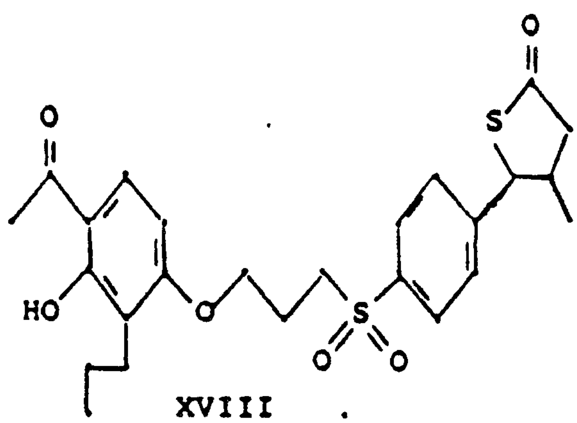

XVIII .

4. Procédé selon les revendications 1 à 3, appliqué à la préparation d'un composé tel que défini dans la revendication 1, dans lequel
$R^1$ est H ou un alkyle inférieur;
chaque $R^3$ est indépendamment un alkyle inférieur ou un alcényle inférieur;
chaque $R^5$ est indépendamment H; $OR^2$; ou les deux $R^5$ peuvent être combinés pour créer un oxygène à double liaison (=O);
chaque $R^7$ est indépendamment $COOR^4$; CHO; $CH_2OH$; un tétrazole; ou Het;

$X^1$, $X^2$, $X^3$ sont chacun indépendamment O, S, SO ou$SO_2$;
chaque n vaut indépendamment 1 ou 2; et chaque p vaut O; ou d'un sel pharmaceutiquement acceptable de celui-ci.

**5.** Procédé selon les revendications 1 à 3, appliqué à la préparation d'un composé tel que défini dans la revendication 4, dans lequel
chaque R est indépendamment H; OH; un alkyle inférieur; un trifluorométhyle; un alcoxy inférieur; un halogène; $N(R^4)_2$; $-(C=O)R^1$; $CH_2OR^4$; CN; $SR^{10}$; $SOR^{10}$, ou $SO_2R^{10}$;
$R^1$ est un alkyle inférieur;
chaque n vaut 1; q vaut O;
ou d'un sel pharmaceutiquement acceptable de celui-ci.

**6.** Procédé selon les revendications 1 à 3, appliqué à la préparation d'un composé tel que défini dans la revendication 5,de formule:

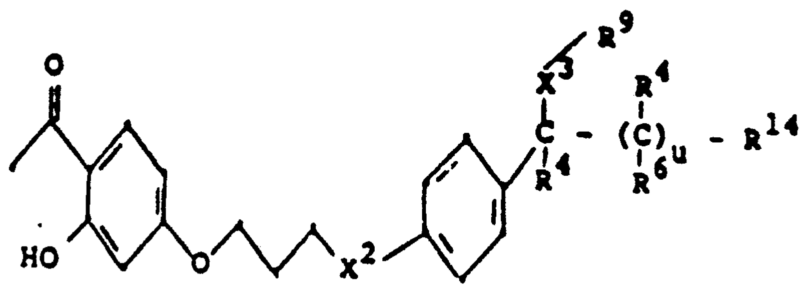

(II)

dans laquelle $R^9$ est

$(CH_2)_a$-Het,

où Het est

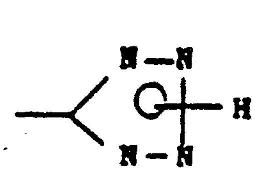
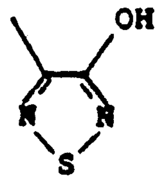
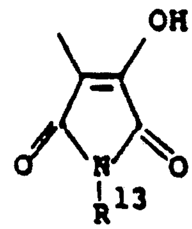

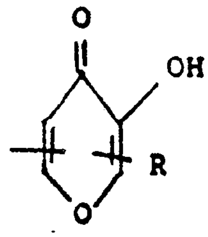
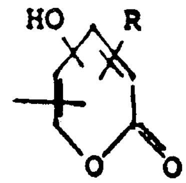
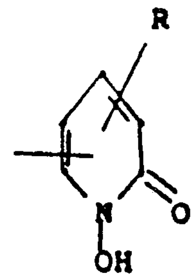

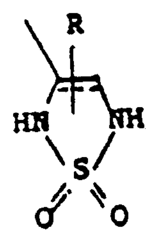
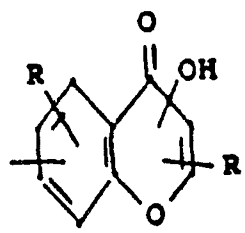
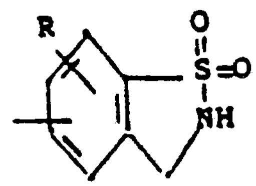

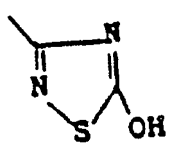 ; où le trait discontinu représente une double liaison facultative.

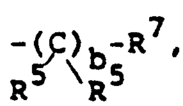

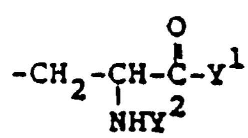 ,

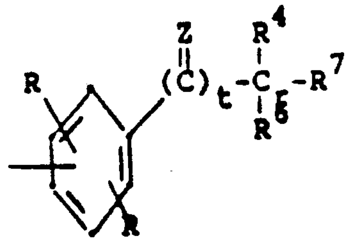 ,

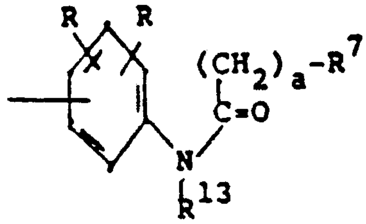 ,

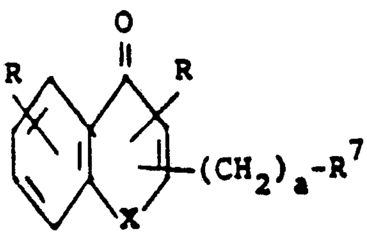 ,

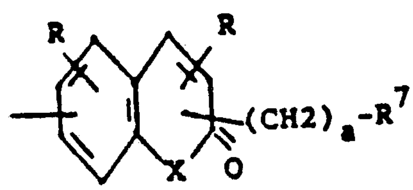 ,

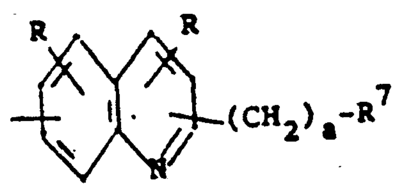 ,

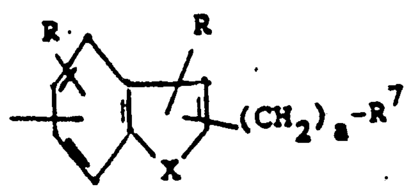 , ou

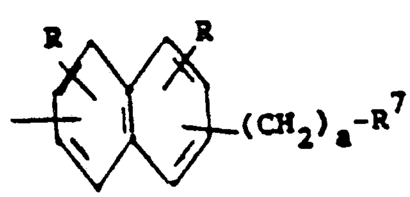 ;

$R^{14}$ est $COOR^4$; u vaut 0 à 4;
ou d'un sel pharmaceutiquement acceptable de celui-ci.

**7.** Procédé selon les revendications 1 à 3, appliqué à la préparation d'un des composés suivants
le sel disodique du 7-((4-(4-((3-(4-acétyl-3-hydroxy-2-propyl-phénoxy)propyl)thio)phényl)-γ-carboxy-β-méthylpropyl)-4-oxo-4-H-1-benzopyranne-2-carboxylate ;
l'acide 4-((1-(4-((3-(4-acétyl-3-hydroxy-2-propylphénoxy)propyl) thio)phényl)-3-carboxypropyl)thio)-γ-oxobenzènebutanoïque ;
l'acide 4-((3-(4-acétyl-3-hydroxy-2-propylphénoxy)propyl)thio)-γ-((3-(carboxyacétyl)amino)phényl)thio-β-méthylbenzènebutanoïque ;
l'acide 4-((1-(4-((3-(4-acétyl-3-hydroxy-2-propylphénoxy)propyl) thio)phényl)-3-carboxypropyl)thio)-β-méthyl-γ-oxobenzènebutanoïque ;
le sel disodique de la N-(S-(γ-(4-((3-(4-acétyl-3-hydroxy-2-propyl-phénoxy)propyl)thio)phényl)-γ-carboxy-β-méthylpropyl)cystéinyl) glycine ;
le sel disodique de l'acide 4-((3-(4-acétyl-3-hydroxy-2-propyl-phénoxy)propyl)thio-γ-((2-carboxyéthyl)-thio)-β-méthylbenzènebutanoïque ;
l'acide 4-((3-(4-acétyl-3-hydroxy-2-propylphénoxy)propyl)thio)-γ-(butylthio)-β-méthylbenzènebutanoïque ;
le sel disodique monohydraté de l'acide 4-((3-(4-acétyl-3-hydroxy-2-propylphénoxy)propyl)thio)-β-méthyl-γ-((1H-tétrazole-5-ylméthyl) thio)benzènebutanoïque ;
le sel de sodium du 4-((3-(4-acétyl-3-hydroxy-2-propylphénoxy) propyl)thio)-β-méthyl-γ-((1H-tétrazole-5-ylméthyl)thio)benzènebutanoate de méthyle ;
le sel disodique monohydraté de l'acide 4-((1-(4-((3-(4-acétyl-3-hydroxy-2-propylphénoxy)propyl)thio)-phényl)-3-carboxy-2-méthyl-propyl)thio)-γ-hydroxy-β-méthylbenzènebutanoïque ;
l'acide 4-((1-(4-((3-(4-acétyl-3-hydroxy-2-propylphénoxy)propyl) thio)phényl)-3-carboxy-2-méthylpropyl)-thio)-β-méthyl-γ-oxobenzènebutanoique monohydraté ;
le sel disodique monohydraté de l'acide 4-((3-(4-acétyl-3-hydroxy-2-propylphénoxy)propyl)thio)-γ-((4-(3-carboxy-1-hydroxypropyl)phényl) thio)benzènebutanoïque ;
le sel disodique hémihydraté de l'acide 4-((3-(4-acétyl-3-hydroxy-2-propylphénoxy)propyl)thio)-γ-((2-carboxyméthyl)thio)-β-méthylbenzènebutanoïque ;
le sel disodique de l'acide 4-((3-(4-acétyl-3-hydroxy-2-propyl-phénoxy)propyl)thio)-γ-((carboxyéthyl)-thio)-β-méthylbenzènebutanoïque ;
le sel disodique monohydraté de l'acide 4-((3-(4-acétyl-3-hydroxy-2-propylphénoxy)propyl)sulfonyl)-γ-(-(2-carboxyéthyl)thio)-β-méthyl-benzènebutanoïque ; ou
le sel de sodium de l'acide 4-((3-(4-acétyl-3-hydroxy-2-propyl-phénoxy)propyl)thio)-γ-((2-N,N-diméthyl-carbonyléthyl)thio-β-méthyl-benzènebutanoïque.

**8.** Procédé pour la préparation d'une composition pharmaceutique utile pour établir un effet d'antagonisme des leucotriènes chez les mammifères, qui comprend le mélange d'un composé tel que préparé selon l'une des revendications 1 à 7 avec un véhicule pharmaceutiquement acceptable .

**9.** Procédé pour la préparation d'une composition pharmaceutique selon la revendication 8, qui comprend en outre le mélange avec une quantité efficace d'un second ingrédient actif qui est un médicament anti-inflammatoire non stéroïdien; un agent analgésique périphérique; un inhibiteur de la cyclooxygénase; un antagoniste des leucotriènes; un inhibiteur des leucotriènes; un antagoniste des récepteurs $H_2$; un agent antihistaminique; un antagoniste des prostaglandines ou un antagoniste du thromboxane.

**10.** Procédé pour la préparation d'une composition pharmaceutique selon la revendication 9, dans lequel le rapport pondéral du composé tel que préparé selon l'une des revendications 1 à 7 au second ingrédient actif se situe entre 200/1 et 1/200.

**11.** Utilisation d'un composé tel que préparé selon l'une des revendications 1 à 7 , pour la préparation d'une composition à utiliser dans la prévention de la synthèse , de l'action ou de la libération de la SRS-A et des leucotriènes $C_4$, $D_4$,$E_4$ et $B_4$ chez les mammifères.

**12.** Utilisation d'un composé tel que préparé selon l'une des revendications 1 à 7, pour la préparation d'une composition à utiliser dans la cytoprotection de mammifères nécessitant une cytoprotection .

## Ansprüche

1. Verbindung der Formel

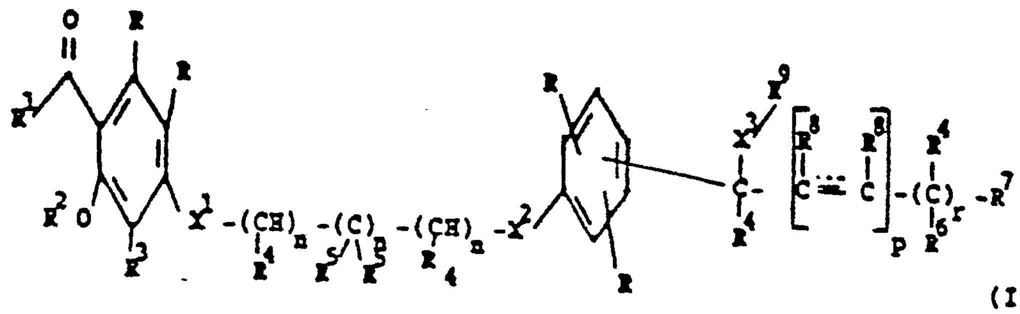

(I)

worin
die gestrichelte Linie eine mögliche Dreifachbindung darstellt;
R jeweils unabhängig H; OH; Niederalkyl; Niederalkenyl, Trifluormethyl; Niederalkoxy; Phenyl; durch Alkyl mit 1 bis 3 Kohlenstoffatomen oder durch Halogen substituiertes Phenyl; Benzyl; Phenethyl; Halogen; $N(R^4)_2$; $-(C=O)R^1$; $CH_2OR^4$; CN; $SR^{10}$; $SOR^{10}$; $SO_2R^{10}$ oder Nitro ist;
$R^1$ H; Niederalkyl oder Niederalkoxy ist;
$R^2$ H; Niederlakyl; $R^4CO$ oder $R^4OCH_2$ ist;
$R^3$ jeweils unabhängig Niederalkyl oder Niederalkenyl ist;
$R^4$ jeweils unabhängig H oder Niederalkyl ist;
$R^5$ jeweils unabhängig H; $OR^2$; Niederalkyl ist oder beide $R^5$ unter Bildung eines doppelt gebundenen Sauerstoff (=O) oder eine $=C(R^4)_2$-Gruppe zusammengenommen werden können;
$R^6$ jeweils unabhängig H; OH oder Niederalkyl ist;
$R^7$ jeweils unabhängig $COOR^4$; CHO; $CH_2OH$; Tetrazol; $CONHSO_2R^{10}$; $NHSO_2R^{10}$; Hydroxymethylketon; Acetoxy-methylketon; $CON(R^4)_2$; CN; Het oder

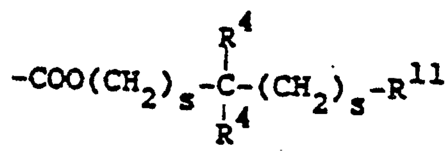

ist;

$R^8$ jeweils unabhängig H oder Niederalkyl ist oder fehlt, wenn eine Dreifachbindung vorhanden ist;

$R^8$ $R^3$,

$(CH_2)_a$-Het,

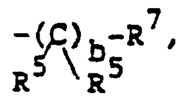

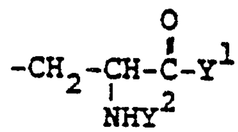

,

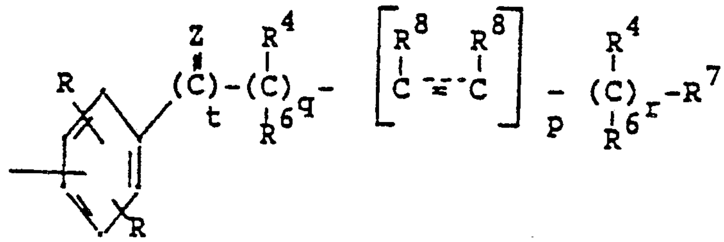

worin die gestrichelte Linie eine mögliche Dreifachbindung darstellt,

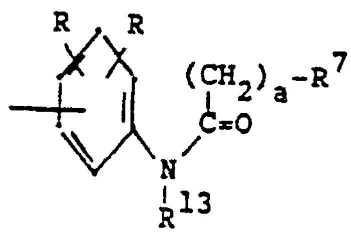

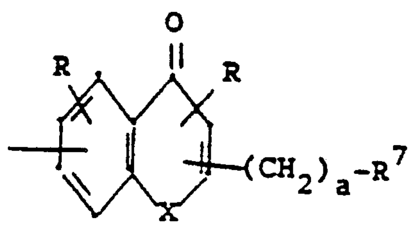

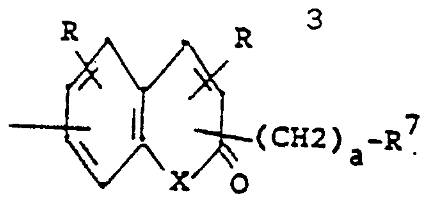

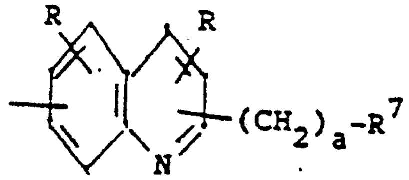

, ,

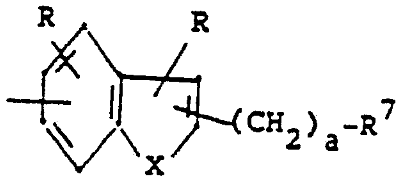

oder

ist; 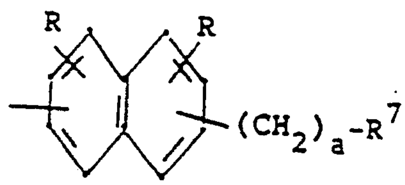

$R^{10}$ jeweils unabhängig OH; $N(R^4)_2$; $CF_3$; Niederalkyl; Niederalkoxy; Phenyl oder durch ein oder mehrere Alkyl- oder Alkoxygruppen mit 1 bis 3 Kohlenstoffatomen, Halogen, Hydroxy, $COOR^4$, CN, Formyl oder Niederalkylacyl substituiertes Phenyl ist;

$R^{11}$ jeweils unabhängig

A) ein monocyclischer oder bicyclischer heterocyclischer Rest mit 3 bis 12 nuclearen Kohlenstoffatomen und 1 bis 2 aus N und S ausgewählten nuclearen Heteroatomen, von denen wenigstens eines N ist, wobei jeder Ring im heterocyclischen Rest aus 5 oder 6 Atomen gebildet ist, oder

B) der Rest $W\text{-}R^{12}$ ist;

$R^{12}$ jeweils unabhängig bis zu 20 Kohlenstoffatome enthält und (1) ein Alkylrest oder (2) ein Alkylacylrest einer organischen acyclischen oder monocyclischen Carbonsäure mit nicht mehr als einem Heteroatom im Ring ist;

$R^{13}$ jeweils unabhängig H oder $R^{10}$ ist;

W jeweils unabhängig O, S oder NH ist;

X jeweils unabhängig O, S oder $NR^{13}$ ist;

$X^1$ und $X^2$ jeweils unabhängig O, S, SO, $SO_2$, $S(O)=NR^4$, $NR^4$, $NCOR^1$, NCN oder $NCONHR^4$ ist;

$X^3$ O, S, SO oder $SO_2$ ist;

$Y^1$ OH oder das N-Ende einer Aminosäure ist, so daß $Y^1H$ eine essentielle Aminosäure ist;

$Y^2$ H oder das C-Ende einer Aminosäure ist, so daß $Y^2OH$ eine essentielle Aminosäure ist;

Z O, H und OH oder H und $R^4$ ist;

a jeweils unabhängig 0 bis 4 ist;

b jeweils unabhängig 1 bis 6 ist;

n jeweils unabhängig 0 bis 6 ist;

p jeweils unabhängig 0 bis 2 ist;

q jeweils unabhängig 0 bis 4 ist;

r jeweils unabhängig 0 bis 4 ist;

s jeweils unabhängig 0 bis 3 ist;

t jeweils unabhängig 0 bis 1 ist;

Het jeweils unabhängig ein heterocyclischer oder heterocyclischer oder heterobicyclischer Ring von

jeweils 5 oder 6 Atomen ist, der ein oder mehrere aus O, N oder S ausgewählte Heteroatome enthält, wobei der heterocyclische oder heterobicyclische Ring ein saures Proton enthält; und die Begriffe "Niederalkyl", "Niederalkenyl" und "Niederalkoxy" jeweils $C_{1-7}$-Alkyl, $C_{2-7}$-Alkenyl und $C_{1-7}$-Alkoxy bedeuten;
oder ein pharmazeutisch annehmbares Salz davon.

**2.** Verbindung nach Anspruch 1, worin
$R^1$ H oder Niederalkyl ist;
$R^2$ H ist;
$R^3$ jeweils unabhängig Niederalkyl oder Niederalkenyl ist;
$R^5$ jeweils unabhängig H; $OR^2$ ist oder beide $R^5$ unter Bildung eines doppelt gebundenen Sauerstoffs (=O) zusammengenommen werden können;
$R^7$ jeweils unabhängig $COOR^4$; CHO; $CH^2OH$; Tetrazol oder Het ist;
$X^1$; $X^2$ und $X^3$ jeweils unabhängig O, S, SO oder $SO_2$ sind;
n jeweils unabhängig 1 oder 2 ist; und
p jeweils 0 ist;
oder ein pharmazeutisch annehmbares Salz davon.

**3.** Verbindung nach Anspruch 2, worin jedes R unabhängig H; OH; Niederalkyl; Trifluormethyl; Niederalkoxy; Halogen; $N(R^4)_2$; $-(C=O)R^1$ $CH_2OR^4$; CN; $SR^{10}$; $SOR^{10}$ oder $SO_2R^{10}$ ist;
$R^1$ Niederalkyl ist;
n jeweils 1 ist; q = O;
oder ein pharmazeutisch annehmbares Salz davon.

**4.** Verbindung nach Anspruch 3 der Formel

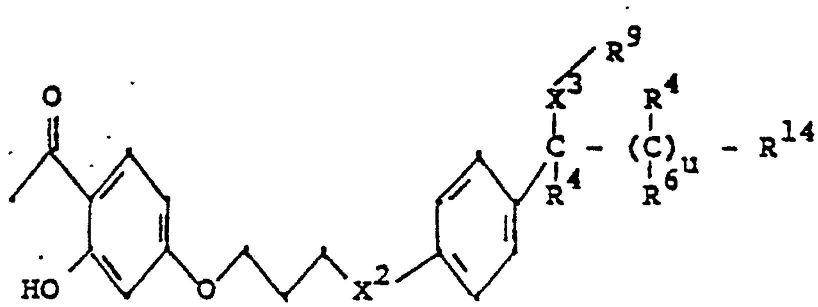

worin $R^9$ $(CH_2)_a$-Het ist, worin

Het

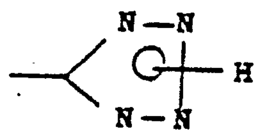

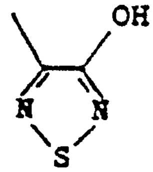

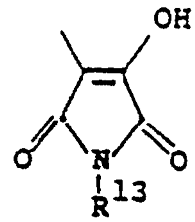

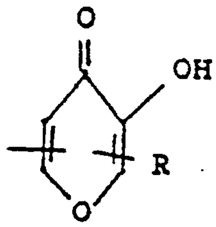

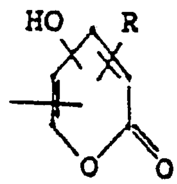

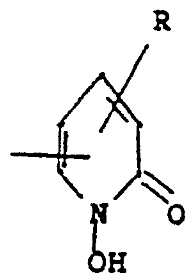

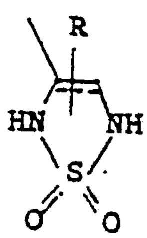

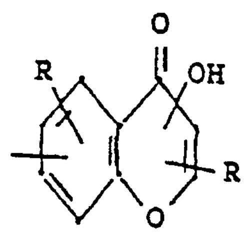

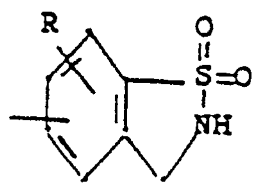

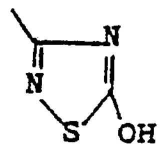

ist; worin die gestrichelte Linie eine mögliche Doppelbindung darstellt;

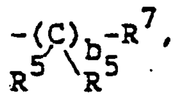

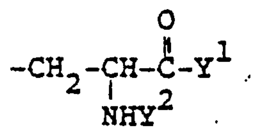

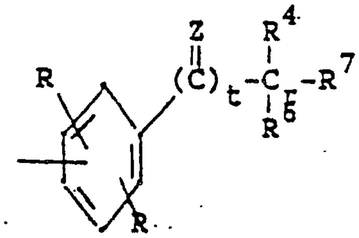

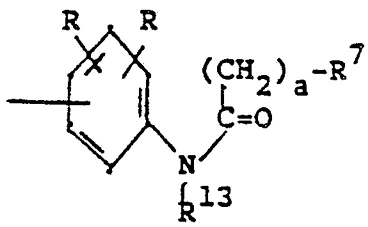

,

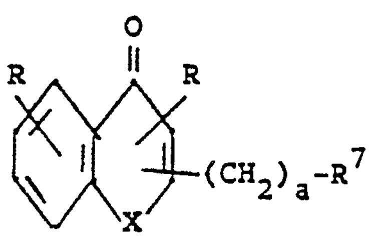

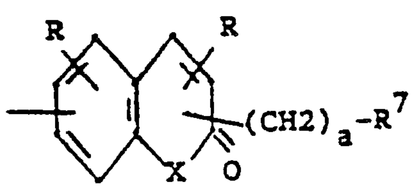

,

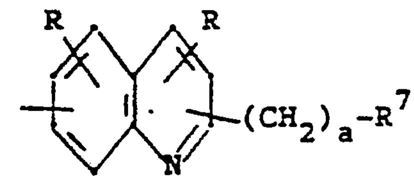,

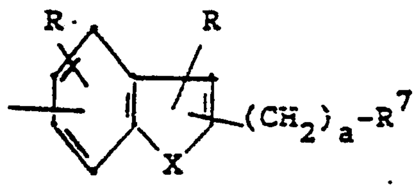 oder

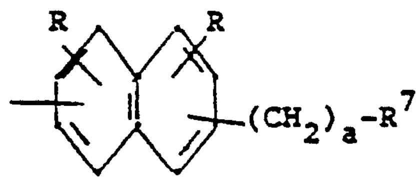

ist;

$R^{14}$ $COOR^4$ ist; u 0 bis 4 ist;

5. Verbindung nach Anspruch 1, welche 7((gamma-(4-((3-(4-Acetyl-3-hydroxy-2-propylphenoxy)-propyl)-thio)phenyl)-gamma-carboxy-β-methyl-propyl)thio)4-oxo-4-H-1-benzopyran-2-carboxylat, Dinatriumsalz;
4-((1-(4-((3-(4-Acetyl-3-hydroxy-2-propylphenoxy)-propyl)thio)phenyl)-3-carboxypropyl)thio)-gamma-oxobenzolbutansäure;
4-((3-(4-Acetyl-3-hydroxy-2-propylphenoxy)propyl)-thio)-gamma-((3-(carboxyacetyl)amino)phenyl)thio-beta-methylbenzolbutansäure;
4-((1-(4-((3-(4-Acetyl-3-hydroxy-2-propylphenoxy)-propyl)thio)phenyl)-3-carboxypropyl)thio)-beta-methyl-gamma-oxobenzolbutansäure;
N(S(gamma-(4-((3-(4-Acetyl-3-hydroxy-2-propylphenoxy)-propyl)thio)phenyl)-gamma-carboxy-β-methylpropyl)-cysteinyl)glycin, Dinatriumsalz;
4-((3-(4-Acetyl-3-hydroxy-2-propylphenoxy)propyl)-thio)-gamma-((2-carboxyethyl)thio)-beta-methyl-benzolbutansäure, Dinatriumsalz; Dinatriumsalz;
4-((3-(4-Acetyl-3-hydroxy-2-propylphenoxy)propyl)-thio)-gamma-(butylthio)-beta-methylbenzolbutansäure;
4-((3-(4-Acetyl-3-hydroxy-2-propylphenoxy)propyl)-thio)-beta-methyl-gamma-((1H-tetrazol-5-yl-methyl)-thio)benzolbutansäure, Dinatriumsalz;
Methyl-4-((3-(4-acetyl-3-hydroxy-2-propylphenoxy)-propyl)thio)-beta-methyl-gamma-((1H-tetrazol-5-yl-methyl)thio)benzolbutanoat, Natriumsalz;
4-((1-(4-((3-(4-Acetyl-3-hydroxy-2-propylphenoxy)-propyl)thio)phenyl)-3-carboxy-2-methylpropyl)thio)-gamma-hydroxy-beta-methylbenzolbutansäure, Dinatriumsalzmonohydrat;
4-((1-(4-((3-(4-Acetyl-3-hydroxy-2-propylphenoxy)-propyl)thio)phenyl)-3-carboxy-2-methylpropyl)thio)-beta-methyl-gamma-oxobenzolbutansäure; Monohydrat;
4-((3-(4-Acetyl-3-hydroxy-2-propylphenoxy)propyl)-thio)-gamma-((4-(3-carboxyl-1-hydroxypropyl)-phenyl)-thiobenzolbutansäure, Dinatriumsalz, Monohydrat;
4-((3-(4-Acetyl-3-hydroxy-2-propylphenoxy)propyl)-thio)-gamma-((2-carboxymethyl)thio)-beta-methylbenzolbutansäure, Dinatriumsalz, Hemihydrat;
4-((3-(4-Acetyl-3-hydroxy-2-propylphenoxy)propyl)-thio)-gamma-((2-carboxyethyl)thio)-beta-methylbenzolbutansäure, Dinatriumsalz;
4-((3-(4-Acetyl-3-hydroxy-2-propylphenoxy)propyl)-sulfonyl-gamma-((2-carboxyethyl)thio)-beta-methylbenzolbutansäure, Dinatriumsalz, Monohydrat; oder

4-((3-(4-Acetyl-3-hydroxy-2-propylphenoxy)propyl)-thio )-gamma-((2-N,N-dimethylcarbonylethyl)thio)-beta-methylbenzolbutansäure, Natriumsalz ist.

6. Zur Antagonisierung der Leukotrienwirkung in Säugetieren geeignete pharmazeutische Zusammensetzung, welche eine Verbindung, wie in einem der Ansprüche 1 bis 5 beansprucht, und einen pharmazeutisch annehmbaren Träger umfaßt.

7. Zusammensetzung nach Anspruch 6, welche weiterhin eine wirksame Menge eines zweiten wirksamen Bestandteils, der ein nicht-steroides entzündungshemmendes Mittel; ein peripheres Analgetikum; ein Cyclooxygenaseinhibitor; ein Leukotrienantagonist; ein Leukotrieninhibitor; ein $H_2$-Rezeptor-Antagonist; ein Antihistaminikum ein Prostaglandinantagonist oder ein Thromboxanantagonist ist.

8. Zusammensetzung nach Anspruch 7, worin das Gewichtsverhältnis der Verbindung nach einem der Ansprüche 1 bis 5 zum zweiten wirksamen Bestandteil im Bereich von 200:1 bis 1:200 liegt.

9. Verbindung nach einem der Ansprüche 1 bis 5 oder Zusammensetzung nach einem der Ansprüche 6 bis 8 zur Verwendung für die Vorbeugung der Synthese, der Wirkung oder der Freisetzung von SRS-A und der Leukotriene $C_4$, $D_4$, $E_4$ und $B_4$ in Säugetieren.

10. Verbindung nach einem der Ansprüche 1 bis 5 oder Zusammensetzung nach einem der Anspüche 6 bis 8 zur Verwendung im Zellschutz bei Säugetieren, die des Zellschutzes bedürfen.

Patentansprüche für folgenden Vertragsstaat: AT

1. Verfahren zur Herstellung einer Verbindung der Formel:

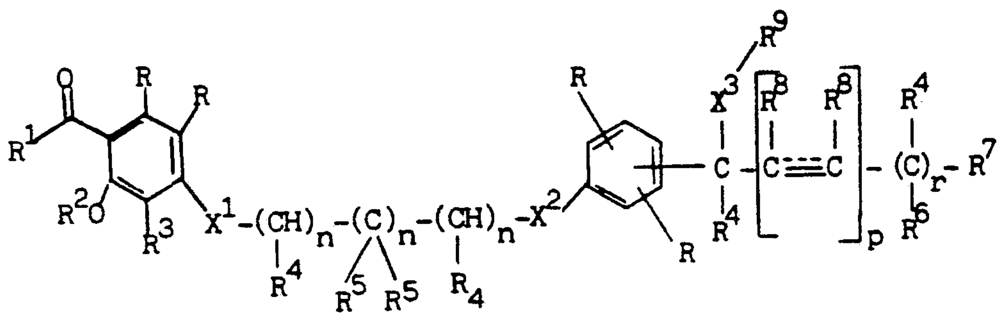

(I) ,

worin:
die durchbrochene Linie eine fakultative Dreifachbindung darstellt;
jeder Rest R unabhängig H; OH; Niederalkyl; Niederalkenyl; Trifluormethyl; Niederalkoxy; Phenyl; Phenyl, das mit Alkyl mit 1 bis 3 Kohlenstoffatomen oder mit Halogen substituiert ist; Benzyl; Phenethyl; Halogen; $N(R^4)_2$; $-(C=O)R^1$; $CH_2OR^4$; CN; $SR^{10}$; $SOR^{10}$; $SO_2R^{10}$; oder Nitro ist;
$R^1$ für H; Niederalkyl; oder Niederalkoxy steht;
$R^2$ für H; Niederalkyl; $R^4CO$ oder $R^4OCH_2$ steht;
jeder Rest $R^3$ unabhängig Niederalkyl oder Niederalkenyl bedeutet;
jeder Rest $R^4$ unabhängig H oder Niederalkyl darstellt;
jeder Rest $R^5$ unabhängig H; $OR^2$; Niederalkyl ist; oder beide Reste $R^5$ kombiniert sein können, um einen doppelt gebundenen Sauerstoff (=O) oder eine $=C(R^4)_2$-Gruppe auszubilden;
jeder Rest $R^6$ unabhängig H; OH; oder Niederalkyl bedeutet;
jeder Rest $R^7$ unabhängig $COOR^4$; CHO; $CH_2OH$; Tetrazol;$CONHSO_2R^{10}$; $NHSO_2R^{10}$; Hydroxymethylketon; Acetoxymethylketon; $CON(R^4)_2$; CN; Het; oder

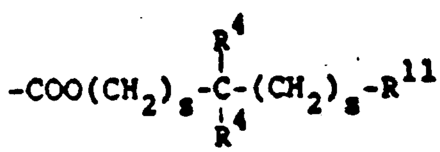

darstellt;

jeder Rest $R^8$ unabhängig H oder Niederalkyl ist, und fehlt, wenn eine Dreifachbindung vorliegt; $R^9$ für $R^3$,

$(CH_2)_a$-Het,

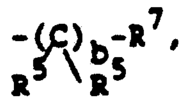

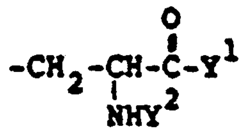
,

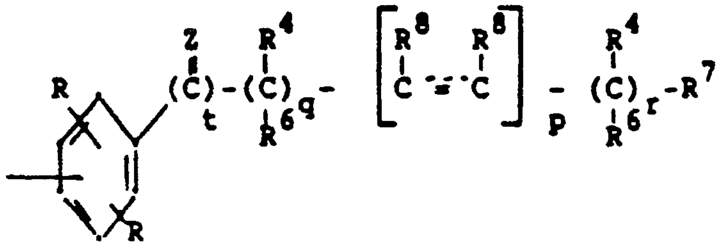
,

worin die durchbrochene Linie eine fakultative Dreifachbindung darstellt,

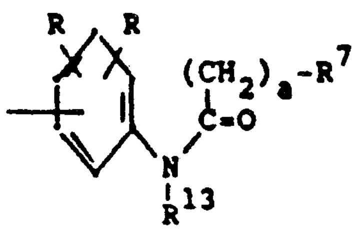
,

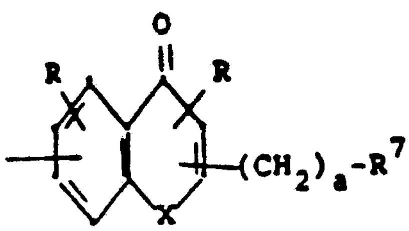
,

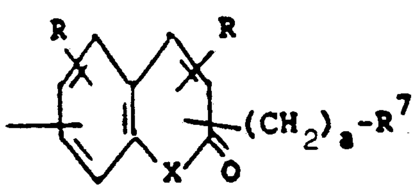
,

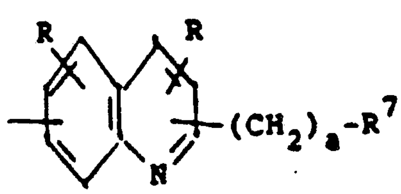
,

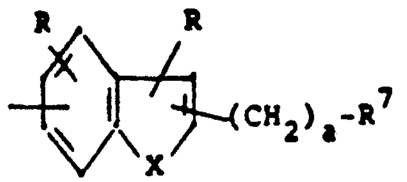 oder

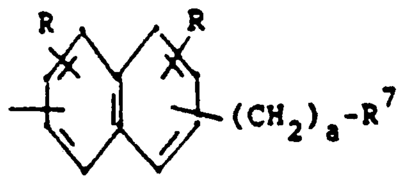

steht; jeder Rest $R^{10}$ unabhängig OH; $N(R^4)_2$; $CF_3$; Niederalkyl; Niederalkoxy; Phenyl; oder Phenyl darstellt, das mit einer oder mehreren Alkyl- oder Alkoxygrupppen mit 1 bis 3 Kohlenstoffatomen, Halogen, Hydroxy, $COOR^4$,CN , Formyl oder Niederalkylacyl substituiert ist;

jeder Rest $R^{11}$ unabhängig

A) ein monocyclischer oder bicyclischer heterocyclischer Rest, mit 3 bis 12 Kohlenstoffatatomen im Kern und 1 bis 2 Heteroatomen im Kern, ausgewählt unter N und S, wobei mindestens ein Atom N ist, und worin jeder Ring im heterocyclischen Ring aus 5 oder 6 Atomen gebildet wird, oder

B) der Rest W-$R^{12}$ ist;

jeder Rest $R^{12}$ unabhängig bis zu 20 Kohlenstoffatome enthält und (1) ein Alkylrest oder (2) ein Alkylacylrest einer organischen acyclischen oder monocyclischen Carbonsäure ist, welche nicht mehr als ein Heteroatam im Ring enthält;

jeder Rest $R^{13}$ unabhängig H oder $R^{10}$ ist;

jeder Rest W unabhängig für O, S oder NH steht;

jeder Rest X unabhängig O, S oder $NR^{13}$ darstellt;

$X^1$ und $X^2$ jeweils unabhängig voneinander O, S, SO, $SO_2$, $S(O)=NR^4$ $NR^4$, $NCOR^1$,NCN oder $NCONHR^4$ sind;

$X^3$ für O, S, SO oder $SO_2$ steht;

$Y^1$ OH oder das N-Ende einer Aminosäure bedeutet, so daß $Y^1H$ eine essentielle Aminosäure ist;

$Y^2$ H oder das C-Ende einer Aminosäure bedeutet, so daß $Y^2OH$ eine essentielle Aminosäure ist;

Z für O, H und OH, oder H und $R^4$ steht;

jedes a unabhängig von 0 bis 4 beträgt;

jedes b unabhängig von 1 bis 6 beträgt;

jedes n unabhängig von 0 bis 6 beträgt;

jedes p unabhängig von 0 bis 2 beträgt;

jedes q unabhängig von 0 bis 4 beträgt;

jedes r unabhängig von 0 bis 4 beträgt;

jedes s unabhängig von 0 bis 3 beträgt;

jedes t unabhängig von 0 bis 1 beträgt;

jeder Rest He t unabhängig einen heterocyclischen oder heterobicyclischen Ring mit jeweils 5 oder 6 Atomen darstellt, welcher ein oder mehrere Heteroatome, ausgewählt unter O, H oder S enthält, welcher heterocyclische oder heterobicyclische Ring ein saures Proton enthält;

und die Bezeichnungen "Niederalkyl", "Niederalkenyl" und "Niederalkoxy" $C_{1-7}$-Alkyl, $C_{2-7}$-Alkenyl bzw. $C_{1-7}$-Alkoxy bedeuten;

oder eines pharmazeutisch annehmbaren Salzes hievon;

umfassend:

Verestern einer Verbindung der Formel X

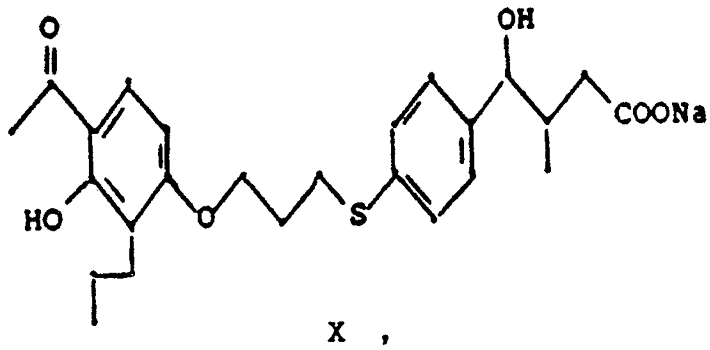

X ,

Umsetzen der so erhaltenen Verbindung der Formel XI

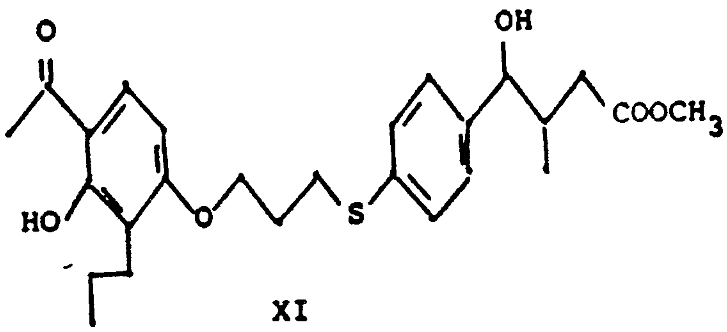

XI

mit einem Thiol der Formel $HSR^9$, worin $R^9$ wie vorstehend definiert ist, in Gegenwart eines Lewis-Säurekatalysators in einem inerten Lösungsmittel;
Hydrolysieren der so erhaltenen Verbindung der Formel XII

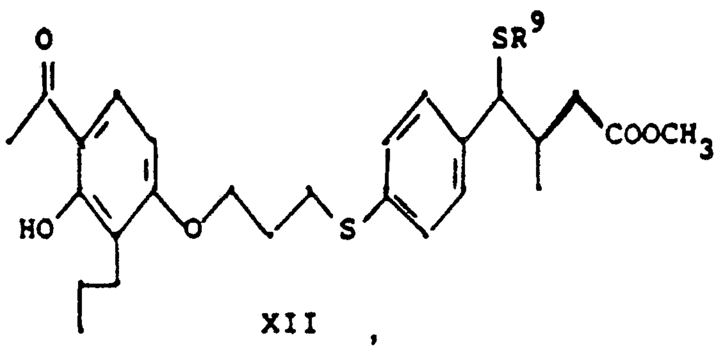

XII ,

worin $R^9$ wie vorstehend definiert ist, mit einer wäßigen Base, um das entsprechende Salz einer Säure der Formel XIII

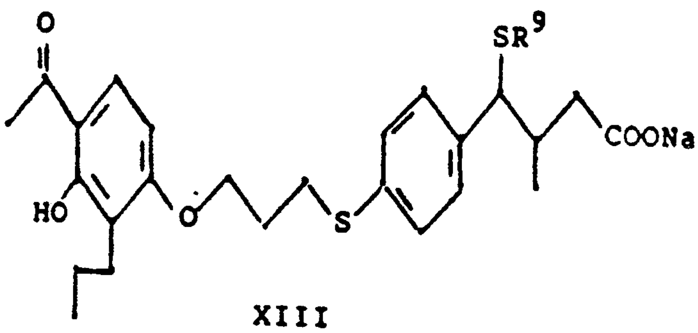

XIII

zu erhalten.

2. Verfahren zur Herstellung einer Verbindung nach Anspruch 1, umfassend

Umsetzen einer Verbindung der Formel XI

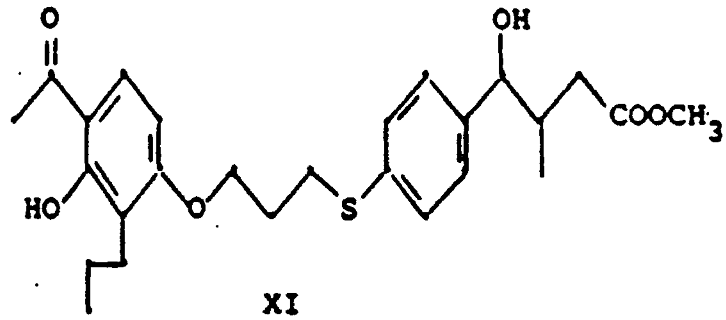

XI

mit Thiomilchsäure in Gegenwart eines Lewis-Säurekatalysators in einem inerten Lösungsmittel; Umsetzen der so erhaltenen Verbindung der Formel XV

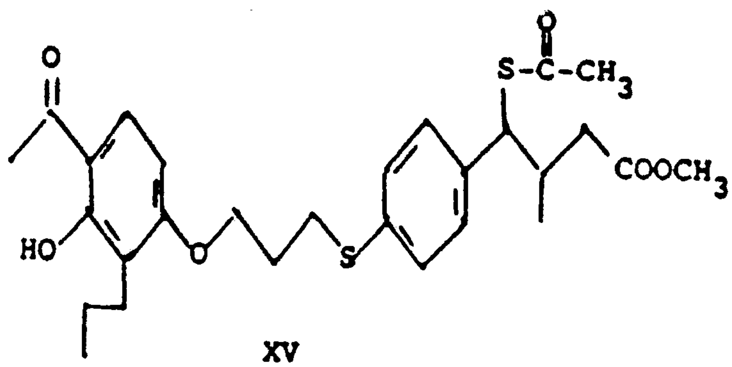

XV

mit einem Alkalimetallalkoxid, gefolgt von Ansäuern; Behandeln der so erhaltenen Verbindung der Formel XVI

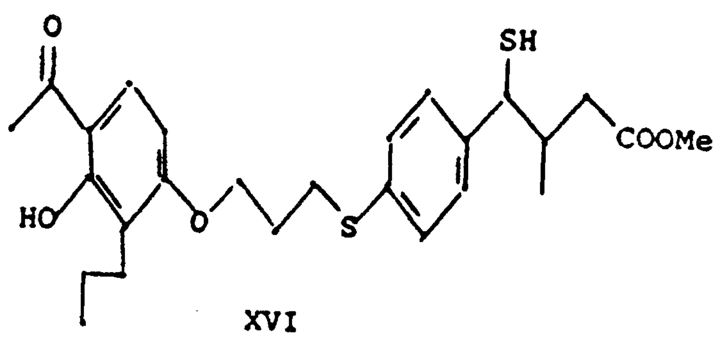

XVI

mit einer starken Base in einem inerten Lösungsmittel bei niedriger Temperatur, um die entsprechenden Thiolactone der Formel XVIIa bzw. XVIIb zu erhalten,

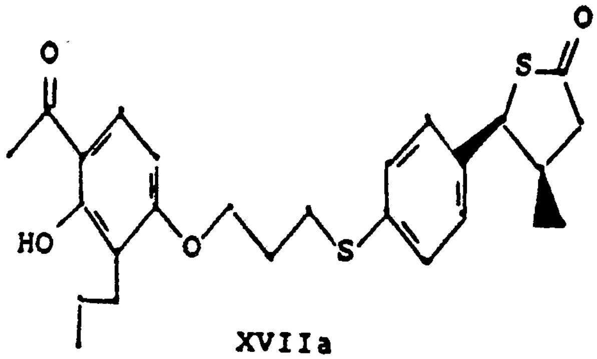

XVIIa

+

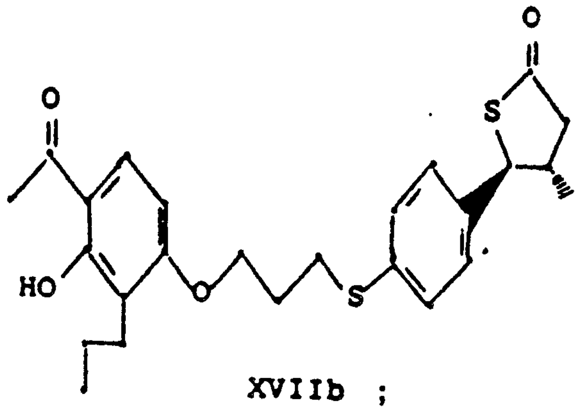

XVIIb ;

Auftrennen der Isomere und Behandeln des Isomers XVIIa bzw. XVIIb mit einem Alkalimetallalkoxid, gefolgt von einer Umsetzung mit einem ω-Halogenalkansäureester oder einem α,β-ungesättigten Alkensäureester;
Hydrolysieren der so erhaltenen Verbindung der Formel XII

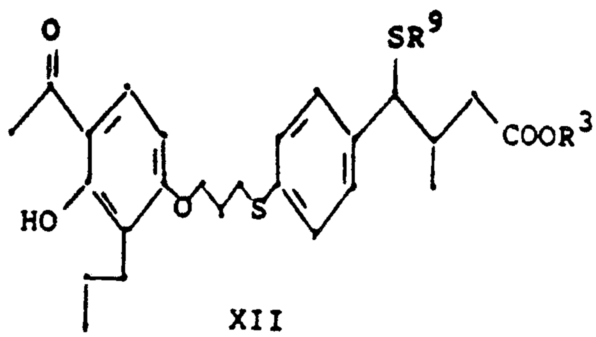

XII

um die entsprechende Disäure der Formel XIII zu erhalten.

3. Verfahren nach Anspruch 2, worin die Verbindungen XVIIa bzw. XVIIb mit organischen Persäuren oder Wasserstoffperoxid oxidiert werden, um die Sulfone der Formel XVIII

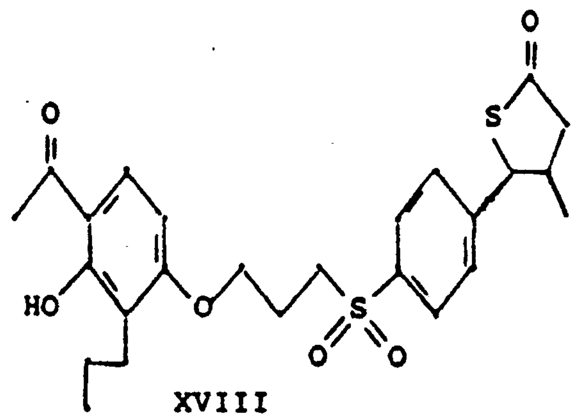

XVIII

zu erhalten.

4. Verfahren nach einem der Anprüche 1 bis 3, angewandt auf die Herstellung einer Verbindung wie in Anspruch 1 definiert, worin $R^1$ H oder Niederalkyl darstellt;
jeder Rest $R^3$ unabhängig Niederalkyl oder Niederalkenyl bedeutet;
jeder Rest $R^5$ unabhängig H; $OR^2$ ist; oder beide Reste $R^5$ kombiniert sein können, um einen doppelt gebundenen Sauerstoff (=O) auszubilden;
jeder Rest $R^7$ unabhängig $COOR^4$, CHO, $CH_2OH$, Tetrazol oder Het ist;
$X^1$, $X^2$ und $X^3$ unabhängig voneinander O, S, SO oder $SO_2$ darstellen;
jedes n unabhängig 1 oder 2 bedeutet; und jedes p O ist;
oder eines pharmazeutisch annehmbaren Salzes hievon.

5. Verfahren nach einem der Ansprüche 1 bis 3, angewandt auf die Herstellung einer Verbindung wie in Anspruch 4 definiert, worin jeder Rest R unabhängig H, OH, Niederalkyl, Trifluormethyl, Niederalkoxy, Halogen, $N(R^4)_2$, $-(C=O)R^1$, $CH_2OR^4$, CN, $SR^{10}$, $SOR^{10}$ oder $SO_2R^{10}$ bedeutet;
$R^1$ Niederalkyl darstellt;
jedes n 1 ist; q den Wert O besitzt;
oder eines pharmazeutisch annehmbaren Salzes hievon.

6. Verfahren nach einem der Ansprüche 1 bis 3, angewandt auf die Herstellung einer Verbindung wie in Anspruch 5 definiert, der Formel:

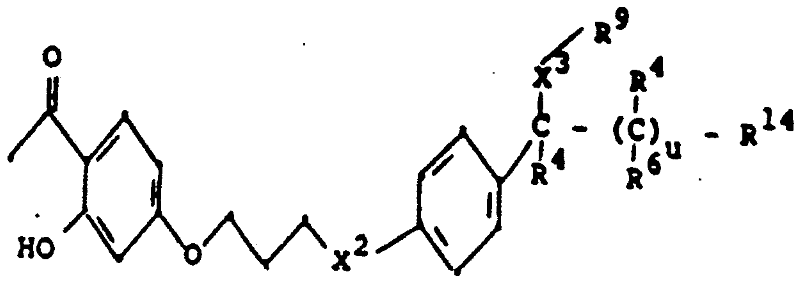

(II),

worin $R^9$ für

$(CH_2)_a$-Het ,

worin der Rest Het

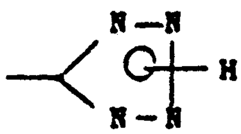

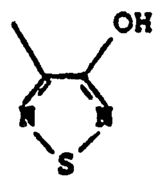

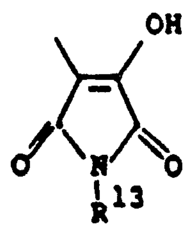

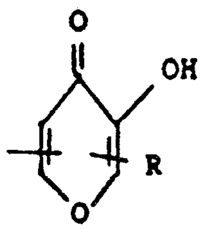

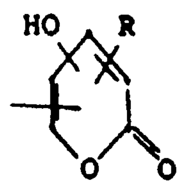

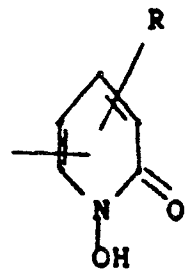

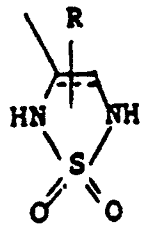 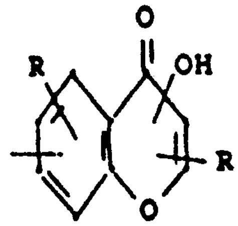 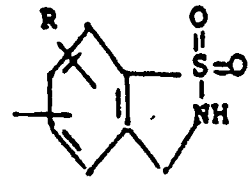

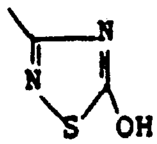 ,

worin die durchbrochene Linie eine fakultative Doppelbindung darstellt, ist,

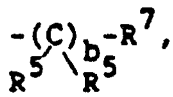

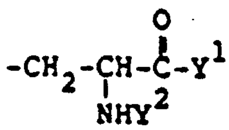 ,

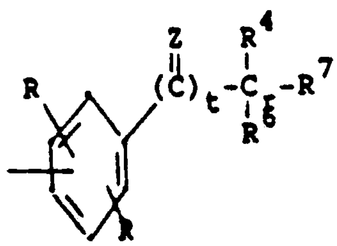 ,

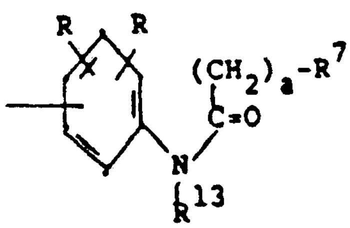 ,

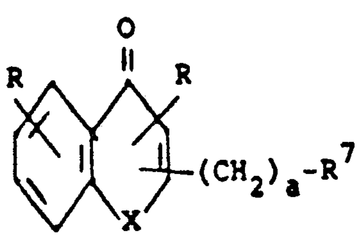 ,

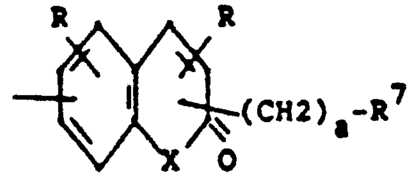

,

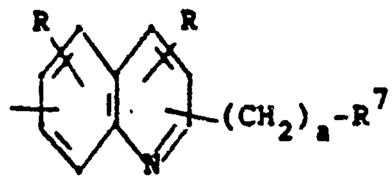

,

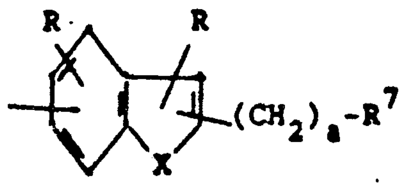

oder

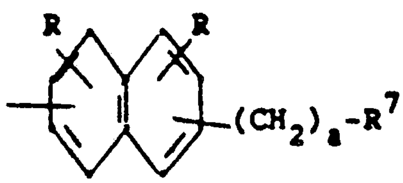

steht;
$R^{14}$ $COOR^4$ bedeutet; u von 0 bis 4 beträgt;
oder eines pharmazeutisch annehmbaren Salzes hievon.

7. Verfahren nach einem der Anspüche 1 bis 3, angewandt auf die Hetellung von einer der Vebindungen
7-((γ-(4-((3-(4-Acetyl-3-hydroxy-2-propylphenoxy)-propyl)thio)phenyl)-γ-carboxy-β-methylpropyl)-4-oxo-4H-1-benzopyran-2-carboxylat-dinatriumsalz;
4-((1-(4-((3-(4-Acetyl-3-hydroxy-2-propylphenoxy)-propyl)thio)phenyl)-3-carboxypropyl)thio)-γ-oxobenzolbutansäure;
4-((3-(4-Acetyl-3-hydroxy-2-propylphenoxy)propyl)thio)-γ-((3-(carboxy-acetyl)amino)phenyl)thio-β-methylbenzolbutansäure;
4-((1-(4-((3-(4-Acetyl-3-hydroxy-2-propylphenoxy)propyl)thio)phenyl)-3-carboxypropyl)thio)-β-methyl-γ-oxobenzolbutansäure;
N-(S-(γ-(4-((3-(4-Acetyl-3-hydroxy-2-propylphenoxy)propyl)thio)-phenyl)-γ-carboxy-β-methylpropyl)-cysteinyl)glycin-dinatriumsalz;
4-((3-(4-Acetyl-3-hydroxy-2-propylphenyl)propyl)thio)-γ-((2-carboxy-ethyl)thio)-β-methylbenzolbutansäure-dinatriumsalz;
4-((3-(4-Acetyl-3-hydroxy-2-propylphenoxy)propyl)thio)-γ-(butylthio)-β-methylbenzolbutansäure;
4-((3-(4-Acetyl-3-hydroxy-2-propylphenoxy)propyl)thio)-β-methyl-γ-((1H-tetrazol-5-yl-methyl)thio)-benzolbutansäure-dinatriumsalz-monohydrat;
Methyl-4-((3-(4-acetyl-3-hydroxy-2-propylphenoxy)propyl)thio)-β-methyl-γ-((1H-tetrazol-5-yl-methyl)thio)-benzolbutanoat-natriumsalz;
4-((1-(4-((3-(4-Acetyl-3-hydroxy-2-propylphenoxy)propyl)thio)phenyl)-3-carboxy-2-methylpropyl)thio)-γ-hydroxy-β-methylbenzolbutansäure-dinatriumsalz-monohydrat;
4-((1-(4-((3-(4-Acetyl-3-hydroxy-2-propylphenoxy)propyl)thio)phenyl)-3-carboxy-2-methylpropyl)thio)-β-methyl-γ-oxobenzolbutansäure-monohydrat;
4-((3-(4-Acetyl-3-hydroxy-2-propylphenoxy)propyl)thio)-γ-((4-(3-carboxy-1-hydroxypropyl)phenyl)thio)-benzolbutansäure-dinatriumsalz-monohydrat;
4-((3-(4-Acetyl-3-hydroxy-2-propylphenoxy)propyl)thio)-γ-((2-carboxy-methyl)thio)-β-methylbenzolbutansäure-dinatriumsalz-hemihydrat;
4-((3-(4-Acetyl-3-hydroxy-2-propylphenoxy)propyl)thio)-γ-((2-carboxy-ethyl)thio)-β-methylbenzolbutansäure-dinatriumsalz;
4-((3-(4-Acetyl-3-hydroxy-2-propylphenoxy)propyl)sulfonyl-γ-((2-carboxyethyl)thio)-β-methylbenzolbutansäure-dinatriumsalz-monohydrat; oder
4-((3-(4-Acetyl-3-hydroxy-2-propylphenoxy)propyl)thio)-γ-((2-N,N-dimethylcarbonylethyl)thio)-β-methylbenzolbutansäure-natriumsalz.

8. Verfahren zur Herstellung einer pharmazeutischen Zusammensetzung, welche zum Antagonisieren von Leukotrienwirkung bei Säugetieren nützlich ist, umfassend das Vermischen einer nach einem der Ansprüche 1 bis 7 hergestellten Verbindung mit einem pharmazeutisch annehmbaren Träger.

9. Verfahren zur Herstellung einer pharmazeutischen Zusammensetzung nach Anspruch 8, welche zusätzlich das Vermischen mit einer wirksamen Menge eines zweiten wirksamen Bestandteils umfaßt, welcher ein nicht-steroides, entzündungshemmendes Arzneimittel; ein peripheres Analgetikum; ein Cyclooxygenaseinhibitor; ein Leukotrienantagonist; ein Leukotrieninhibitor; ein $H_2$-Rezeptor-Antagonist; ein Antihistaminikum: ein Prostaglandinantagonist oder ein Thromboxanantagonist ist.

10. Verfahren zur Herstellung einer pharmazeutischen Zussammensetzung nach Anspruch 9, worin das Gewichtsvernältnis von der nach einem der Ansprüche 1 bis 7 hergestellten Verbindung zum zweiten wirksamen Bestandteil von 200:1 bis 1:200 reicht.

11. Verwendung einer nach einem der Ansprüche 1 bis 7 hergestellten Verbindung zur Herstellung einer Zusammensetzung, welche zur Verhinderung der Synthese, der Wirkung oder der Freisetzung von SRS-A und den Leukotrienen $C_4$, $D_4$, $E_4$ und $B_4$ bei Säugetieren verwendet wird.

12. Verwendung einer nach einem der Ansprüche 1 bis 7 hergestellten Verbindung zur Herstellung einer Zusammensetzung, welche zum Schutz von Zellen bei Säugetieren angewandt wird, die einen solchen Schutz von Zellen benötigen.